(19) **Europäisches Patentamt**

**European Patent Office**

**Office européen des brevets**

(11) **EP 0 694 067 B1**

(12) **EUROPEAN PATENT SPECIFICATION**

(45) Date of publication and mention
of the grant of the patent:
**08.02.2006 Bulletin 2006/06**

(51) Int Cl.:
*C12N 15/12* (2006.01)      *A61K 39/35* (2006.01)
*C12N 15/62* (2006.01)      *G01N 33/53* (2006.01)
*C07K 14/435* (2006.01)

(21) Application number: **93910592.0**

(22) Date of filing: **14.04.1993**

(86) International application number:
**PCT/US1993/003471**

(87) International publication number:
**WO 1994/024281 (27.10.1994 Gazette 1994/24)**

(54) **T CELL EPITOPES OF THE MAJOR ALLERGENS FROM DERMATOPHAGOIDES (HOUSE DUST MITE)**

T-ZELL EPITOPE DER HAUPTALLERGENE VON DERMATOPHAGOIDES (HAUSSTAUBMILBE)

EPITOPES DE LYMPHOCYTES T DES PRINCIPAUX ALLERGENES DE DERMATOPHAGOIDES (ACARIENS DETRITICOLES)

(84) Designated Contracting States:
**AT BE CH DE DK ES FR GB GR IE IT LI LU MC NL PT SE**

(43) Date of publication of application:
**31.01.1996 Bulletin 1996/05**

(73) Proprietor: **IMMULOGIC PHARMACEUTICAL CORPORATION**
**Waltham,**
**Massachusetts 02154 (US)**

(72) Inventors:
• GARMAN, Richard D.
  Arlington, MA 02174 (US)
• KUO, Mei-chang
  Winchester, MA 01890 (US)
• GREENSTEIN, Julia L.
  West Newton, MA 02165 (US)
• ROGERS, Bruce L.
  Belmont, MA 02178 (US)
• Franzén, Henry,M.
  Watertown, MA 02172 (US)

(74) Representative: **Price, Vincent Andrew et al**
**Fry Heath & Spence LLP**
**The Gables**
**Massetts Road**
**Horley**
**Surrey RH6 7DQ (GB)**

(56) References cited:
WO-A-88/10297          WO-A-92/04445
WO-A-93/08279

• JOURNAL OF IMMUNOLOGY, vol.148, no.3, 1 February 1992, BALTIMORE US pages 738 - 745 H.YSSEL ET AL. 'T Cell activation-inducing epitopes of the house dust mite allergen Der pI' cited in the application
• CLINICAL AND EXPERIMENTAL ALLERGY, vol.21, 1991 pages 161 - 166 K.Y.CHUA ET AL. 'IgE binding studies with large peptides expressed from Der pII cDNA constructs'
• ANNUAL REVIEW OF IMMUNOLOGY, vol.9, 1991, PALO ALTO, CA US pages 67 - 95 R.E.O'HEHIR ET AL. 'The specificity and regulation of T-cell responsiveness to allergens' cited in the application

SEQUENCE LISTING

    (1) GENERAL INFORMATION:

        (i) APPLICANT: Kuo, Mei-chang
        Garman, Richard
        Greenstein, Julia

        (ii) TITLE OF INVENTION: T CELL EPITOPES OF THE MAJOR ALLERGENS FROM DERMATOPHAGOIDES (HOUSE DUST MITE)

        (iii) NUMBER OF SEQUENCES: 119

        (iv) CORRESPONDENCE ADDRESS:

            (A) ADDRESSEE: LAHIVE & COCKFIELD

            (B) STREET: 60 STATE STREET SUITE 510

            (C) CITY: BOSTON

            (D) STATE: MA

            (E) COUNTRY: USA

(F) ZIP: 02109

(v) COMPUTER READABLE FORM:

    (A) MEDIUM TYPE: Floppy disk
(B) COMPUTER: IBM PC compatible
(C) OPERATING SYSTEM: PC-DOS/MS-DOS
(D) SOFTWARE: ASCII TEXT

(vi) CURRENT APPLICATION DATA:

    (A) APPLICATION NUMBER: US 07/881,396
(B) FILING DATE: 08-MAY-1992
(C) CLASSIFICATION:

(viii) ATTORNEY/AGENT INFORMATION:

    (A) NAME: MANDRAGOURAS, AMY E.
(B) REGISTRATION NUMBER: P36,207
(C) REFERENCE/DOCKET NUMBER: IMI-012/IPC-017C

(ix) TELECOMMUNICATION INFORMATION:

    (A) TELEPHONE: (617) 227-7400
(B) TELEFAX: (617) 227-5941

(2) INFORMATION FOR SEQ ID NO:1:

(i) SEQUENCE CHARACTERISTICS:

    (A) LENGTH: 834 base pairs
(B) TYPE: nucleic acid
(C) STRANDEDNESS: single
(D) TOPOLOGY: linear

(ii) MOLECULE TYPE: cDNA

(ix) FEATURE:

    (A) NAME/KEY: CDS

    (B) LOCATION: 1..738

(xi) SEQUENCE DESCRIPTION: SEQ ID NO:1:

AAA AAC CGA TTT TTG ATG AGT GCA GAA GCT TTT GAA CAC CTC AAA ACT
48
Lys Asn Arg Phe Leu Met Ser Ala Glu Ala Phe Glu His Leu Lys Thr
1                5                10                    15

CAA TTC GAT TTG AAT GCT GAA ACT AAC GCC TGC AGT ATC AAT GGA AAT
96
Gln Phe Asp Leu Asn Ala Glu Thr Asn Ala Cys Ser Ile Asn Gly Asn
                 20                25                    30

GCT CCA GCT GAA ATC GAT TTG CGA CAA ATG CGA ACT GTC ACT CCC ATT
144
Ala Pro Ala Glu Ile Asp Leu Arg Gln Met Arg Thr Val Thr Pro Ile
          35                40                    45

CGT ATG CAA GGA GGC TGT GGT TCA TGT TGG GCT TTC TCT GGT GTT GCC
192
Arg Met Gln Gly Gly Cys Gly Ser Cys Trp Ala Phe Ser Gly Val Ala
      50                55                60

GCA ACT GAA TCA GCT TAT TTG GCT CAC CGT AAT CAA TCA TTG GAT CTT
240
Ala Thr Glu Ser Ala Tyr Leu Ala His Arg Asn Gln Ser Leu Asp Leu
65                70                75                    80

GCT GAA CAA GAA TTA GTC GAT TGT GCT TCC CAA CAC GGT TGT CAT GGT
288
Ala Glu Gln Glu Leu Val Asp Cys Ala Ser Gln His Gly Cys His Gly
                 85                90                    95

GAT ACC ATT CCA CGT GGT ATT GAA TAC ATC CAA CAT AAT GGT GTC GTC
336
Asp Thr Ile Pro Arg Gly Ile Glu Tyr Ile Gln His Asn Gly Val Val
                 100                105                   110

CAA GAA AGC TAC TAT CGA TAC GTT GCA CGA GAA CAA TCA TGC CGA CGA
384
Gln Glu Ser Tyr Tyr Arg Tyr Val Ala Arg Glu Gln Ser Cys Arg Arg
      115                120                   125

CCA AAT GCA CAA CGT TTC GGT ATC TCA AAC TAT TGC CAA ATT TAC CCA
432
Pro Asn Ala Gln Arg Phe Gly Ile Ser Asn Tyr Cys Gln Ile Tyr Pro
     130                135                   140

CCA AAT GCA AAC AAA ATT CGT GAA GCT TTG GCT CAA ACC CAC AGC GCT
480
Pro Asn Ala Asn Lys Ile Arg Glu Ala Leu Ala Gln Thr His Ser Ala
145                150                   155                   160

ATT GCC GTC ATT ATT GGC ATC AAA GAT TTA GAC GCA TTC CGT CAT TAT
528
Ile Ala Val Ile Ile Gly Ile Lys Asp Leu Asp Ala Phe Arg His Tyr
               165                170                   175

GAT GGC CGA ACA ATC ATT CAA CGC GAT AAT GGT TAC CAA CCA AAC TAT
576
Asp Gly Arg Thr Ile Ile Gln Arg Asp Asn Gly Tyr Gln Pro Asn Tyr
               180                185                   190

```
CAC GCT GTC AAC ATT GTT GGT TAC AGT AAC GCA CAA GGT GTC GAT TAT
624
His Ala Val Asn Ile Val Gly Tyr Ser Asn Ala Gln Gly Val Asp Tyr
        195                 200                 205

TGG ATC GTA CGA AAC AGT TGG GAT ACC AAT TGG GGT GAT AAT GGT TAC
672
Trp Ile Val Arg Asn Ser Trp Asp Thr Asn Trp Gly Asp Asn Gly Tyr
    210                 215                 220

GGT TAT TTT GCT GCC AAC ATC GAT TTG ATG ATG ATT GAA GAA TAT CCA
720
Gly Tyr Phe Ala Ala Asn Ile Asp Leu Met Met Ile Glu Glu Tyr Pro
225                 230                 235                 240

TAT GTT GTC ATT CTC TAAACAAAAA GACAATTTCT TATATGATTG TCACTAATTT
775
Tyr Val Val Ile Leu
                245

ATTTAAAATC AAAATTTTTT AGAAAATGAA TAAATTCATT CACAAAAATT AAAAAAAAA
834
```

(2) INFORMATION FOR SEQ ID NO:2:

(i) SEQUENCE CHARACTERISTICS:

(A) LENGTH: 245 amino acids
(B) TYPE: amino acid
(D) TOPOLOGY: linear

(ii) MOLECULE TYPE: protein
(xi) SEQUENCE DESCRIPTION: SEQ ID NO:2:

```
Lys Asn Arg Phe Leu Met Ser Ala Glu Ala Phe Glu His Leu Lys Thr
  1               5                  10                  15

Gln Phe Asp Leu Asn Ala Glu Thr Asn Ala Cys Ser Ile Asn Gly Asn
             20                  25                  30

Ala Pro Ala Glu Ile Asp Leu Arg Gln Met Arg Thr Val Thr Pro Ile
         35                  40                  45

Arg Met Gln Gly Gly Cys Gly Ser Cys Trp Ala Phe Ser Gly Val Ala
     50                  55                  60

Ala Thr Glu Ser Ala Tyr Leu Ala His Arg Asn Gln Ser Leu Asp Leu
 65                  70                  75                  80

Ala Glu Gln Glu Leu Val Asp Cys Ala Ser Gln His Gly Cys His Gly
                 85                  90                  95

Asp Thr Ile Pro Arg Gly Ile Glu Tyr Ile Gln His Asn Gly Val Val
            100                 105                 110

Gln Glu Ser Tyr Tyr Arg Tyr Val Ala Arg Glu Gln Ser Cys Arg Arg
            115                 120                 125

Pro Asn Ala Gln Arg Phe Gly Ile Ser Asn Tyr Cys Gln Ile Tyr Pro
    130                 135                 140
```

```
    145                 150                 155                 160

    Ile Ala Val Ile Ile Gly Ile Lys Asp Leu Asp Ala Phe Arg His Tyr
                 165                 170                 175

    Asp Gly Arg Thr Ile Ile Gln Arg Asp Asn Gly Tyr Gln Pro Asn Tyr
                 180                 185                 190

    His Ala Val Asn Ile Val Gly Tyr Ser Asn Ala Gln Gly Val Asp Tyr
                 195                 200                 205

    Trp Ile Val Arg Asn Ser Trp Asp Thr Asn Trp Gly Asp Asn Gly Tyr
        210                 215                 220

    Gly Tyr Phe Ala Ala Asn Ile Asp Leu Met Met Ile Glu Glu Tyr Pro
    225                 230                 235                 240

    Tyr Val Val Ile Leu
                245
```

(2) INFORMATION FOR SEQ ID NO:3:

   (i) SEQUENCE CHARACTERISTICS:

      (A) LENGTH: 588 base pairs
      (B) TYPE: nucleic acid
      (C) STRANDEDNESS: single
      (D) TOPOLOGY: linear

   (ii) MOLECULE TYPE: cDNA
   (ix) FEATURE:

(A) NAME/KEY: CDS
(B) LOCATION: 69..509

(xi) SEQUENCE DESCRIPTION: SEQ ID NO:3:

```
CACAAATTCT TCTTTCTTCC TTACTACTGA TCATTAATCT GAAAACAAAA CCAAACAAAC
60

CATTCAAA ATG ATG TAC AAA ATT TTG TGT CTT TCA TTG TTG GTC GCA GCC
110
         Met Met Tyr Lys Ile Leu Cys Leu Ser Leu Leu Val Ala Ala
          1               5                  10

GTT GCT CGT GAT CAA GTC GAT GTC AAA GAT TGT GCC AAT CAT GAA ATC
158
Val Ala Arg Asp Gln Val Asp Val Lys Asp Cys Ala Asn His Glu Ile
 15               20                  25                      30

AAA AAA GTT TTG GTA CCA GGA TGC CAT GGT TCA GAA CCA TGT ATC ATT
206
Lys Lys Val Leu Val Pro Gly Cys His Gly Ser Glu Pro Cys Ile Ile
                  35                  40                      45

CAT CGT GGT AAA CCA TTC CAA TTG GAA GCC GTT TTC GAA GCC AAC CAA
254
His Arg Gly Lys Pro Phe Gln Leu Glu Ala Val Phe Glu Ala Asn Gln
                  50                  55                      60

AAC ACA AAA ACG GCT AAA ATT GAA ATC AAA GCC TCA ATC GAT GGT TTA
302
Asn Thr Lys Thr Ala Lys Ile Glu Ile Lys Ala Ser Ile Asp Gly Leu
          65                  70                  75

GAA GTT GAT GTT CCC GGT ATC GAT CCA AAT GCA TGC CAT TAC ATG AAA
350
Glu Val Asp Val Pro Gly Ile Asp Pro Asn Ala Cys His Tyr Met Lys
     80                  85                  90

TGC CCA TTG GTT AAA GGA CAA CAA TAT GAT ATT AAA TAT ACA TGG AAT
398
Cys Pro Leu Val Lys Gly Gln Gln Tyr Asp Ile Lys Tyr Thr Trp Asn
 95              100                 105                     110

GTT CCG AAA ATT GCA CCA AAA TCT GAA AAT GTT GTC GTC ACT GTT AAA
446
Val Pro Lys Ile Ala Pro Lys Ser Glu Asn Val Val Val Thr Val Lys
                 115                 120                     125

GTT ATG GGT GAT GAT GGT GTT TTG GCC TGT GCT ATT GCT ACT CAT GCT
494
Val Met Gly Asp Asp Gly Val Leu Ala Cys Ala Ile Ala Thr His Ala
                 130                 135                     140

AAA ATC CGC GAT TAAATAAACA AAATTTATTG ATTTTGTAAT CACAAATGAT
546
Lys Ile Arg Asp
             145

TGATTTTCTT TCCAAAAAAA AAATAAATAA AATTTTGGGA AT
588
```

(2) INFORMATION FOR SEQ ID NO:4:

    (i) SEQUENCE CHARACTERISTICS:

        (A) LENGTH: 146 amino acids
        (B) TYPE: amino acid
        (D) TOPOLOGY: linear

    (ii) MOLECULE TYPE: protein
    (xi) SEQUENCE DESCRIPTION: SEQ ID NO:4:

```
Met Met Tyr Lys Ile Leu Cys Leu Ser Leu Leu Val Ala Ala Val Ala
 1               5               10              15

Arg Asp Gln Val Asp Val Lys Asp Cys Ala Asn His Glu Ile Lys Lys
            20              25              30

Val Leu Val Pro Gly Cys His Gly Ser Glu Pro Cys Ile Ile His Arg
            35              40              45

Gly Lys Pro Phe Gln Leu Glu Ala Val Phe Glu Ala Asn Gln Asn Thr
        50              55              60

Lys Thr Ala Lys Ile Glu Ile Lys Ala Ser Ile Asp Gly Leu Glu Val
    65              70              75              80

Asp Val Pro Gly Ile Asp Pro Asn Ala Cys His Tyr Met Lys Cys Pro
                85              90              95

Leu Val Lys Gly Gln Gln Tyr Asp Ile Lys Tyr Thr Trp Asn Val Pro
                100             105             110

Lys Ile Ala Pro Lys Ser Glu Asn Val Val Val Thr Val Lys Val Met
            115             120             125

Gly Asp Asp Gly Val Leu Ala Cys Ala Ile Ala Thr His Ala Lys Ile
    130             135             140

Arg Asp
    145
```

(2) INFORMATION FOR SEQ ID NO:5:

    (i) SEQUENCE CHARACTERISTICS:

        (A) LENGTH: 1072 base pairs
        (B) TYPE: nucleic acid
        (C) STRANDEDNESS: single
        (D) TOPOLOGY: linear

    (ii) MOLECULE TYPE: cDNA
    (ix) FEATURE:

        (A) NAME/KEY: CDS
        (B) LOCATION: 36..1001

    (xi) SEQUENCE DESCRIPTION: SEQ ID NO:5:

```
CGTTTTCTTC CATCAAAATT AAAAATTCAT CAAAA ATG AAA TTC GTT TTG GCC
53
                                          Met Lys Phe Val Leu Ala
                                           1                   5

ATT GCC TCT TTG TTG GTA TTG AGC ACT GTT TAT GCT CGT CCA GCT TCA
101
Ile Ala Ser Leu Leu Val Leu Ser Thr Val Tyr Ala Arg Pro Ala Ser
              10              15                  20

ATC AAA ACT TTT GAA GAA TTC AAA AAA GCC TTC AAC AAA AAC TAT GCC
149
Ile Lys Thr Phe Glu Glu Phe Lys Lys Ala Phe Asn Lys Asn Tyr Ala
          25              30              35

ACC GTT GAA GAG GAA GAA GTT GCC CGT AAA AAC TTT TTG GAA TCA TTG
197
Thr Val Glu Glu Glu Glu Val Ala Arg Lys Asn Phe Leu Glu Ser Leu
      40              45              50

AAA TAT GTT GAA GCT AAC AAA GGT GCC ATC AAC CAT TTG TCC GAT TTG
245
Lys Tyr Val Glu Ala Asn Lys Gly Ala Ile Asn His Leu Ser Asp Leu
 55              60              65                  70

TCA TTG GAT GAA TTC AAA AAC CGT TAT TTG ATG AGT GCT GAA GCT TTT
293
Ser Leu Asp Glu Phe Lys Asn Arg Tyr Leu Met Ser Ala Glu Ala Phe
              75              80                  85
```

```
GAA CAA CTC AAA ACT CAA TTC GAT TTG AAT GCC GAA ACA AGC GCT TGC
341
Glu Gln Leu Lys Thr Gln Phe Asp Leu Asn Ala Glu Thr Ser Ala Cys
             90                  95                 100

CGT ATC AAT TCG GTT AAC GTT CCA TCG GAA TTG GAT TTA CGA TCA CTG
389
Arg Ile Asn Ser Val Asn Val Pro Ser Glu Leu Asp Leu Arg Ser Leu
         105                 110                 115

CGA ACT GTC ACT CCA ATC CGT ATG CAA GGA GGC TGT GGT TCA TGT TGG
437
Arg Thr Val Thr Pro Ile Arg Met Gln Gly Gly Cys Gly Ser Cys Trp
     120                 125                 130

GCT TTC TCT GGT GTT GCC GCA ACT GAA TCA GCT TAT TTG GCC TAC CGT
485
Ala Phe Ser Gly Val Ala Ala Thr Glu Ser Ala Tyr Leu Ala Tyr Arg
135                 140                 145                 150

AAC ACG TCT TTG GAT CTT TCT GAA CAG GAA CTC GTC GAT TGC GCA TCT
533
Asn Thr Ser Leu Asp Leu Ser Glu Gln Glu Leu Val Asp Cys Ala Ser
                 155                 160                 165

CAA CAC GGA TGT CAC GGC GAT ACA ATA CCA AGA GGC ATC GAA TAC ATC
581
Gln His Gly Cys His Gly Asp Thr Ile Pro Arg Gly Ile Glu Tyr Ile
                 170                 175                 180

CAA CAA AAT GGT GTC GTT GAA GAA AGA AGC TAT CCA TAC GTT GCA CGA
629
Gln Gln Asn Gly Val Val Glu Glu Arg Ser Tyr Pro Tyr Val Ala Arg
         185                 190                 195

GAA CAA CGA TGC CGA CGA CCA AAT TCG CAA CAT TAC GGT ATC TCA AAC
677
Glu Gln Arg Cys Arg Arg Pro Asn Ser Gln His Tyr Gly Ile Ser Asn
     200                 205                 210

TAC TGC CAA ATT TAT CCA CCA GAT GTG AAA CAA ATC CGT GAA GCT TTG
725
Tyr Cys Gln Ile Tyr Pro Pro Asp Val Lys Gln Ile Arg Glu Ala Leu
215                 220                 225                 230

ACT CAA ACA CAC ACA GCT ATT GCC GTC ATT ATT GGC ATC AAA GAT TTG
773
Thr Gln Thr His Thr Ala Ile Ala Val Ile Ile Gly Ile Lys Asp Leu
                 235                 240                 245

AGA GCT TTC CAA CAT TAT GAT GGA CGA ACA ATC ATT CAA CAT GAC AAT
821
Arg Ala Phe Gln His Tyr Asp Gly Arg Thr Ile Ile Gln His Asp Asn
             250                 255                 260

GGT TAT CAA CCA AAC TAT CAT GCC GTC AAC ATT GTC GGT TAC GGA AGT
869
Gly Tyr Gln Pro Asn Tyr His Ala Val Asn Ile Val Gly Tyr Gly Ser
         265                 270                 275
```

```
ACA CAA GGC GAC GAT TAT TGG ATC GTA CGA AAC AGT TGG GAT ACT ACC
917
Thr Gln Gly Asp Asp Tyr Trp Ile Val Arg Asn Ser Trp Asp Thr Thr
    280                 285                 290

TGG GGA GAT AGC GGA TAC GGA TAT TTC CAA GCC GGA AAC AAC CTC ATG
965
Trp Gly Asp Ser Gly Tyr Gly Tyr Phe Gln Ala Gly Asn Asn Leu Met
295                 300                 305                 310

ATG ATC GAA CAA TAT CCA TAT GTT GTA ATC ATG TGAACATTTG AAATTGAATA
1018
Met Ile Glu Gln Tyr Pro Tyr Val Val Ile Met
                315                 320

TATTTATTTG TTTTCAAAAT AAAAACAACT ACTCTTGCGA GTATTTTTTA CTCG
1072
```

(2) INFORMATION FOR SEQ ID NO:6:

   (i) SEQUENCE CHARACTERISTICS:

      (A) LENGTH: 321 amino acids
      (B) TYPE: amino acid
      (D) TOPOLOGY: linear

   (ii) MOLECULE TYPE: protein
   (xi) SEQUENCE DESCRIPTION: SEQ ID NO:6:

```
Met Lys Phe Val Leu Ala Ile Ala Ser Leu Leu Val Leu Ser Thr Val
  1               5                  10                  15

Tyr Ala Arg Pro Ala Ser Ile Lys Thr Phe Glu Glu Phe Lys Lys Ala
            20                  25                  30

Phe Asn Lys Asn Tyr Ala Thr Val Glu Glu Glu Glu Val Ala Arg Lys
        35                  40                  45

Asn Phe Leu Glu Ser Leu Lys Tyr Val Glu Ala Asn Lys Gly Ala Ile
        50                  55                  60

Asn His Leu Ser Asp Leu Ser Leu Asp Glu Phe Lys Asn Arg Tyr Leu
65                  70                  75                  80

Met Ser Ala Glu Ala Phe Glu Gln Leu Lys Thr Gln Phe Asp Leu Asn
                85                  90                  95

Ala Glu Thr Ser Ala Cys Arg Ile Asn Ser Val Asn Val Pro Ser Glu
            100                 105                 110

Leu Asp Leu Arg Ser Leu Arg Thr Val Thr Pro Ile Arg Met Gln Gly
            115                 120                 125

Gly Cys Gly Ser Cys Trp Ala Phe Ser Gly Val Ala Ala Thr Glu Ser
    130                 135                 140

Ala Tyr Leu Ala Tyr Arg Asn Thr Ser Leu Asp Leu Ser Glu Gln Glu
145                 150                 155                 160

Leu Val Asp Cys Ala Ser Gln His Gly Cys His Gly Asp Thr Ile Pro
            165                 170                 175


Arg Gly Ile Glu Tyr Ile Gln Gln Asn Gly Val Val Glu Glu Arg Ser
            180                 185                 190

Tyr Pro Tyr Val Ala Arg Glu Gln Arg Cys Arg Arg Pro Asn Ser Gln
        195                 200                 205

His Tyr Gly Ile Ser Asn Tyr Cys Gln Ile Tyr Pro Pro Asp Val Lys
    210                 215                 220

Gln Ile Arg Glu Ala Leu Thr Gln Thr His Thr Ala Ile Ala Val Ile
225                 230                 235                 240

Ile Gly Ile Lys Asp Leu Arg Ala Phe Gln His Tyr Asp Gly Arg Thr
            245                 250                 255

Ile Ile Gln His Asp Asn Gly Tyr Gln Pro Asn Tyr His Ala Val Asn
        260                 265                 270

Ile Val Gly Tyr Gly Ser Thr Gln Gly Asp Asp Tyr Trp Ile Val Arg
        275                 280                 285

Asn Ser Trp Asp Thr Thr Trp Gly Asp Ser Gly Tyr Gly Tyr Phe Gln
    290                 295                 300

Ala Gly Asn Asn Leu Met Met Ile Glu Gln Tyr Pro Tyr Val Val Ile
305                 310                 315                 320

Met
```

(2) INFORMATION FOR SEQ ID NO:7:

    (i) SEQUENCE CHARACTERISTICS:

        (A) LENGTH: 491 base pairs
        (B) TYPE: nucleic acid
        (C) STRANDEDNESS: single
        (D) TOPOLOGY: linear

    (ii) MOLECULE TYPE: cDNA
    (ix) FEATURE:

        (A) NAME/KEY: CDS
        (B) LOCATION: 1..390

    (xi) SEQUENCE DESCRIPTION: SEQ ID NO:7:

```
GAT CAA GTC GAT GTT AAA GAT TGT GCC AAC AAT GAA ATC AAA AAA GTA
48
Asp Gln Val Asp Val Lys Asp Cys Ala Asn Asn Glu Ile Lys Lys Val
 1               5                   10                  15

ATG GTC GAT GGT TGC CAT GGT TCT GAT CCA TGC ATA ATC CAT CGT GGT
96
Met Val Asp Gly Cys His Gly Ser Asp Pro Cys Ile Ile His Arg Gly
                20                  25                  30

AAA CCA TTC ACT TTG GAA GCC TTA TTC GAT GCC AAC CAA AAC ACT AAA
144
Lys Pro Phe Thr Leu Glu Ala Leu Phe Asp Ala Asn Gln Asn Thr Lys
                35                  40                  45
```

```
ACC GCT AAA ACT GAA ATC AAA GCC AGC CTC GAT GGT CTT GAA ATT GAT
192
Thr Ala Lys Thr Glu Ile Lys Ala Ser Leu Asp Gly Leu Glu Ile Asp
        50                  55                  60

GTT CCC GGT ATT GAT ACC AAT GCT TGC CAT TTT ATG AAA TGT CCA TTG
240
Val Pro Gly Ile Asp Thr Asn Ala Cys His Phe Met Lys Cys Pro Leu
65                  70                  75                      80

GTT AAA GGT CAA CAA TAT GAT GCC AAA TAT ACA TGG AAT GTG CCC AAA
288
Val Lys Gly Gln Gln Tyr Asp Ala Lys Tyr Thr Trp Asn Val Pro Lys
                    85                  90                      95

ATT GCA CCA AAA TCT GAA AAC GTT GTC GTT ACA GTC AAA CTT GTT GGT
336
Ile Ala Pro Lys Ser Glu Asn Val Val Val Thr Val Lys Leu Val Gly
            100                 105                 110

GAT AAT GGT GTT TTG GCT TGC GCT ATT GCT ACC CAC GCT AAA ATC CGT
384
Asp Asn Gly Val Leu Ala Cys Ala Ile Ala Thr His Ala Lys Ile Arg
            115                 120                 125

GAT TAAAAAAAAA AAATAAATAT GAAAATTTTC ACCAACATCG AACAAAATTC
437
Asp
    130

AATAACCAAA ATTTGAATCA AAACGGAAT TCCAAGCTGA GCGCCGGTCG CTAC
491
```

(2) INFORMATION FOR SEQ ID NO:8:

    (i) SEQUENCE CHARACTERISTICS:

        (A) LENGTH: 129 amino acids
        (B) TYPE: amino acid
        (D) TOPOLOGY: linear

    (ii) MOLECULE TYPE: protein
    (xi) SEQUENCE DESCRIPTION: SEQ ID NO:8:

```
Asp Gln Val Asp Val Lys Asp Cys Ala Asn Asn Glu Ile Lys Lys Val
1               5                   10                  15

Met Val Asp Gly Cys His Gly Ser Asp Pro Cys Ile Ile His Arg Gly
            20                  25                  30

Lys Pro Phe Thr Leu Glu Ala Leu Phe Asp Ala Asn Gln Asn Thr Lys
            35                  40                  45

Thr Ala Lys Thr Glu Ile Lys Ala Ser Leu Asp Gly Leu Glu Ile Asp
        50                  55                  60

Val Pro Gly Ile Asp Thr Asn Ala Cys His Phe Met Lys Cys Pro Leu
65                  70                  75                      80

Val Lys Gly Gln Gln Tyr Asp Ala Lys Tyr Thr Trp Asn Val Pro Lys
                    85                  90                      95
```

```
Ile Ala Pro Lys Ser Glu Asn Val Val Val Thr Val Lys Leu Val Gly
              100                 105                 110

Asp Asn Gly Val Leu Ala Cys Ala Ile Ala Thr His Ala Lys Ile Arg
              115                 120                 125

Asp
```

(2) INFORMATION FOR SEQ ID NO:9:

(i) SEQUENCE CHARACTERISTICS:

(A) LENGTH: 20 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:9:

```
Thr Asn Ala Cys Ser Ile Asn Gly Asn Ala Pro Ala Glu Ile Asp Leu
1               5                   10                  15

Arg Gln Met Arg
              20
```

(2) INFORMATION FOR SEQ ID NO:10:

(i) SEQUENCE CHARACTERISTICS:

(A) LENGTH: 27 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:10:

```
Glu Ile Asp Leu Arg Gln Met Arg Thr Val Thr Pro Ile Arg Met Gln
1               5                   10                  15

Gly Gly Cys Gly Ser Cys Trp Ala Phe Ser Gly
              20                  25
```

(2) INFORMATION FOR SEQ ID NO:11:

(i) SEQUENCE CHARACTERISTICS:

(A) LENGTH: 29 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:11:

```
        Thr Val Thr Pro Ile Arg Met Gln Gly Gly Cys Gly Ser Cys Trp Ala
        1               5               10                  15

        Phe Ser Gly Val Ala Ala Thr Glu Ser Ala Tyr Leu Ala
                        20                  25
```

(2) INFORMATION FOR SEQ ID NO:12:

    (i) SEQUENCE CHARACTERISTICS:

        (A) LENGTH: 21 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:12:

```
        Val Ala Ala Thr Glu Ser Ala Tyr Leu Ala His Arg Asn Gln Ser Leu
        1               5               10                  15

        Asp Leu Ala Glu Gln
                    20
```

(2) INFORMATION FOR SEQ ID NO:13:

    (i) SEQUENCE CHARACTERISTICS:

        (A) LENGTH: 22 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:13:

```
        His Arg Asn Gln Ser Leu Asp Leu Ala Glu Gln Glu Leu Val Asp Cys
        1               5               10                  15

        Ala Ser Gln His Gly Cys
                    20
```

(2) INFORMATION FOR SEQ ID NO:14:

    (i) SEQUENCE CHARACTERISTICS:

        (A) LENGTH: 21 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:14:

```
        Glu Leu Val Asp Cys Ala Ser Gln His Gly Cys His Gly Asp Thr Ile
        1               5                   10                  15

        Pro Arg Gly Ile Glu
                        20
```

(2) INFORMATION FOR SEQ ID NO:15:

    (i) SEQUENCE CHARACTERISTICS:

        (A) LENGTH: 28 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:15:

```
        Gly Asp Thr Ile Pro Arg Gly Ile Glu Tyr Ile Gln His Asn Gly Val
        1               5                   10                  15

        Val Gln Glu Ser Tyr Tyr Arg Tyr Val Ala Arg Glu
                        20                  25
```

(2) INFORMATION FOR SEQ ID NO:16:

    (i) SEQUENCE CHARACTERISTICS:

        (A) LENGTH: 20 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:16:

```
        Glu Tyr Ile Gln His Asn Gly Val Val Gln Glu Ser Tyr Tyr Arg Tyr
        1               5                   10                  15

        Val Ala Arg Glu
                    20
```

(2) INFORMATION FOR SEQ ID NO:17:

    (i) SEQUENCE CHARACTERISTICS:

        (A) LENGTH: 25 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:17:

```
His Asn Gly Val Val Gln Glu Ser Tyr Tyr Arg Tyr Val Ala Arg Glu
1               5                   10                  15

Gln Ser Cys Arg Arg Pro Asn Ala Gln
            20                  25
```

(2) INFORMATION FOR SEQ ID NO:18:

    (i) SEQUENCE CHARACTERISTICS:

        (A) LENGTH: 19 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:18:

```
Gln Ser Cys Arg Arg Pro Asn Ala Gln Arg Phe Gly Ile Ser Asn Tyr
1               5                   10                  15

Cys Gln Ile
```

(2) INFORMATION FOR SEQ ID NO:19:

    (i) SEQUENCE CHARACTERISTICS:

        (A) LENGTH: 22 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:19:

```
Arg Phe Gly Ile Ser Asn Tyr Cys Gln Ile Tyr Pro Pro Asn Ala Asn
1               5                   10                  15

Lys Ile Arg Glu Ala Leu
            20
```

(2) INFORMATION FOR SEQ ID NO:20:

    (i) SEQUENCE CHARACTERISTICS:

        (A) LENGTH: 24 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:20:

```
Tyr Pro Pro Asn Ala Asn Lys Ile Arg Glu Ala Leu Ala Gln Thr His
1               5                   10                  15

Ser Ala Ile Ala Val Ile Ile Gly
                20
```

(2) INFORMATION FOR SEQ ID NO:21:

    (i) SEQUENCE CHARACTERISTICS:

        (A) LENGTH: 26 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:21:

```
Ala Gln Thr His Ser Ala Ile Ala Val Ile Ile Gly Ile Lys Asp Leu
1               5                   10                  15

Asp Ala Phe Arg His Tyr Asp Gly Arg Thr
            20                  25
```

(2) INFORMATION FOR SEQ ID NO:22:

    (i) SEQUENCE CHARACTERISTICS:

        (A) LENGTH: 26 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:22:

```
Ile Lys Asp Leu Asp Ala Phe Arg His Tyr Asp Gly Thr Arg Ile Ile
1               5                   10                  15

Gln Arg Asp Asn Gly Tyr Gln Pro Asn Tyr
            20                  25
```

(2) INFORMATION FOR SEQ ID NO:23:

    (i) SEQUENCE CHARACTERISTICS:

        (A) LENGTH: 23 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:23:

```
        Ile Ile Gln Arg Asp Asn Gly Tyr Gln Pro Asn Tyr His Ala Val Asn
        1               5                   10              15

        Ile Val Gly Tyr Ser Asn Ala
                        20
```

(2) INFORMATION FOR SEQ ID NO:24:

    (i) SEQUENCE CHARACTERISTICS:

        (A) LENGTH: 22 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:24:

```
        His Ala Val Asn Ile Val Gly Tyr Ser Asn Ala Gln Gly Val Asp Tyr
        1               5                   10              15

        Trp Ile Val Arg Asn Ser
                        20
```

(2) INFORMATION FOR SEQ ID NO:25:

    (i) SEQUENCE CHARACTERISTICS:

        (A) LENGTH: 24 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:25:

```
        Gln Gly Val Asp Tyr Trp Ile Val Arg Asn Ser Trp Asp Thr Asn Trp
        1               5                   10              15

        Gly Asp Asn Gly Tyr Gly Tyr Phe
                        20
```

(2) INFORMATION FOR SEQ ID NO:26:

    (i) SEQUENCE CHARACTERISTICS:

        (A) LENGTH: 26 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:26:

```
Gly Asp Asn Gly Tyr Gly Tyr Phe Ala Ala Asn Ile Asp Leu Met Met
1               5               10              15

Ile Glu Glu Tyr Pro Tyr Val Val Ile Leu
              20              25
```

(2) INFORMATION FOR SEQ ID NO:27:

(i) SEQUENCE CHARACTERISTICS:

(A) LENGTH: 28 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:27:

```
Thr Asn Ala Cys Ser Ile Asn Gly Asn Ala Pro Ala Glu Ile Asp Leu
1               5               10              15

Arg Gln Met Arg Thr Val Thr Pro Ile Arg Met Gln
              20              25
```

(2) INFORMATION FOR SEQ ID NO:28:

(i) SEQUENCE CHARACTERISTICS:

(A) LENGTH: 24 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:28:

```
Ser Ile Asn Gly Asn Ala Pro Ala Glu Ile Asp Leu Arg Gln Met Arg
1               5               10              15

Thr Val Thr Pro Ile Arg Met Gln
              20
```

(2) INFORMATION FOR SEQ ID NO:29:

(i) SEQUENCE CHARACTERISTICS:

(A) LENGTH: 29 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:29:

```
Ala Phe Ser Gly Val Ala Ala Thr Glu Ser Ala Tyr Leu Ala His Arg
1               5               10              15
Asn Gln Ser Leu Asp Leu Ala Glu Gln Glu Leu Val Asp
            20              25
```

(2) INFORMATION FOR SEQ ID NO:30:

    (i) SEQUENCE CHARACTERISTICS:

        (A) LENGTH: 25 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:30:

```
Val Ala Ala Thr Glu Ser Ala Tyr Leu Ala His Arg Asn Gln Ser Leu
1               5               10              15
Asp Leu Ala Glu Gln Glu Leu Val Asp
            20              25
```

(2) INFORMATION FOR SEQ ID NO:31:

    (i) SEQUENCE CHARACTERISTICS:

        (A) LENGTH: 25 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ D ·NO:31:

```
Ala Phe Ser Gly Val Ala Ala Thr Glu Ser Ala Tyr Leu Ala His Arg
1               5               10              15
Asn Gln Ser Leu Asp Leu Ala Glu Gln
            20              25
```

(2) INFORMATION FOR SEQ ID NO:32:

    (i) SEQUENCE CHARACTERISTICS:

        (A) LENGTH: 29 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:32:

```
        Val Ala Ala Thr Glu Ser Ala Tyr Leu Ala His Arg Asn Gln Ser Le
        1               5                   10                  15

        Asp Leu Ala Glu Gln Glu Leu Val Asp Cys Ala Ser Gln
                        20                  25
```

(2) INFORMATION FOR SEQ ID NO:33:

    (i) SEQUENCE CHARACTERISTICS:

      (A) LENGTH: 28 amino acids
      (B) TYPE: amino acid
      (D) TOPOLOGY: linear

    (ii) MOLECULE TYPE: peptide
    (v) FRAGMENT TYPE: N-terminal
    (xi) SEQUENCE DESCRIPTION: SEQ ID NO:33:

```
        Glu Tyr Ile Gln His Asn Gly Val Val Gln Glu Ser Tyr Tyr Arg Tyr
        1               5                   10                  15

        Val Ala Arg Glu Gln Cys Arg Arg Pro Asn Ala Gln
                        20                  25
```

(2) INFORMATION FOR SEQ ID NO:34:

    (i) SEQUENCE CHARACTERISTICS:

      (A) LENGTH: 29 amino acids
      (B) TYPE: amino acid
      (D) TOPOLOGY: linear

    (ii) MOLECULE TYPE: peptide
    (v) FRAGMENT TYPE: N-terminal
    (xi) SEQUENCE DESCRIPTION: SEQ ID NO:34:

```
        Asp Thr Ile Pro Arg Gly Ile Glu Tyr Ile Gln His Asn Gly Val Val
        1               5                   10                  15

        Gln Glu Ser Tyr Tyr Arg Tyr Val Ala Arg Glu Gln Ser
                        20                  25
```

(2) INFORMATION FOR SEQ ID NO:35:

    (i) SEQUENCE CHARACTERISTICS:

      (A) LENGTH: 29 amino acids
      (B) TYPE: amino acid
      (D) TOPOLOGY: linear

    (ii) MOLECULE TYPE: peptide
    (v) FRAGMENT TYPE: N-terminal
    (xi) SEQUENCE DESCRIPTION: SEQ ID NO:35:

```
Gln Ile Tyr Pro Pro Asn Ala Asn Lys Ile Arg Glu Ala Leu Ala Gln
1               5                   10                  15

Thr His Ser Ala Ile Ala Val Ile Ile Gly Ile Lys Asp
                20              25
```

(2) INFORMATION FOR SEQ ID NO:36:

    (i) SEQUENCE CHARACTERISTICS:

        (A) LENGTH: 22 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:36:

```
Gln Ile Tyr Pro Pro Asn Ala Asn Lys Ile Arg Glu Ala Leu Ala Gln
1               5                   10                  15

Thr His Ser Ala Ile Ala
                20
```

(2) INFORMATION FOR SEQ ID NO:37:

    (i) SEQUENCE CHARACTERISTICS:

        (A) LENGTH: 26 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:37:

```
Ile Ile Gly Ile Lys Asp Leu Asp Ala Phe Arg His Tyr Asp Gly Arg
1               5                   10                  15

Thr Ile Ile Gln Arg Asp Asn Gly Tyr Gln
                20                  25
```

(2) INFORMATION FOR SEQ ID NO:38:

    (i) SEQUENCE CHARACTERISTICS:

        (A) LENGTH: 25 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:38:

```
Arg Asp Asn Gly Tyr Gln Phe Asn Tyr His Ala Val Asn Ile Val Gly
1               5                   10                  15

Tyr Ser Asn Ala Gln Gly Val Asp Tyr
            20                  25
```

(2) INFORMATION FOR SEQ ID NO:39:

(i) SEQUENCE CHARACTERISTICS:

(A) LENGTH: 29 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:39:

```
Asn Ile Val Gly Tyr Ser Asn Ala Gln Gly Val Asp Tyr Trp Ile Val
1               5                   10                  15

Arg Asn Ser Trp Asp Thr Asn Trp Gly Asp Asn Gly Tyr
            20                  25
```

(2) INFORMATION FOR SEQ ID NO:40:

(i) SEQUENCE CHARACTERISTICS:

(A) LENGTH: 23 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:40:

```
Asn Ile Val Gly Tyr Ser Asn Ala Gln Gly Val Asp Tyr Trp Ile Val
1               5                   10                  15

Arg Asn Ser Trp Asp Thr Asn
            20
```

(2) INFORMATION FOR SEQ ID NO:41:

(i) SEQUENCE CHARACTERISTICS:

(A) LENGTH: 20 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:41:

```
Asp Gln Val Asp Val Lys Asp Cys Ala Asn His Glu Ile Lys Lys Val
1               5                   10                  15

Leu Val Pro Gly
            20
```

(2) INFORMATION FOR SEQ ID NO:42:

    (i) SEQUENCE CHARACTERISTICS:

        (A) LENGTH: 25 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:42:

```
His Glu Ile Lys Lys Val Leu Val Pro Gly Cys His Gly Ser Glu Pro
1               5                   10                  15

Cys Ile Ile His Arg Gly Lys Pro Phe
            20              25
```

(2) INFORMATION FOR SEQ ID NO:43:

    (i) SEQUENCE CHARACTERISTICS:

        (A) LENGTH: 29 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:43:

```
His Gly Ser Glu Pro Cys Ile Ile His Arg Gly Lys Pro Phe Gln Leu
1               5                   10                  15

Glu Ala Val Phe Glu Ala Asn Gln Asn Thr Lys Thr Ala
            20              25
```

(2) INFORMATION FOR SEQ ID NO:44:

    (i) SEQUENCE CHARACTERISTICS:

        (A) LENGTH: 25 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:44:

```
        Gln Leu Glu Ala Val Phe Glu Ala Asn Gln Asn Thr Lys Thr Ala Lys
        1               5                   10                  15

        Ile Glu Ile Lys Ala Ser Ile Asp Gly
                        20                  25
```

(2) INFORMATION FOR SEQ ID NO:45:

(i) SEQUENCE CHARACTERISTICS:

(A) LENGTH: 27 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:45:

```
        Lys Ile Glu Ile Lys Ala Ser Ile Asp Gly Leu Glu Val Asp Val Pro
        1               5                   10                  15

        Gly Ile Asp Pro Asn Ala Cys His Tyr Met Lys
                        20                  25
```

(2) INFORMATION FOR SEQ ID NO:46:

(i) SEQUENCE CHARACTERISTICS:

(A) LENGTH: 26 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:46:

```
        Leu Glu Val Asp Val Pro Gly Ile Asp Pro Asn Ala Cys His Tyr Met
        1               5                   10                  15

        Lys Cys Pro Leu Val Lys Gly Gln Gln Tyr
                        20                  25
```

(2) INFORMATION FOR SEQ ID NO:47:

(i) SEQUENCE CHARACTERISTICS:

(A) LENGTH: 27 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:47:

```
Cys Pro Leu Val Lys Gly Gln Gln Tyr Asp Ile Lys Tyr Thr Trp Asn
1               5               10              15

Val Pro Lys Ile Ala Pro Lys Ser Glu Asn Val
            20              25
```

(2) INFORMATION FOR SEQ ID NO:48:

    (i) SEQUENCE CHARACTERISTICS:

        (A) LENGTH: 26 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:48:

```
Asp Ile Lys Tyr Thr Trp Asn Val Pro Lys Ile Ala Pro Lys Ser Glu
1               5               10              15

Asn Val Val Val Thr Val Lys Val Met Gly
            20              25
```

(2) INFORMATION FOR SEQ ID NO:49:

    (i) SEQUENCE CHARACTERISTICS:

        (A) LENGTH: 25 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:49:

```
Val Val Thr Val Lys Val Met Gly Asp Asp Gly Val Leu Ala Cys Ala
1               5               10              15

Ile Ala Thr His Ala Lys Ile Arg Asp
            20              25
```

(2) INFORMATION FOR SEQ ID NO:50:

    (i) SEQUENCE CHARACTERISTICS:

        (A) LENGTH: 26 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:50:

```
        Asp Gln Val Asp Val Lys Asp Cys Ala Asn His Glu Ile Lys Lys Val
        1               5               10                      15

        Leu Val Pro Gly Cys His Gly Ser Glu Pro
                    20              25
```

(2) INFORMATION FOR SEQ ID NO:51:

(i) SEQUENCE CHARACTERISTICS:

(A) LENGTH: 26 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:51:

```
        Asp Gln Val Asp Val Lys Asp Glu Ala Asn His Glu Ile Lys Lys Val
        1               5               10                      15

        Leu Val Pro Gly Cys His Gly Ser Glu Pro
                    20              25
```

(2) INFORMATION FOR SEQ ID NO:52:

(i) SEQUENCE CHARACTERISTICS:

(A) LENGTH: 26 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:52:

```
        Asp Gln Val Asp Val Lys Asp Ser Ala Asn His Glu Ile Lys Lys Val
        1               5               10                      15

        Leu Val Pro Gly Cys His Gly Ser Glu Pro
                    20              25
```

(2) INFORMATION FOR SEQ ID NO:53:

(i) SEQUENCE CHARACTERISTICS:

(A) LENGTH: 26 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:53:

```
        Asp Gln Val Asp Val Lys Asp Cys Ala Asn His Glu Ile Lys Lys Val
        1               5                   10                  15

        Leu Val Pro Gly Glu His Gly Ser Glu Pro
                    20          .           25
```

(2) INFORMATION FOR SEQ ID NO:54:

    (i) SEQUENCE CHARACTERISTICS:

        (A) LENGTH: 26 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:54:

```
        Asp Gln Val Asp Val Lys Asp Cys Ala Asn His Glu Ile Lys Lys Val
        1               5                   10                  15

        Leu Val Pro Gly Ser His Gly Ser Glu Pro
                    20                  25
```

(2) INFORMATION FOR SEQ ID NO:55:

    (i) SEQUENCE CHARACTERISTICS:

        (A) LENGTH: 26 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:55:

```
        Asp Gln Val Asp Val Lys Asp Glu Ala Asn His Glu Ile Lys Lys Val
        1               5                   10                  15

        Leu Val Pro Gly Glu His Gly Ser Glu Pro
                    20                  25
```

(2) INFORMATION FOR SEQ ID NO:56:

    (i) SEQUENCE CHARACTERISTICS:

        (A) LENGTH: 26 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:56:

```
Asp Gln Val Asp Val Lys Asp Ser Ala Asn His Glu Ile Lys Lys Val
1               5                   10                  15

Leu Val Pro Gly Ser His Gly Ser Glu Pro
              20              25
```

(2) INFORMATION FOR SEQ ID NO:57:

    (i) SEQUENCE CHARACTERISTICS:

        (A) LENGTH: 20 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:57:

```
Asp Gln Val Asp Val Lys Asp Glu Ala Asn His Glu Ile Lys Lys Val
1               5                   10                  15

Leu Val Pro Gly
              20
```

(2) INFORMATION FOR SEQ ID NO:58:

    (i) SEQUENCE CHARACTERISTICS:

        (A) LENGTH: 20 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:58:

```
Asp Gln Val Asp Val Lys Asp Ser Ala Asn His Glu Ile Lys Lys Val
1               5                   10                  15

Leu Val Pro Gly
              20
```

(2) INFORMATION FOR SEQ ID NO:59:

    (i) SEQUENCE CHARACTERISTICS:

        (A) LENGTH: 16 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:59:

```
            His Glu Ile Lys Lys Val Leu Val Pro Gly Cys His Gly Ser Glu Pro
            1               5                   10                  15
```

(2) INFORMATION FOR SEQ ID NO:60:

    (i) SEQUENCE CHARACTERISTICS:

        (A) LENGTH: 16 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:60:

```
            His Glu Ile Lys Lys Val Leu Val Pro Gly Glu His Gly Ser Glu Pro
            1               5                   10                  15
```

(2) INFORMATION FOR SEQ ID NO:61:

    (i) SEQUENCE CHARACTERISTICS:

        (A) LENGTH: 16 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:61:

```
            His Glu Ile Lys Lys Val Leu Val Pro Gly Ser His Gly Ser Glu Pro
            1               5                   10                  15
```

(2) INFORMATION FOR SEQ ID NO:62:

    (i) SEQUENCE CHARACTERISTICS:

        (A) LENGTH: 28 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:62:

```
            Lys Pro Phe Gln Leu Glu Ala Val Phe Glu Ala Asn Gln Asn Thr Lys
            1               5                   10                  15

            Thr Ala Lys Ile Glu Ile Lys Ala Ser Thr Asp Gly
                            20                  25
```

(2) INFORMATION FOR SEQ ID NO:63:

    (i) SEQUENCE CHARACTERISTICS:

(A) LENGTH: 28 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:63:

```
Gln Leu Glu Ala Val Phe Glu Ala Asn Gln Asn Thr Lys Thr Ala Lys
1               5                   10                  15
Ile Glu Ile Lys Ala Ser Ile Asp Gly Leu Glu Val
            20                  25
```

(2) INFORMATION FOR SEQ ID NO:64:

(i) SEQUENCE CHARACTERISTICS:

(A) LENGTH: 27 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:64:

```
Phe Glu Ala Asn Gln Asn Thr Lys Thr Ala Lys Ile Glu Ile Lys Ala
1               5                   10                  15
Ser Ile Asp Gly Leu Glu Val Asp Val Pro Gly
            20                  25
```

(2) INFORMATION FOR SEQ ID NO:65:

(i) SEQUENCE CHARACTERISTICS:

(A) LENGTH: 23 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:65:

```
Gln Asn Thr Lys Thr Ala Lys Ile Glu Ile Lys Ala Ser Ile Asp Gly
1               5                   10                  15
Leu Glu Val Asp Val Pro Gly
            20
```

(2) INFORMATION FOR SEQ ID NO:66:

(i) SEQUENCE CHARACTERISTICS:

(A) LENGTH: 26 amino acids

(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:66:

```
Pro Leu Val Lys Gly Gln Gln Tyr Asp Ile Lys Tyr Thr Trp Asn Val
1               5                   10                  15

Pro Lys Ile Ala Pro Lys Ser Glu Asn Val            .
            20                  25
```

(2) INFORMATION FOR SEQ ID NO:67:

(i) SEQUENCE CHARACTERISTICS:

(A) LENGTH: 26 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:67:

```
Pro Leu Val Lys Gly Gln Gln Tyr Asp Ile Lys Tyr Thr Tyr Asn Val
1               5                   10                  15

Pro Lys Ile Ala Pro Lys Ser Glu Asn Val
            20                  25
```

(2) INFORMATION FOR SEQ ID NO:68:

(i) SEQUENCE CHARACTERISTICS:

(A) LENGTH: 13 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:68:

```
Lys Ser Glu Asn Val Val Val Thr Val Lys Val Met Gly
1               5                   10
```

(2) INFORMATION FOR SEQ ID NO:69:

(i) SEQUENCE CHARACTERISTICS:

(A) LENGTH: 23 amino acids
(B) TYPE: amino acid
(D) TOPOLOGY: linear

(ii) MOLECULE TYPE: peptide
(v) FRAGMENT TYPE: N-terminal

(xi) SEQUENCE DESCRIPTION: SEQ ID NO:69:


```
        Thr Val Lys Val Met Gly Asp Asp Gly Val Leu Ala Cys Ala Ile Ala
        1               5                   10                  15

        Thr His Ala Lys Ile Arg Asp
                    20
```

(2) INFORMATION FOR SEQ ID NO:70:

(i) SEQUENCE CHARACTERISTICS:

(A) LENGTH: 23 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:70:


```
        Thr Val Lys Val Met Gly Asp Asp Gly Val Leu Ala Glu Ala Ile Ala
        1               5                   10                  15

        Thr His Ala Lys Ile Arg Asp
                    20
```

(2) INFORMATION FOR SEQ ID NO:71:

(i) SEQUENCE CHARACTERISTICS:

(A) LENGTH: 23 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:71:


```
        Thr Val Lys Val Leu Gly Asp Asp Gly Val Leu Ala Ser Ala Ile Ala
        1               5                   10                  15



                        Thr His Ala Lys Ile Arg Asp
                                    20
```

(2) INFORMATION FOR SEQ ID NO:72:

(i) SEQUENCE CHARACTERISTICS:

(A) LENGTH: 21 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:72:

```
Thr Ser Ala Cys Arg Ile Asn Ser Val Asn Val Pro Ser Glu Leu Asp
1               5                   10                  15

Leu Arg Ser Leu Arg
                20
```

(2) INFORMATION FOR SEQ ID NO:73:

(i) SEQUENCE CHARACTERISTICS:

(A) LENGTH: 27 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:73:

```
Glu Leu Asp Leu Arg Ser Leu Arg Thr Val Thr Pro Ile Arg Met Gln
1               5                   10                  15

Gly Gly Cys Gly Ser Cys Trp Ala Phe Ser Gly
                20                  25
```

(2) INFORMATION FOR SEQ ID NO:74:

(i) SEQUENCE CHARACTERISTICS:

(A) LENGTH: 29 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:74:

```
Thr Val Thr Pro Ile Arg Met Gln Gly Gly Cys Gly Ser Cys Trp Ala
1               5                   10                  15

Phe Ser Gly Val Ala Ala Thr Glu Ser Ala Tyr Leu Ala
                20                  25
```

(2) INFORMATION FOR SEQ ID NO:75:

(i) SEQUENCE CHARACTERISTICS:

(A) LENGTH: 21 amino acids
(B) TYPE: amino acid
(D) TOPOLOGY: linear

(ii) MOLECULE TYPE: peptide
(v) FRAGMENT TYPE: N-terminal

(xi) SEQUENCE DESCRIPTION: SEQ ID NO:75:

```
Val Ala Ala Thr Glu Ser Ala Tyr Leu Ala Tyr Arg Asn Thr Ser Leu
1               5            .    10              15

Asp Leu Ser Glu Gln
              20
```

(2) INFORMATION FOR SEQ ID NO:76:

(i) SEQUENCE CHARACTERISTICS:

(A) LENGTH: 22 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:76:

```
Tyr Arg Asn Thr Ser Leu Asp Leu Ser Glu Gln Glu Leu Val Asp Cys
1               5                 10              15

Ala Ser Gln His Gly Cys
              20
```

(2) INFORMATION FOR SEQ ID NO:77:

(i) SEQUENCE CHARACTERISTICS:

(A) LENGTH: 21 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:77:

```
Glu Leu Val Asp Cys Ala Ser Gln His Gly Cys His Gly Asp Thr Ile
1               5                 10              15

Pro Arg Gly Ile Glu
              20
```

(2) INFORMATION FOR SEQ ID NO:78:

(i) SEQUENCE CHARACTERISTICS:

(A) LENGTH: 28 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:78:

```
Gly Asp Thr Ile Pro Arg Gly Ile Glu Tyr Ile Gln Gln Asn Gly Val
1               5                   10                  15

Val Glu Glu Arg Ser Tyr Pro Tyr Val Ala Arg Glu
            20              25
```

(2) INFORMATION FOR SEQ ID NO:79:

   (i) SEQUENCE CHARACTERISTICS:

      (A) LENGTH: 25 amino acids
      (B) TYPE: amino acid
      (D) TOPOLOGY: linear

   (ii) MOLECULE TYPE: peptide
   (v) FRAGMENT TYPE: N-terminal
   (xi) SEQUENCE DESCRIPTION: SEQ ID NO:79:

```
Gln Asn Gly Val Val Glu Glu Arg Ser Tyr Pro Tyr Val Ala Arg Glu
1               5                   10                  15

Gln Arg Cys Arg Arg Pro Asn Ser Gln
            20              25
```

(2) INFORMATION FOR SEQ ID NO:80:

   (i) SEQUENCE CHARACTERISTICS:

      (A) LENGTH: 19 amino acids
      (B) TYPE: amino acid
      (D) TOPOLOGY: linear

   (ii) MOLECULE TYPE: peptide
   (v) FRAGMENT TYPE: N-terminal
   (xi) SEQUENCE DESCRIPTION: SEQ ID NO:80:

```
Gln Arg Cys Arg Arg Pro Asn Ser Gln His Tyr Gly Ile Ser Asn Tyr
1               5                   10                  15

Cys Gln Ile
```

(2) INFORMATION FOR SEQ ID NO:81:

   (i) SEQUENCE CHARACTERISTICS:

      (A) LENGTH: 22 amino acids
      (B) TYPE: amino acid
      (D) TOPOLOGY: linear

   (ii) MOLECULE TYPE: peptide
   (v) FRAGMENT TYPE: N-terminal
   (xi) SEQUENCE DESCRIPTION: SEQ ID NO:81:

```
His Tyr Gly Ile Ser Asn Tyr Cys Gln Ile Tyr Pro Pro Asp Val Lys
1               5                   10                  15
```

```
Gln Ile Arg Glu Ala Leu
20
```

(2) INFORMATION FOR SEQ ID NO:82:

(i) SEQUENCE CHARACTERISTICS:

(A) LENGTH: 24 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:82:

```
Tyr Pro Pro Asp Val Lys Gln Ile Arg Glu Ala Leu Phe Gln Thr His
1               5                   10                  15
Thr Ala Ile Ala Val Ile Ile Gly
            20
```

(2) INFORMATION FOR SEQ ID NO:83:

(i) SEQUENCE CHARACTERISTICS:

(A) LENGTH: 26 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:83:

```
Thr Gln Thr His Thr Ala Ile Ala Val Ile Ile Gly Ile Lys Asp Leu
1               5                   10                  15
Arg Ala Phe Gln His Tyr Asp Gly Arg Thr
            20                  25
```

(2) INFORMATION FOR SEQ ID NO:84:

(i) SEQUENCE CHARACTERISTICS:

(A) LENGTH: 26 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:84:

```
          Ile Lys Asp Leu Arg Ala Phe Gln His Tyr Asp Gly Arg Thr Ile Ile
          1               5                   10                  15

          Gln His Asp Asn Gly Tyr Gln Pro Asn Tyr
                          20                  25
```

(2) INFORMATION FOR SEQ ID NO:85:

   (i) SEQUENCE CHARACTERISTICS:

      (A) LENGTH: 15 amino acids
      (B) TYPE: amino acid
      (D) TOPOLOGY: linear

   (ii) MOLECULE TYPE: peptide
   (v) FRAGMENT TYPE: N-terminal
   (xi) SEQUENCE DESCRIPTION: SEQ ID NO:85:

```
          Asp Gly Arg Thr Ile Ile Gln His Asp Asn Gly Tyr Gln Pro Asn
          1               5                   10                  15
```

(2) INFORMATION FOR SEQ ID NO:86:

   (i) SEQUENCE CHARACTERISTICS:

      (A) LENGTH: 23 amino acids
      (B) TYPE: amino acid
      (D) TOPOLOGY: linear

   (ii) MOLECULE TYPE: peptide
   (v) FRAGMENT TYPE: N-terminal
   (xi) SEQUENCE DESCRIPTION: SEQ ID NO:86:

```
          Ile Ile Gln His Asp Asn Gly Tyr Gln Pro Asn Tyr His Ala Val Asn
          1               5                   10                  15

          Ile Val Gly Tyr Gly Ser Thr
                          20
```

(2) INFORMATION FOR SEQ ID NO:87:

   (i) SEQUENCE CHARACTERISTICS:

      (A) LENGTH: 22 amino acids
      (B) TYPE: amino acid
      (D) TOPOLOGY: linear

   (ii) MOLECULE TYPE: peptide
   (v) FRAGMENT TYPE: N-terminal
   (xi) SEQUENCE DESCRIPTION: SEQ ID NO:87:

```
His Ala Val Asn Ile Val Gly Tyr Gly Ser Thr Gln Gly Asp Asp Tyr
1               5                   10                  15

Trp Ile Val Arg Asn Ser
                20
```

(2) INFORMATION FOR SEQ ID NO:88:

    (i) SEQUENCE CHARACTERISTICS:

        (A) LENGTH: 24 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:88:

```
Gln Gly Asp Asp Tyr Trp Ile Val Arg Asn Ser Trp Asp Thr Thr Trp
1               5                   10                  15

Gly Asp Ser Gly Tyr Gly Tyr Phe
                20
```

(2) INFORMATION FOR SEQ ID NO:89:

    (i) SEQUENCE CHARACTERISTICS:

        (A) LENGTH: 26 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:89:

```
Gly Asp Ser Gly Tyr Gly Tyr Phe Gln Ala Gly Asn Asn Leu Met Met
1               5                   10                  15

Ile Glu Gln Tyr Pro Tyr Val Val Ile Met
                20                  25
```

(2) INFORMATION FOR SEQ ID NO:90:

    (i) SEQUENCE CHARACTERISTICS:

        (A) LENGTH: 29 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:90:

```
Thr Ser Ala Cys Arg Ile Asn Ser Val Asn Val Pro Ser Glu Leu Asp
1               5               10              15

Leu Arg Ser Leu Arg Thr Val Thr Pro Ile Arg Met Gln
            20              25
```

(2) INFORMATION FOR SEQ ID NO:91:

    (i) SEQUENCE CHARACTERISTICS:

        (A) LENGTH: 25 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:91:

```
Arg Ile Asn Ser Val Asn Val Pro Ser Glu Leu Asp Leu Arg Ser Leu
1               5               10              15

Arg Thr Val Thr Pro Ile Arg Met Gln
            20              25
```

(2) INFORMATION FOR SEQ ID NO:92:

    (i) SEQUENCE CHARACTERISTICS:

        (A) LENGTH: 29 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID-NO:92:

```
Ala Phe Ser Gly Val Ala Ala Thr Glu Ser Ala Tyr Leu Ala Tyr Arg
1               5               10              15

Asn Thr Ser Leu Asp Leu Ser Glu Gln Glu Leu Val Asp
            20              25
```

(2) INFORMATION FOR SEQ ID NO:93:

    (i) SEQUENCE CHARACTERISTICS:

        (A) LENGTH: 25 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:93:

```
Val Ala Ala Thr Glu Ser Ala Tyr Leu Ala Tyr Arg Asn Thr Ser Leu
1               5                   10                  15

Asp Leu Ser Glu Gln Glu Leu Val Asp
            20                  25
```

(2) INFORMATION FOR SEQ ID NO:94:

    (i) SEQUENCE CHARACTERISTICS:

        (A) LENGTH: 29 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:94:

```
Val Ala Ala Thr Glu Ser Ala Tyr Leu Ala Tyr Arg Asn Thr Ser Leu
1               5                   10                  15

Asp Leu Ser Glu Gln Glu Leu Val Asp Cys Ala Ser Gln
            20                  25
```

(2) INFORMATION FOR SEQ ID NO:95:

    (i) SEQUENCE CHARACTERISTICS:

        (A) LENGTH: 29 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:95:

```
Glu Tyr Ile Gln Gln Asn Gly Val Val Glu Glu Arg Ser Tyr Pro Tyr
1               5                   10                  15

Val Ala Arg Glu Gln Arg Cys Arg Arg Pro Asn Ser Gln
            20              25
```

(2) INFORMATION FOR SEQ ID NO:96:

    (i) SEQUENCE CHARACTERISTICS:

        (A) LENGTH: 29 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:96:

```
Asp Thr Ile Pro Arg Gly Ile Glu Tyr Ile Gln Gln Asn Gly Val Val
1               5                   10                  15

Glu Glu Arg Ser Tyr Pro Tyr Val Ala Arg Glu Gln Arg
                20              25
```

(2) INFORMATION FOR SEQ ID NO:97:

    (i) SEQUENCE CHARACTERISTICS:

        (A) LENGTH: 29 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:97:

```
Gln Ile Tyr Pro Pro Asp Val Lys Gln Ile Arg Glu Ala Leu Thr Gln
1               5                   10                  15

Thr His Thr Ala Ile Ala Val Ile Ile Gly Ile Lys Asp
                20                  25
```

(2) INFORMATION FOR SEQ ID NO:98:

    (i) SEQUENCE CHARACTERISTICS:

        (A) LENGTH: 22 amino acids
        (B) TYPE: amino acid
        (D) TOPOLOGY: linear

    (ii) MOLECULE TYPE: peptide
    (v) FRAGMENT TYPE: N-terminal


    (xi) SEQUENCE DESCRIPTION: SEQ ID NO:98:

```
Gln Ile Tyr Pro Pro Asp Val Lys Gln Ile Arg Glu Ala Leu Thr Gln
1               5                   10                  15

Thr His Thr Ala Ile Ala
                20
```

(2) INFORMATION FOR SEQ ID NO:99:

    (i) SEQUENCE CHARACTERISTICS:

        (A) LENGTH: 26 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:99:

```
Ile Ile Gly Ile Lys Asp Leu Arg Ala Phe Gln His Tyr Asp Gly Arg
1               5                   10                  15

Thr Ile Ile Gln His Asp Asn Gly Tyr Gln
              20                  25
```

(2) INFORMATION FOR SEQ ID NO:100:

    (i) SEQUENCE CHARACTERISTICS:

        (A) LENGTH: 25 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:100:

```
His Asp Asn Gly Tyr Gln Pro Asn Tyr His Ala Val Asn Ile Val Gly
1               5                   10                  15

Tyr Gly Ser Thr Gln Gly Asp Asp Tyr
              20                  25
```

(2) INFORMATION FOR SEQ ID NO:101:

    (i) SEQUENCE CHARACTERISTICS:

        (A) LENGTH: 29 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:101:

```
Asn Ile Val Gly Tyr Gly Ser Thr Gln Gly Asp Asp Tyr Trp Ile Val
1               5                   10                  15

Arg Asn Ser Trp Asp Thr Thr Trp Gly Asp Ser Gly Tyr
              20                  25
```

(2) INFORMATION FOR SEQ ID NO:102:

    (i) SEQUENCE CHARACTERISTICS:

        (A) LENGTH: 23 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:102:

```
        Asn Ile Val Gly Tyr Gly Ser Thr Gln Gly Asp Asp Tyr Trp Ile Val
        1               5                   10                  15

        Arg Asn Ser Trp Asp Thr Thr
                    20
```

(2) INFORMATION FOR SEQ ID NO:103:

    (i) SEQUENCE CHARACTERISTICS:

        (A) LENGTH: 20 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:103:

```
        Asp Gln Val Asp Val Lys Asp Cys Ala Asn Asn Glu Ile Lys Lys Val
        1               5                   10                  15

        Met Val Asp Gly
                    20
```

(2) INFORMATION FOR SEQ ID NO:104:

    (i) SEQUENCE CHARACTERISTICS:

        (A) LENGTH: 25 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:104:

```
        Asn Glu Ile Lys Lys Val Met Val Asp Gly Cys His Gly Ser Asp Pro
        1               5                   10                  15

        Cys Ile Ile His Arg Gly Lys Pro Phe
                    20                  25
```

(2) INFORMATION FOR SEQ ID NO:105:

    (i) SEQUENCE CHARACTERISTICS:

        (A) LENGTH: 29 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID No:105:

```
    His Gly Ser Asp Pro Cys Ile Ile His Arg Gly Lys Pro Phe Thr Leu
    1               5             .   10                  15

    Glu Ala Leu Phe Asp Ala Asn Gln Asn Thr Lys Thr Ala
                    20              25
```

(2) INFORMATION FOR SEQ ID NO:106:

(i) SEQUENCE CHARACTERISTICS:

(A) LENGTH: 19 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:106:

```
    His Arg Gly Lys Pro Phe Thr Leu Glu Ala Leu Phe Asp Ala Asn Gln
    1               5                   10                  15

    Asn Thr Lys
```

(2) INFORMATION FOR SEQ ID NO:107:

(i) SEQUENCE CHARACTERISTICS:

(A) LENGTH: 25 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:107:

```
    Thr Leu Glu Ala Leu Phe Asp Ala Asn Gln Asn Thr Lys Thr Ala Lys
    1               5                   10                  15

    Ile Glu Ile Lys Ala Ser Leu Asp Gly
                    20              25
```

(2) INFORMATION FOR SEQ ID NO:108:

(i) SEQUENCE CHARACTERISTICS:

(A) LENGTH: 26 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:108:

```
Gln Asn Thr Lys Thr Ala Lys Ile Glu Ile Lys Ala Ser Leu Asp Gly
1               5                   10                  15

Leu Glu Ile Asp Val Pro Gly Ile Asp Thr
            20                  25
```

(2) INFORMATION FOR SEQ ID NO:109:

    (i) SEQUENCE CHARACTERISTICS:

        (A) LENGTH: 27 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:109:

```
Lys Ile Glu Ile Lys Ala Ser Leu Asp Gly Leu Glu Ile Asp Val Pro
1               5                   10                  15

Gly Ile Asp Thr Asn Ala Cys His Phe Met Lys
            20                  25
```

(2) INFORMATION FOR SEQ ID NO:110:

    (i) SEQUENCE CHARACTERISTICS:

        (A) LENGTH: 26 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:110:

```
Leu Glu Ile Asp Val Pro Gly Ile Asp Thr Asn Ala Cys His Phe Met
1               5                   10                  15

Lys Cys Pro Leu Val Lys Gly Gln Gln Tyr
            20                  25
```

(2) INFORMATION FOR SEQ ID NO:111:

    (i) SEQUENCE CHARACTERISTICS:

        (A) LENGTH: 27 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:111:

```
Cys Pro Leu Val Lys Gly Gln Gln Tyr Asp Ala Lys Tyr Thr Trp Asn
1               5                   10                  15

            Val Pro Lys Ile Ala Pro Lys Ser Glu Asn Val
                        20                  25
```

(2) INFORMATION FOR SEQ ID NO:112:

    (i) SEQUENCE CHARACTERISTICS:

        (A) LENGTH: 26 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:112:

```
Asp Ala Lys Tyr Thr Trp Asn Val Pro Lys Ile Ala Pro Lys Ser Glu
1               5                   10                  15

Asn Val Val Val Thr Val Lys Leu Val Gly
            20                  25
```

(2) INFORMATION FOR SEQ ID NO:113:

    (i) SEQUENCE CHARACTERISTICS:

        (A) LENGTH: 35 amino acids
        (B) TYPE: amino acid
        (D) TOPOLOGY: linear

    (ii) MOLECULE TYPE: peptide
    (v) FRAGMENT TYPE: N-terminal
    (xi) SEQUENCE DESCRIPTION: SEQ ID NO:113:

```
Pro Lys Ile Ala Pro Lys Ser Glu Asn Val Val Val Thr Val Lys Leu
1               5                   10                  15

Val Gly Asp Asn Gly Val Leu Ala Cys Ala Ile Ala Thr His Ala Lys
            20                  25                  30

Ile Arg Asp
        35
```

(2) INFORMATION FOR SEQ ID NO:114:

    (i) SEQUENCE CHARACTERISTICS:

        (A) LENGTH: 25 amino acids
        (B) TYPE: amino acid
        (D) TOPOLOGY: linear

    (ii) MOLECULE TYPE: peptide

(v) FRAGMENT TYPE: N-terminal

(xi) SEQUENCE DESCRIPTION: SEQ ID NO:114:

```
Val Val Thr Val Lys Leu Val Gly Asp Asn Gly Val Leu Ala Cys Ala
1               5                   10                  15

Ile Ala Thr His Ala Lys Ile Arg Asp
            20                  25
```

(2) INFORMATION FOR SEQ ID NO:115:

(i) SEQUENCE CHARACTERISTICS:

(A) LENGTH: 28 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:115:

```
Lys Pro Phe Thr Leu Glu Ala Leu Phe Asp Ala Asn Gln Asn Thr Lys
1               5                   10                  15

Thr Ala Lys Ile Glu Ile Lys Ala Ser Leu Asp Gly
            20                  25
```

(2) INFORMATION FOR SEQ ID NO:116:

(i) SEQUENCE CHARACTERISTICS:

(A) LENGTH: 28 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:116:

```
Thr Leu Glu Ala Leu Phe Asp Ala Asn Gln Asn Thr Lys Thr Ala Lys
1               5                   10                  15

Ile Glu Ile Lys Ala Ser Leu Asp Gly Leu Glu Ile
            20                  25
```

(2) INFORMATION FOR SEQ ID NO:117:

(i) SEQUENCE CHARACTERISTICS:

(A) LENGTH: 22 amino acids
(B) TYPE: amino acid
(D) TOPOLOGY: linear

(ii) MOLECULE TYPE: peptide
(v) FRAGMENT TYPE: N-terminal

(xi) SEQUENCE DESCRIPTION: SEQ ID NO:117:

```
His Arg Asn Gln Ser Leu Asp Leu Ala Glu Gln Asp Leu Val Asp Cys
1               5                   10                  15

Ala Ser Gln His Gly Cys
                20
```

(2) INFORMATION FOR SEQ ID NO:118:

(i) SEQUENCE CHARACTERISTICS:

(A) LENGTH: 21 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:118:

```
Asp Leu Val Asp Cys Ala Ser Gln His Gly Cys His Gly Asp Thr Ile
1               5                   10                  15

Pro Arg Gly Ile Glu                       .
                20
```

(2) INFORMATION FOR SEQ ID NO:119:

(i) SEQUENCE CHARACTERISTICS:

(A) LENGTH: 29 amino acids
(B) TYPE: amino acid
(D) TOPOLOGY: linear

(ii) MOLECULE TYPE: peptide
(v) FRAGMENT TYPE: N-terminal
(xi) SEQUENCE DESCRIPTION: SEQ ID NO:119:

```
His Gly Ser Glu Pro Cys Ile Ile His Arg Gly Lys Pro Phe Gln Leu
1               5                   10                  15

Glu Ala Val Phe Glu Ala Val Gln Asn Thr Lys Thr Ala
                20                  25
                                                    .
```

**Description**

**Background of the Invention**

Recent reports have documented the importance of responses to the Group I (e.g., Der p I and Der f I) and Group II (e.g., Der p II and Der f II) protein allergens in house dust mite allergy. For example, it has been documented that over 60% of patients have at least 50% of their anti-mite antibodies directed towards these proteins (e.g., Lind, P. et al., Allergy, 39:259-274 (1984); van der Zee. J.S. et al., Journal Allergy and Clinical Immunology, 81:884-896 (1988)). It is possible that children show a greater degree of reactivity to the Group I and Group II allergens (Thompson, P.J., et al., Immunology, 64:301-314 (1988)). Allergy to mites of the genus Dermatophagoides (D.) is associated with conditions such as asthma, rhinitis and ectopic dermatitis. Two species, D. pteronyssinus and D. farinae, predominate and, as a result, considerable effort has been expended in trying to identify the allergens produced by these two species.

A concerted effort has been made to characterize by gene cloning the major allergens from both D. pteronyssinus and D. farinae. Consequently, several publications have reported the complete nucleotide sequences of several allergens including Der p I (Thomas, W.R., et al., International Archives of Allergy and Applied Immunology. 85: 127- 129 (1988); and Chua, K.Y., et al., Journal of Experimental Medicine, 167:175-182 (1988)), Der p II (Chua, K.Y., et al., International Archives of Allergy and Applied Immunology. 91:118-123 (1990)), Der f I (Dilworth, R.J., et al., Clinical and Experimental Allergy 21:25-32 (1891)), Der f II (Yuuki, T., et al., Japan Journal Allergol., 39:557-461 (1990); and Trudinger, M., et al., Clinical and Experimental Allergy, 21:33-37 (1991)) and a low molecular weight allergen (Ovey, E.R., et al., Journal of Experimental Medicine, 170:1457-1462 (1989)).

The published nucleotide sequences of cDNAs encoding Der p I and Der f I demonstrate that these two proteins are highly homologous at the amino acid level (81 % identity) and that the mature protein products are comprised of 222 and 223 residues, respectively (Chua. K.Y., et al., Journal of Experimental Medicine, 167:175-182 (1988): and Dilworth R.J., et al., supra)). The protein allergens Der p II and Der f II are both comprised of 129 residues, and are also highly homologous (88% identity) in amino acid sequence (Trudinger, M., et al. supra; Yuuki. T., et al. supra); Chua. K.Y.; et al. International Archives of Allergy and Applied Immunology 91:118-123 (1990)).

The isolation of cDNAs clones encoding Der p I and Der p II has permitted antibody binding studies on the recombinant antigens (Green. W.K., et al., International Archives of Allergy and Applied Immunology. 92:30-38 (1990); Chua, K.Y., et al., International Archives of Allergy and Applied Immunology, 91:124-129 (1990)). Complementary DNA fragments of Der p I have been expressed in E. coli and IgE binding studies with pooled human mite allergic IgE sera have demonstrated binding and non-binding regions throughout the molecule (Thomas, W.R., et al., In: Epitopes of Atopic Allergens, Proceedings of Workshop from XIV Congress of the European Academy of Allergy and Clinical Immunology, Berlin, Sept. 1989. pp 77-82). T cell epitopes of Der p I have been reported (O'Hehir, R.E., et al., Annual Review Immunology, 9:67-95 (1991); Stewart, G.A., et al., In: Epitopes of Atopic Allergens, Proceedings of Workshop from XIV Congress of the European Academy of Allergy and Clinical Immunology. Berlin, Sept. 1989. pp 41-47; Yessel, H., et al., In: T cell Activation in Health and Disease: Discrimination Between Immunity and Tolerance, Conference 22-26 Sept., 1990. Trinity College, Oxford, U.K. and Hessel, H., et al., Journal of Immunology, 148 (3): 738-745 (Feb. 1, 1992).

**Summary of the Invention**

The present invention provides isolated modified peptides of the major protein allergens of the genus Dermatophagoides as defined in the claims.

Also provided are peptides of the invention which have been further modified having similar or enhanced therapeutic properties as the corresponding naturally-occurring allergen or portion thereof, but having reduced side effects, as well as modified peptides having improved properties such as increased solubility and stability. Peptides of the invention are capable of modifying, in a house dust mite-sensitive individual to whom they are administered, the allergic response of the individual to a house dust mite allergen or an allergen immunologically cross-reactive with house dust mite allergen. Methods of treatment or of diagnosis of sensitivity to house dust mite in an individual and therapeutic compositions comprising one or more peptides of the invention are also described.

**Brief Description of the Drawings**

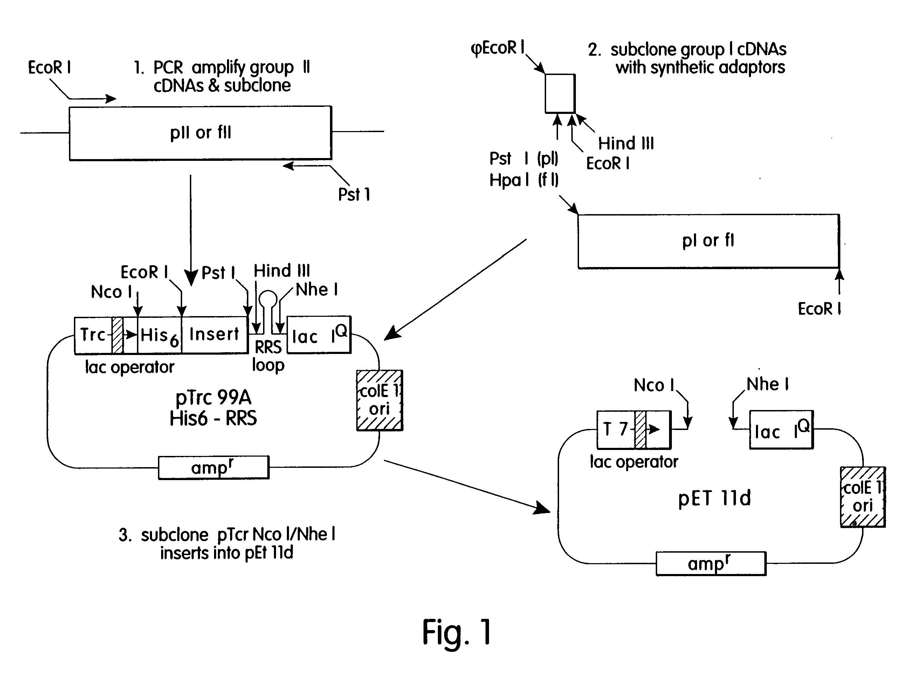

Fig. 1 shows the subcloning and expression of Group I and Group II allergens from D. pteronyssinus and D. farinae.

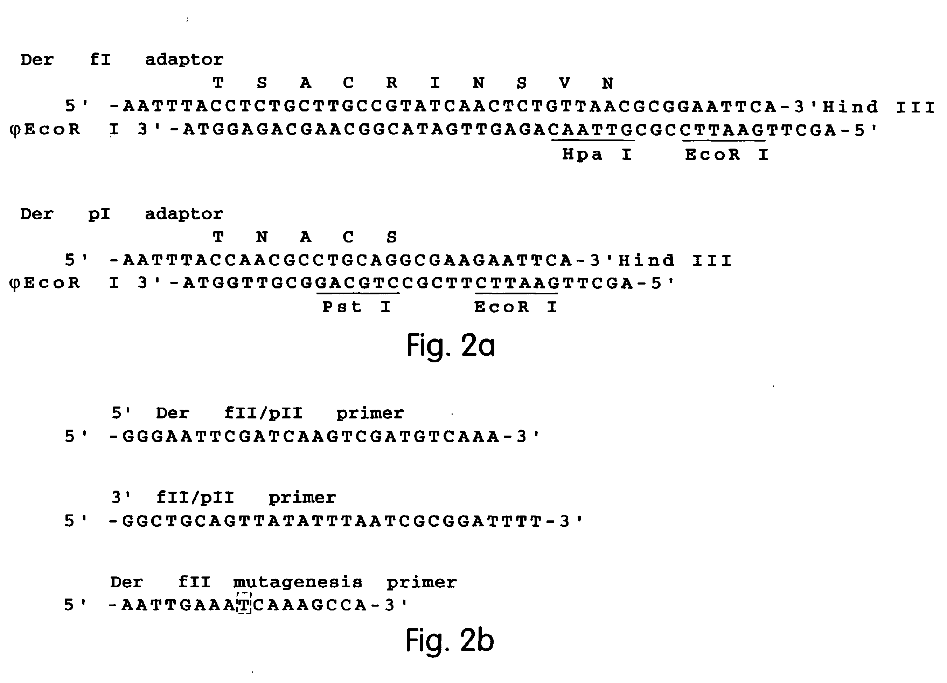

Fig. 2a shows adaptors used in the expression of Der p I and Der f I and Fig. 2b shows primers for amplification of Der f II and Der p II and a Der f II mutagenesis primer.

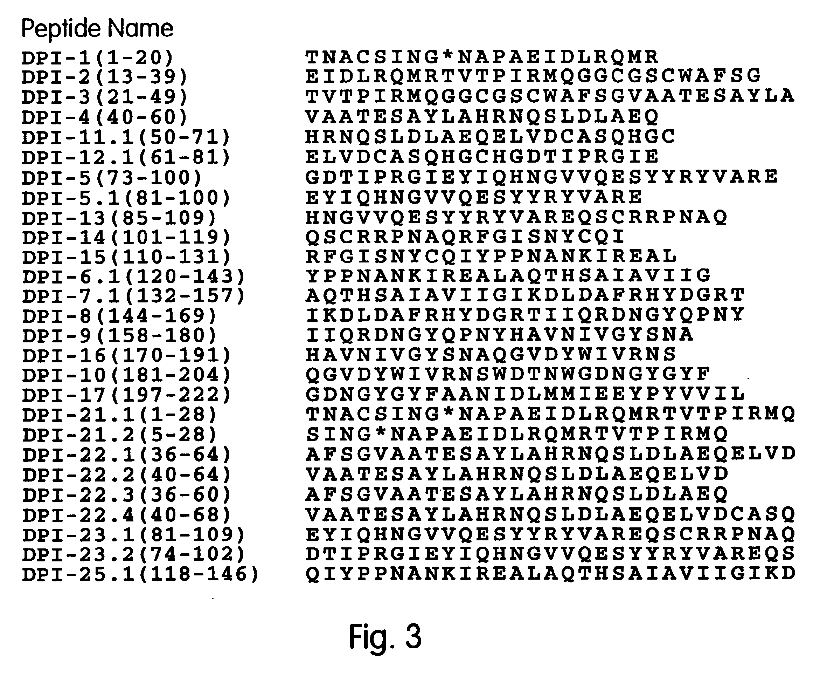

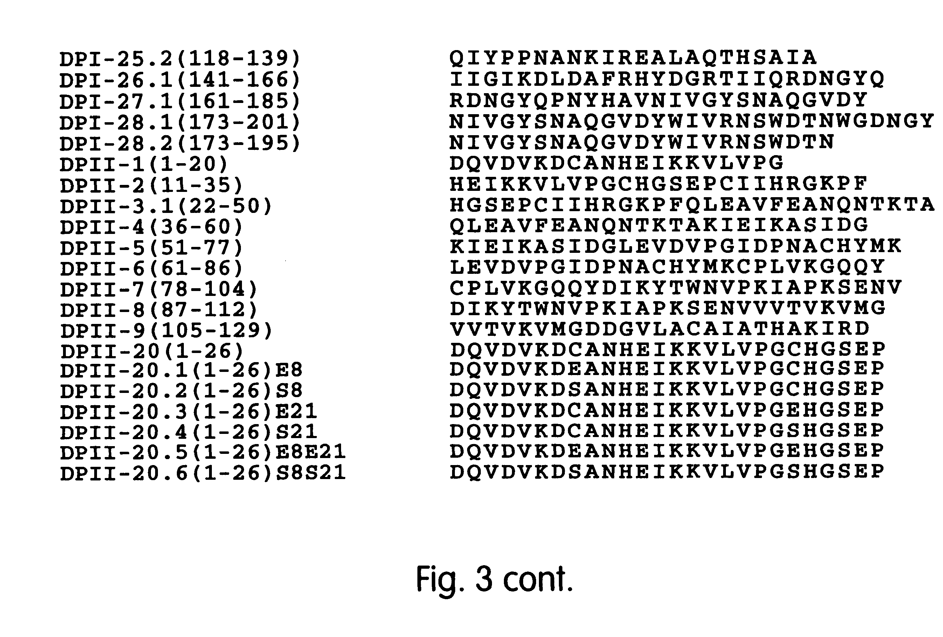

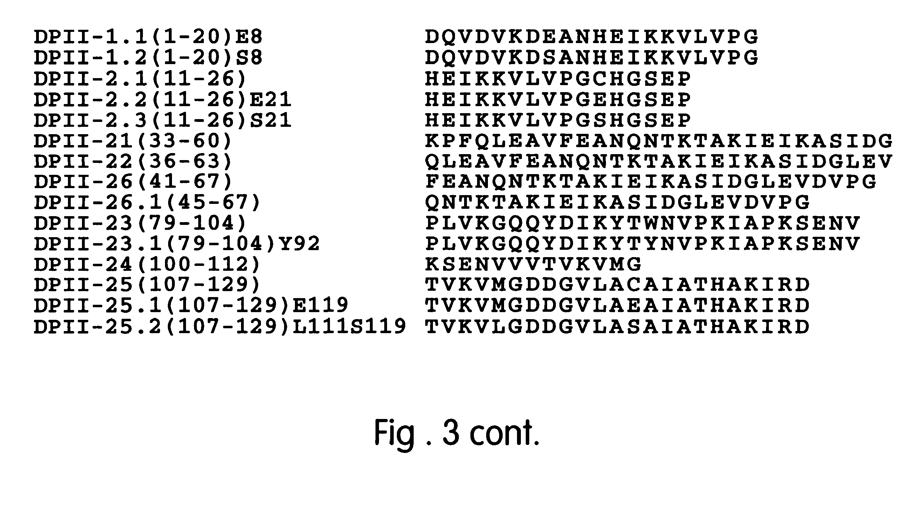

Fig. 3 shows various peptides of desired lengths derived from the Der p I and Der p II protein allergens.

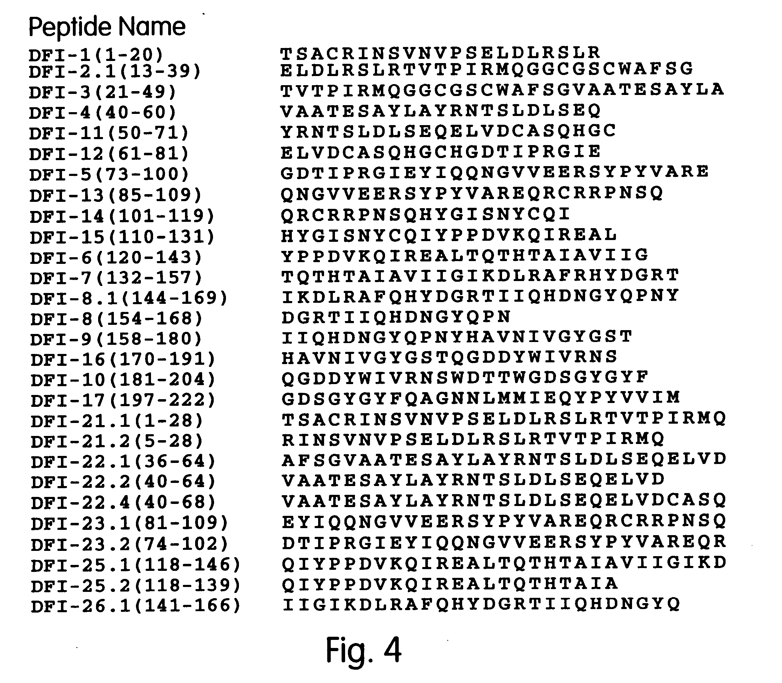

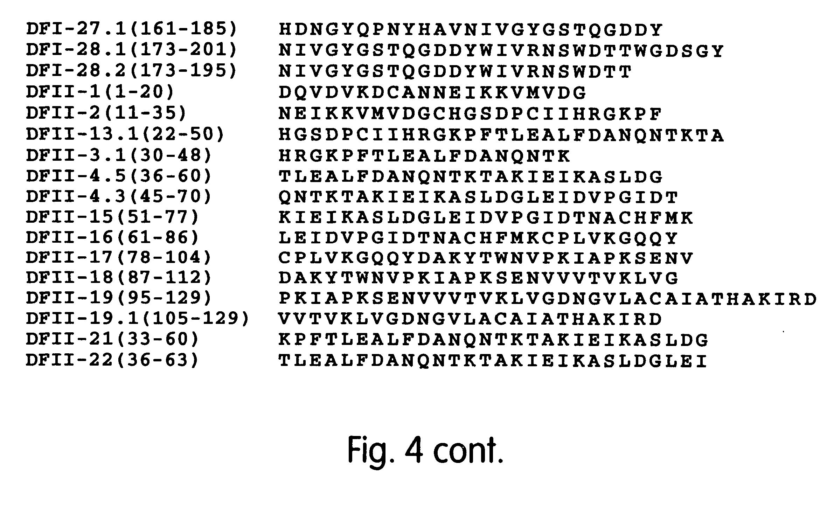

Fig. 4 shows various peptides of desired lengths derived from the Der f I and Der f II protein allergens.

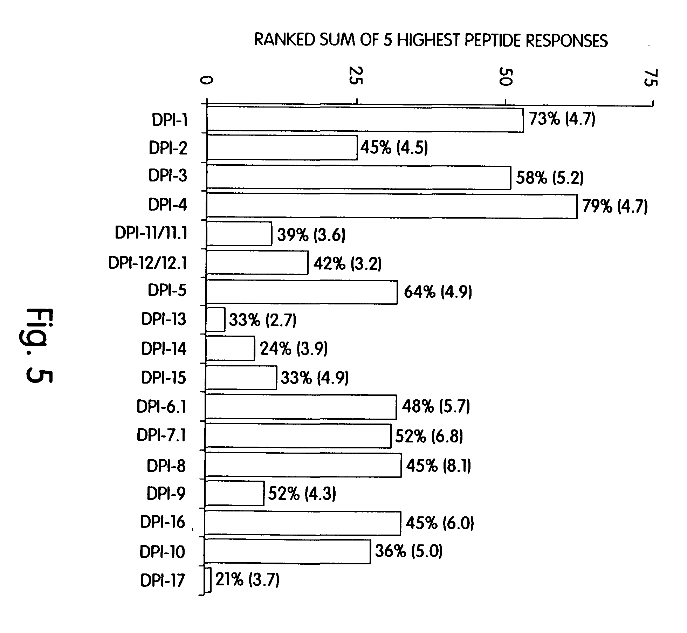

Fig. 5 is a graphic representation depicting the responses of T cell lines from 33 patients primed in vitro to either purified native (N) or recombinant (R) Der p I protein and analyzed for response to various overlapping Der p I peptides by percent of responses with a T cell Stimulation Index (S.I.) of at least 2 within the individuals tested, the mean T cell stimulation index of positive responses for the peptide and the ranked sum of peptide responses.

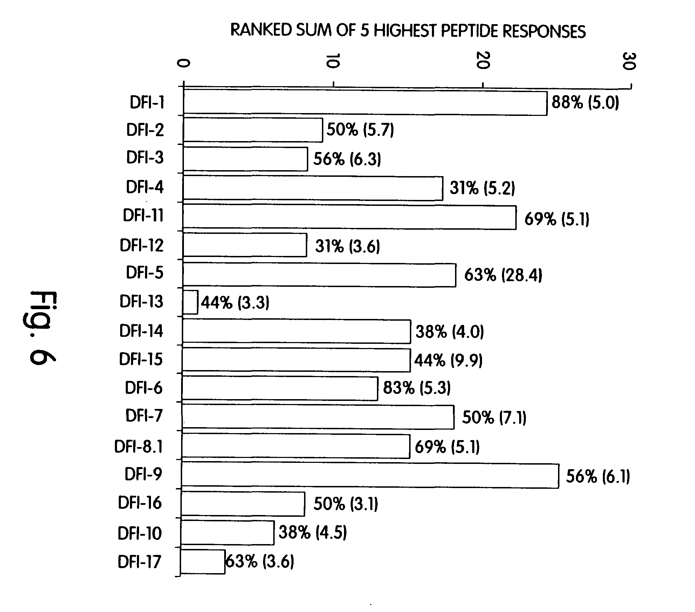

Fig. 6 is a graphic representation depicting the responses of T cell lines from 16 patients primed in vitro to the Der f I protein and analyzed for response to various overlapping Der f I peptides by percent of responses with an (S.I.) of at least 2 within the individuals tested, the mean T cell stimulation index of positive responses for the peptide and the ranked sum of peptide responses.

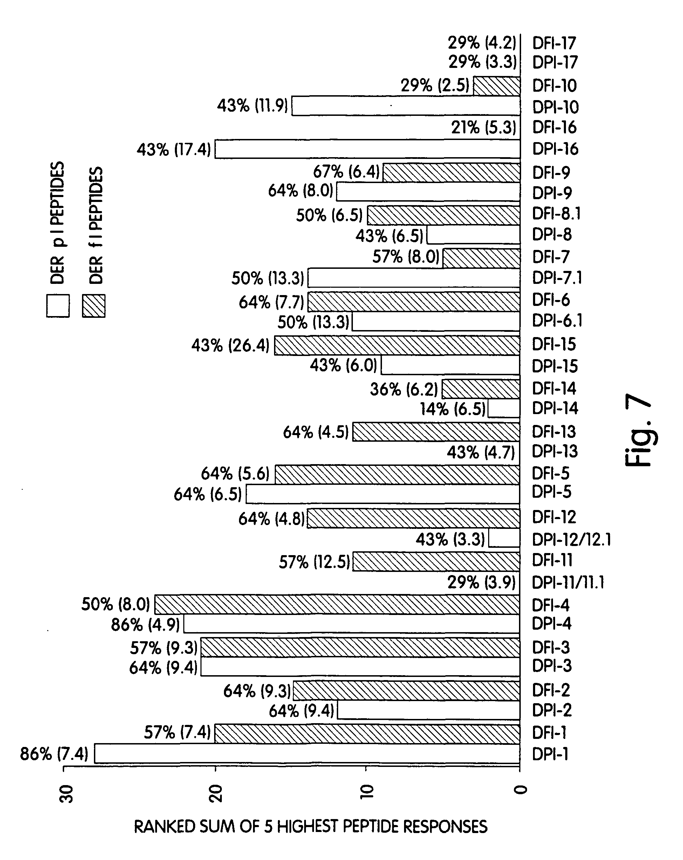

Fig. 7 is a graphic representation depicting the responses of T cell lines from 14 patients primed in vitro to the Der p I protein and analyzed for response to various overlapping Der p I peptides and substantially matching Der f I peptides by percent of responses with an S.I. of at least 2 within the individuals tested, the mean T cell stimulation index of positive responses for the peptide and the ranked sum of peptide responses.

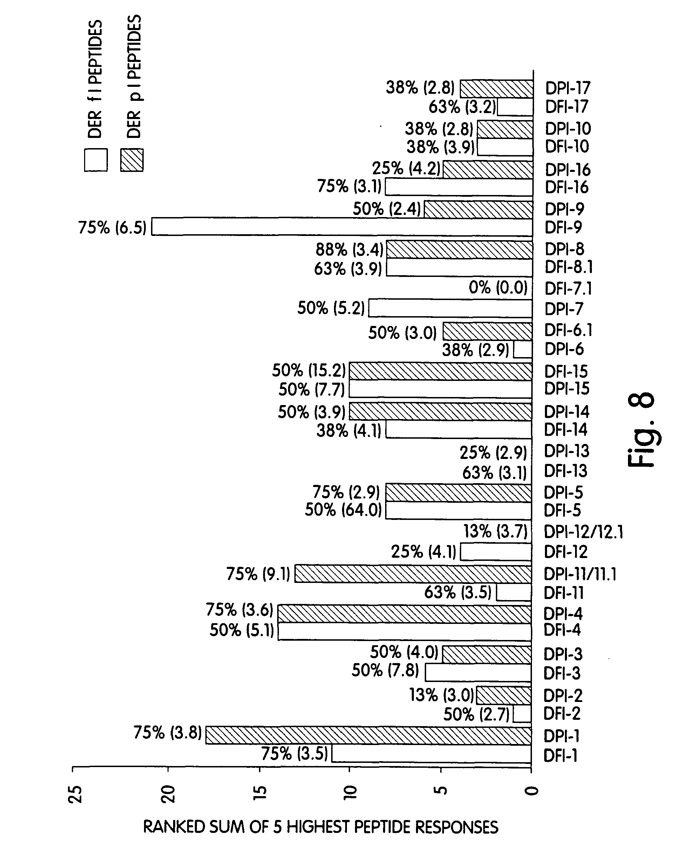

Fig. 8 is a graphic representation depicting the responses of T cell lines from 8 patients primed in vitro to the Der f I protein and analyzed for response to various overlapping Der f I peptides and substantially matching Der p I peptides by percent of responses with an S.I. of at least 2 within the individuals tested, the mean T cell stimulation index of positive responses for the peptide and the ranked sum of peptide responses.

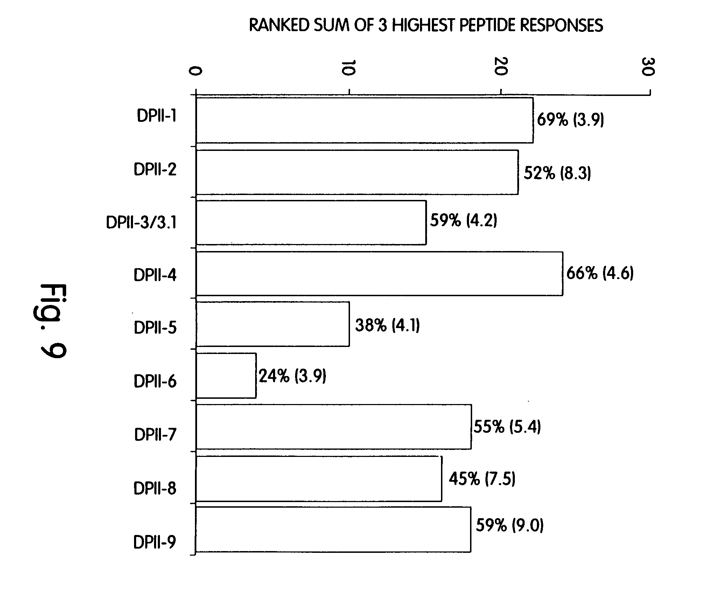

Fig. 9 is a graphic representation depicting the responses of T cell lines from 29 patients primed in vitro to the Der p II protein and analyzed for response to various overlapping Der p II peptides by percent of responses with an S.I. of at least 2 within the individuals tested, the mean T cell stimulation index of positive responses for the peptide and the ranked sum of peptide responses.

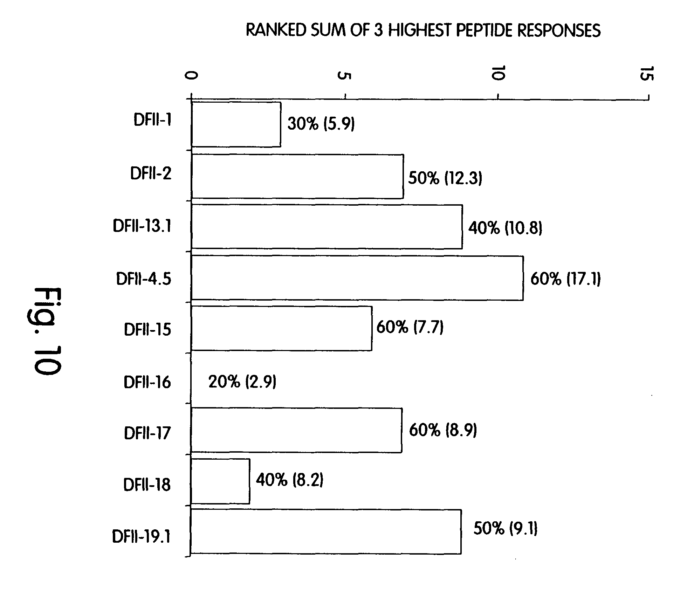

Fig. 10 is a graphic representation depicting the responses of T cell lines from 10 patients primed in vitro to the Der f II protein and analyzed for response to various overlapping Der f II peptides by percent of responses with an S.I. of at least 2 within the individuals tested, the mean T cell stimulation index of positive responses for the peptide and the ranked sum of peptide responses.

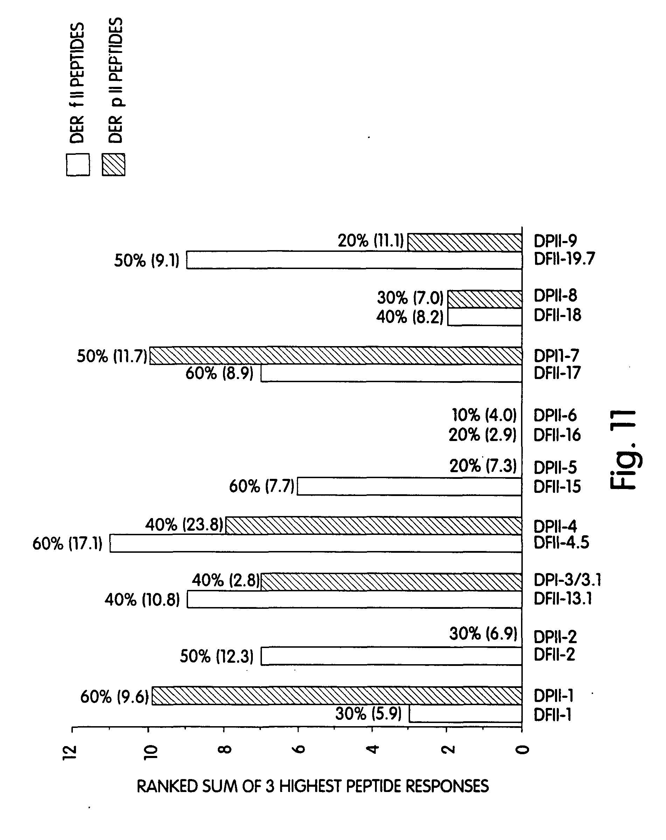

Fig. 11 is a graphic representation depicting the responses of T cell lines from 10 patients primed in vitro to the Der f II protein and analyzed for response to various overlapping Der f II peptides and substantially matching Der p II peptides by percent of responses with an S.I. of at least 2 within the individuals tested, the mean T cell stimulation index of positive responses for the peptide and the ranked sum of peptide responses.

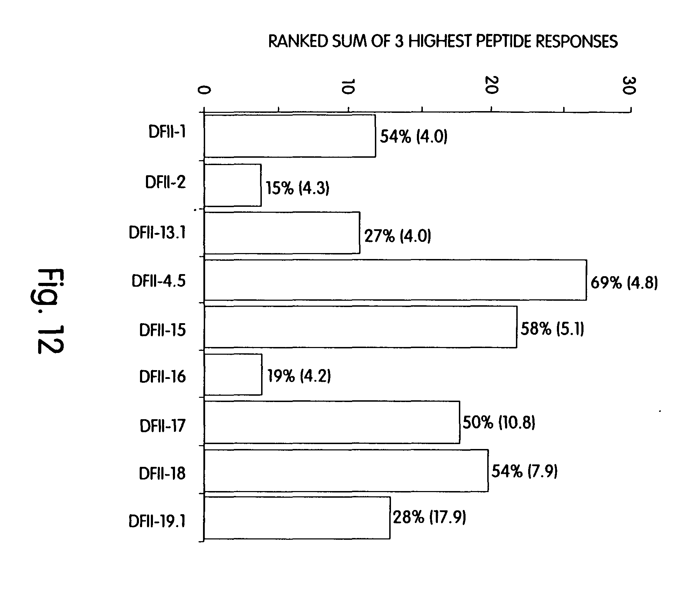

Fig. 12 is a graphic representation depicting the responses of T cell lines from 26 patients primed in vitro to the Der p II protein and analyzed for response to various overlapping Der f II peptides by percent of responses with an S.I. of at least 2 within the individuals tested, the mean T cell stimulation index of positive responses for the peptide and the ranked sum of peptide responses.

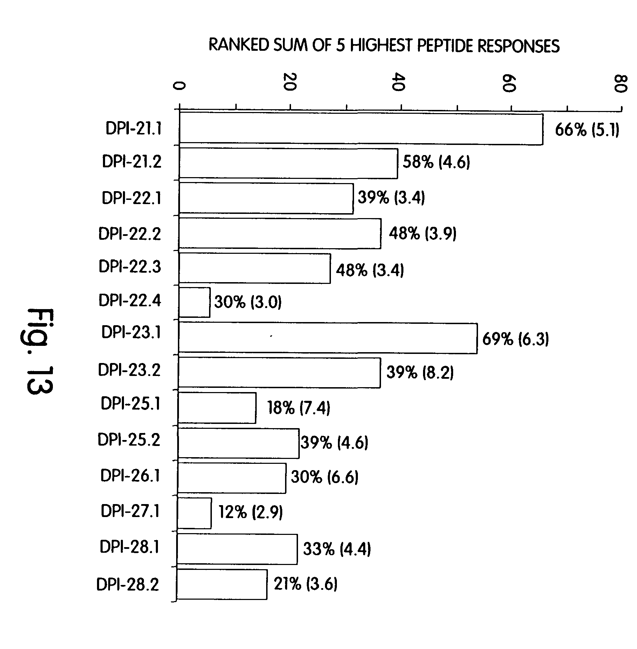

Fig. 13 is a graphic representation depicting the responses of T cell lines from 33 patients primed in vitro to the Der p I protein and analyzed for response to selected peptides of desired lengths derived from the Der p I protein allergen by percent of responses with an S.I. of at least 2 within the individuals tested, the mean T cell stimulation index of positive responses for the peptide and the ranked sum of peptide responses.

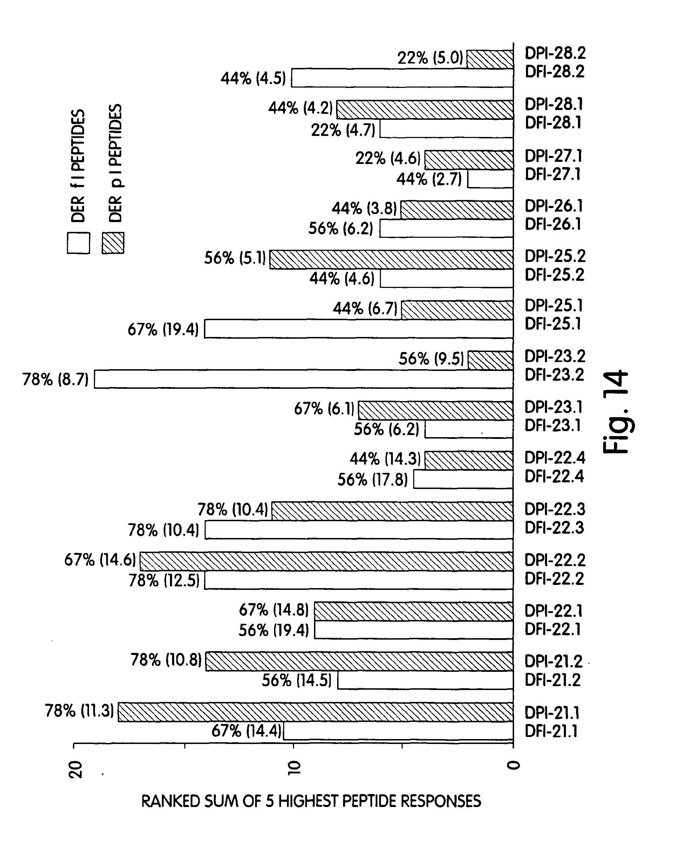

Fig. 14 is a graphic representation depicting the responses of T cell lines from 9 patients primed in vitro to the Der f I protein and analyzed for response to selected peptides of desired lengths derived from the Der f I protein allergen, by percent of responses with an S.I. of at least 2 within the individuals tested and the mean T cell stimulation index of positive responses for the peptide and the ranked sum of peptide responses.

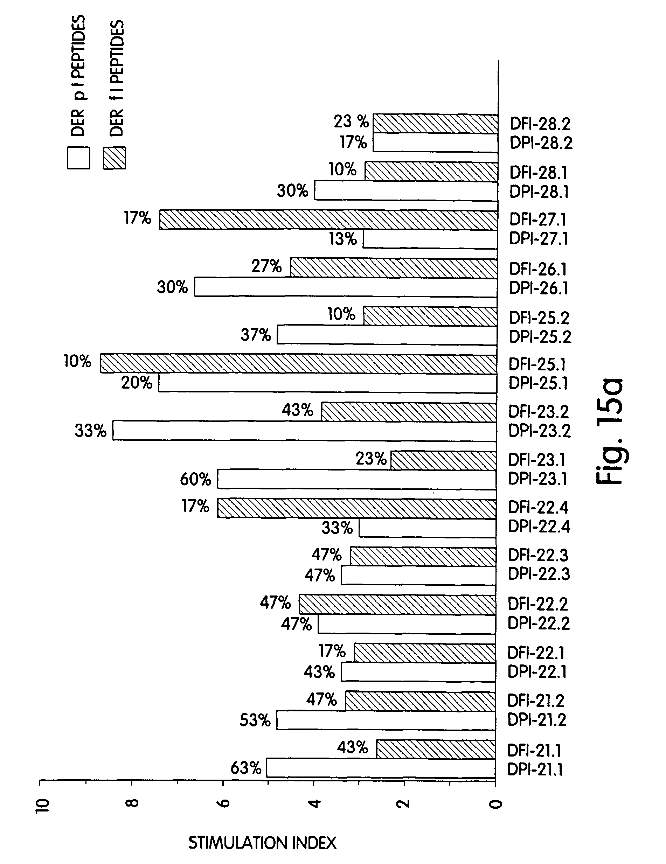

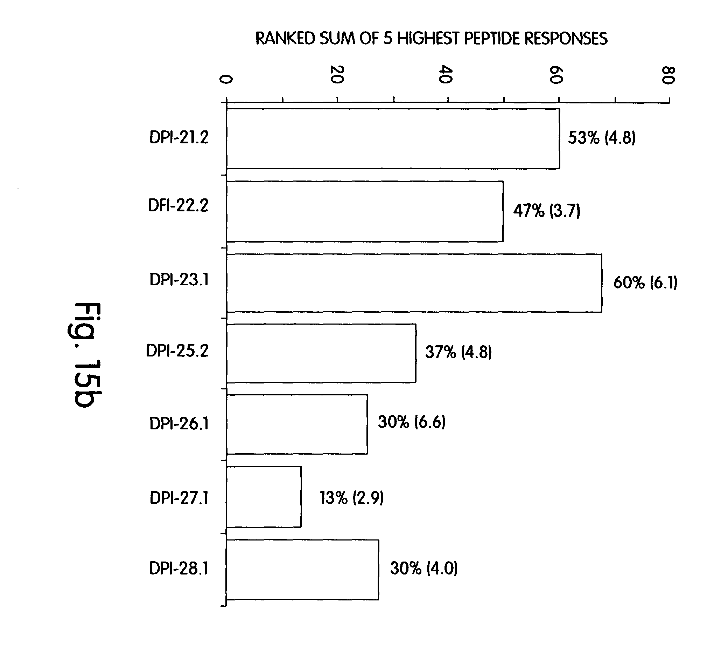

Fig. 15a is a graphic representation depicting the responses of T cell lines from 30 matched patients primed in vitro to the Der p I protein and analyzed for response to selected peptides of desired lengths derived from the Der p I and the Der f I protein allergens, by percent of responses with an S.I. of at least 2 within the individuals tested and the mean T cell stimulation index of positive responses for the peptide.

Fig. 15b is a graphic representation derived from the same data shown in Fig. 15a showing the response of Der p I primed T cells to preferred Der p I peptides analyzed by percent of response with an S.I. of at least two within the individuals tested (above each bar), the mean T cell stimulation index (above each bar in parenthesis) and the ranked sum of peptide responses.

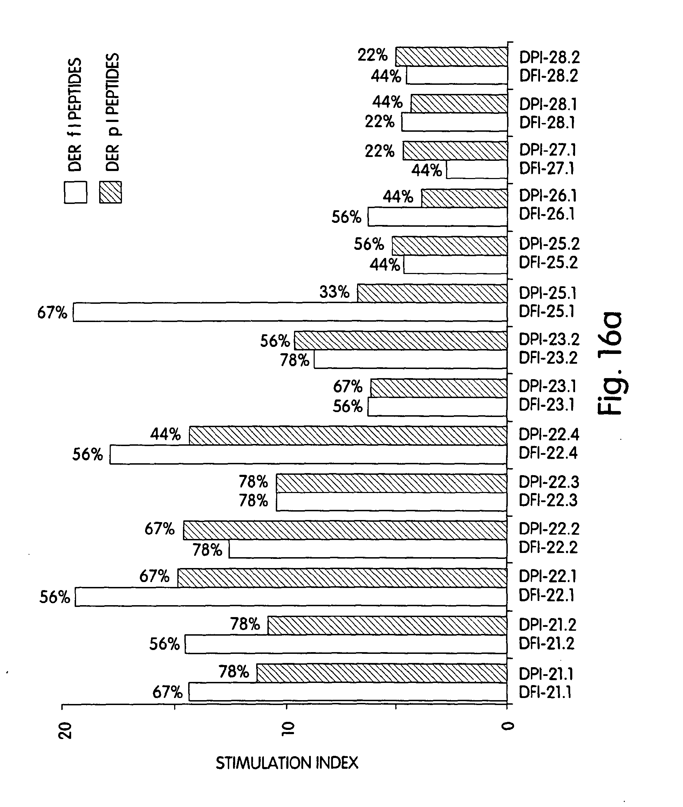

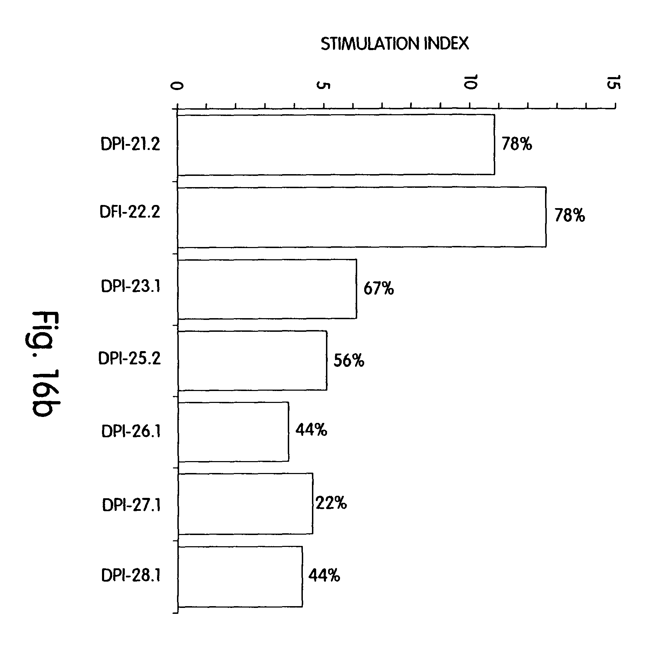

Fig. 16a is a graphic representation depicting the responses of T cell lines from 9 patients primed in vitro to the Der f I protein and analyzed for response to selected peptides of desired lengths derived from the Der f I and Der p I protein allergens, by percent of responses with an S.I. of at least 2 within the individuals tested and the mean T cell stimulation index of responses with an S.I. of at least 2 for the peptide.

Fig. 16b is a graphic representation derived from the same data shown in Fig. 16a showing the response of Der p I primed T cell lines to preferred Der f I and Der p I peptides by percent of responses with an S.I. of at least 2 within the individuals tested and the mean T cell stimulation index of responses with an S.I. of at least 2 for the peptide.

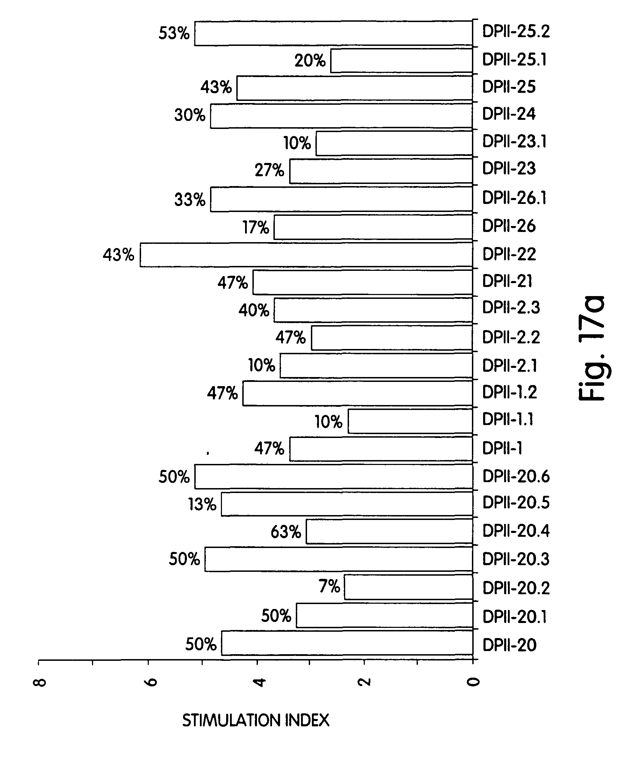

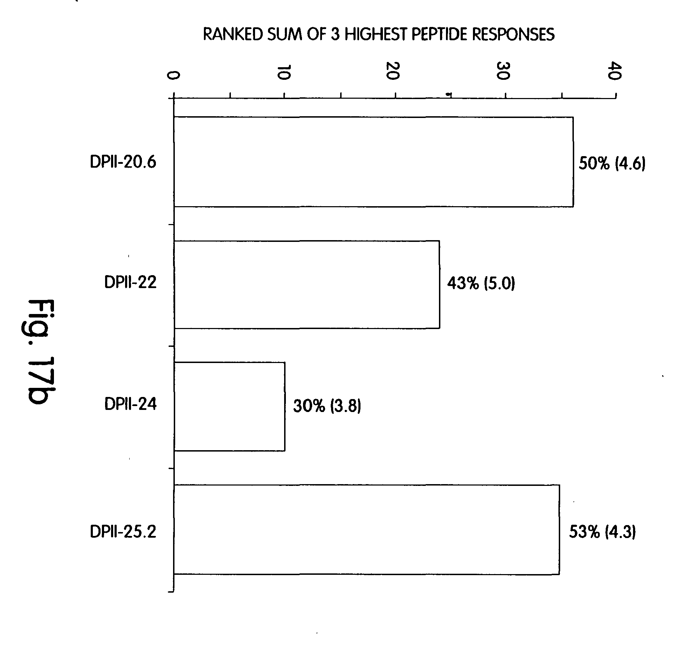

Fig. 17a is a graphic representation depicting the response of T cells from 29 patients primed in vitro to the Der p II protein and analyzed for response to selected peptides of desired lengths derived from the Der p II protein, by the T cell stimulation index of a response with an S.I. of at least 2 for the peptide.

Fig. 17b is a graphic representation depicting the response of 30 patients primed in vitro to the Der p II protein and analyzed for response to selected peptides derived from the Der p II protein by percent of responses with an S.I. of at least 2 within the individuals tested (above each bar), the mean T cell stimulation index (above each bar in parenthesis) and the ranked sum of peptide response (X-axis).

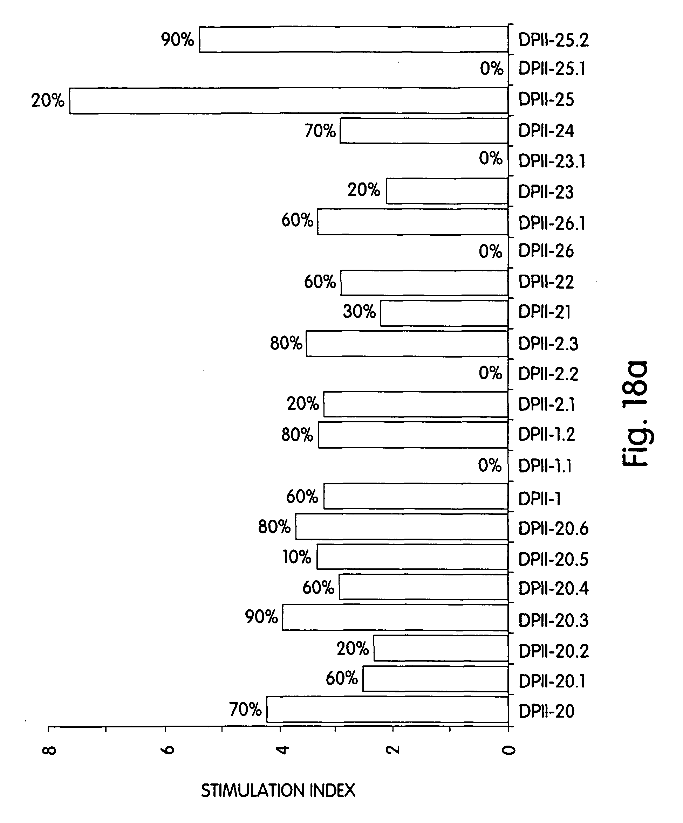

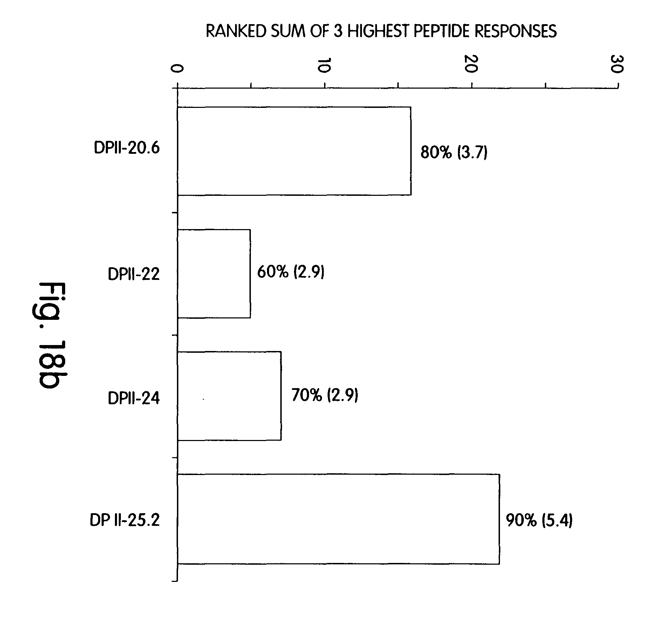

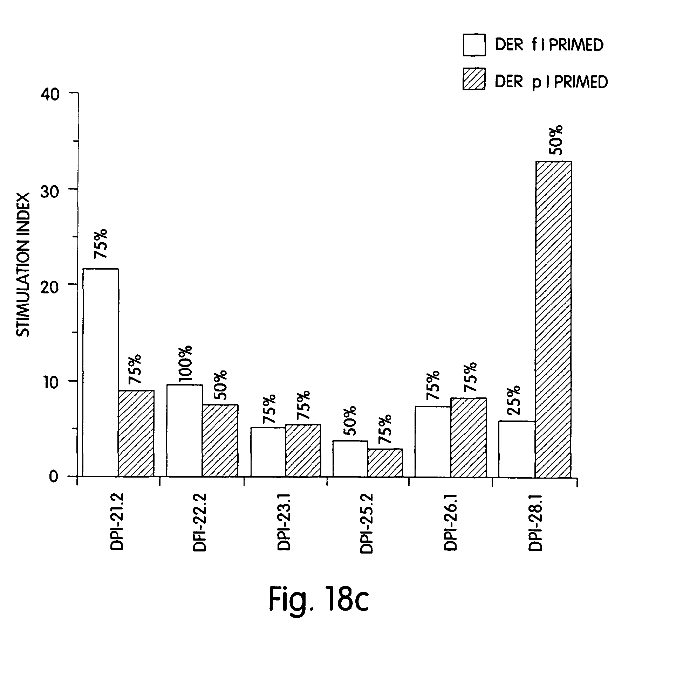

Fig. 18a is a graphic representation depicting the response of T cells from 10 patients primed in vitro to the Der f II protein and analyzed for response to selected peptides of desired lengths derived from the Der p II and Der f II protein, by the T cell stimulation index of a response with an S.I. of at least 2 for the peptide.

Fig. 18b is a graphic representation derived from the same data shown in Fig. 18a showing the response of Der f II primed T cell lines to preferred Der p II peptides analyzed by percent responses with an S.I. of at least 2 within the individuals tested (above each bar), the mean T cell stimulation index (above each bar in parenthesis) and the ranked sum of peptide responses (X-axis).

Fig. 18c is a graphic representation depicting the responses of T cell lines from 4 matched patients primed in vitro with mite group I allergen and analyzed for response to preferred Der p I and Der f I peptides by percent of responses with an S.I. of at least 2 within the individuals tested and the mean T cell stimulation index of positive responses for the peptide.

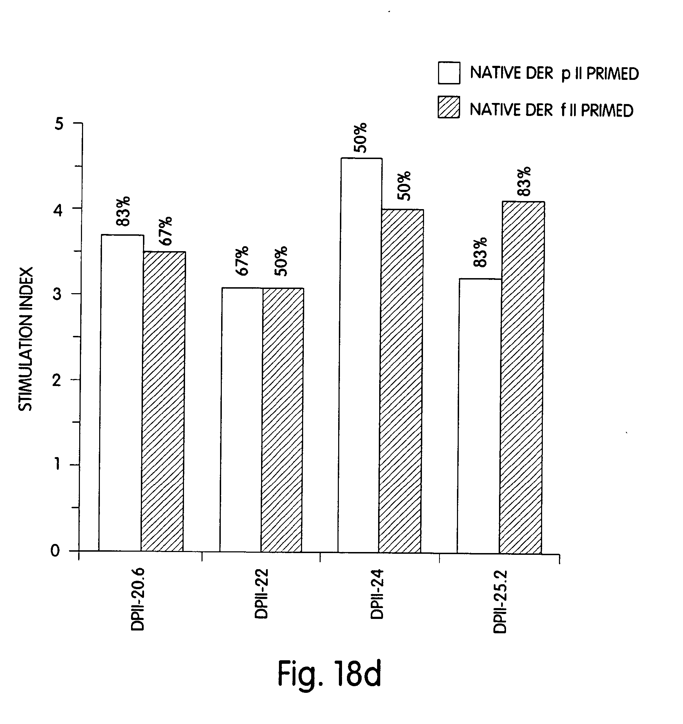

Fig. 18d is a graphic representation depicting the responses of T cell lines from 6 matched patients primed in vitro with mite group II allergen and analyzed for response to preferred Der p II peptides by percent of responses with an S.I. of at least 2 within the individuals tested and the mean stimulation index of positive responses for the peptide.

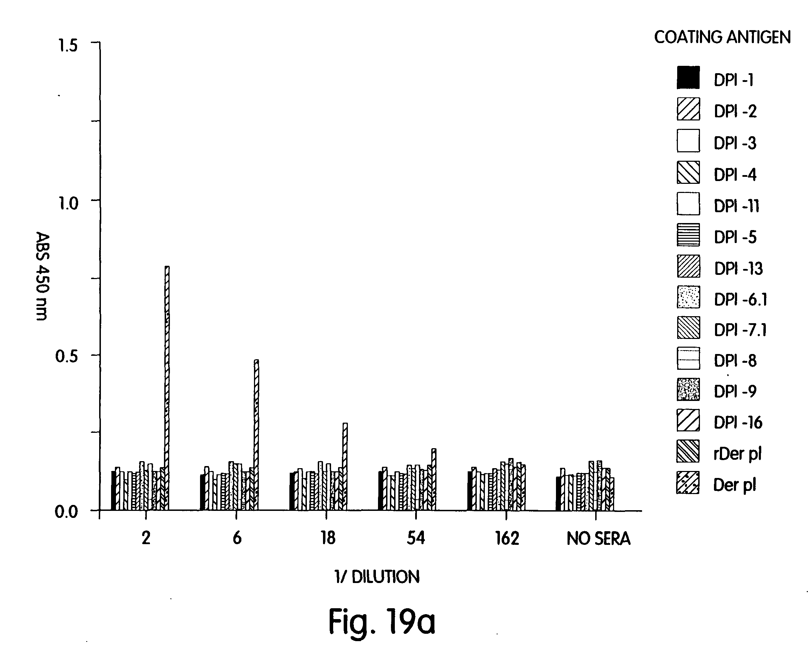

Fig. 19a-19b are graphic representations of the results of a direct binding assay of IgE to affinity purified and recombinant Der p I and Der p II proteins and certain Der p I and Der p II overlapping peptides.

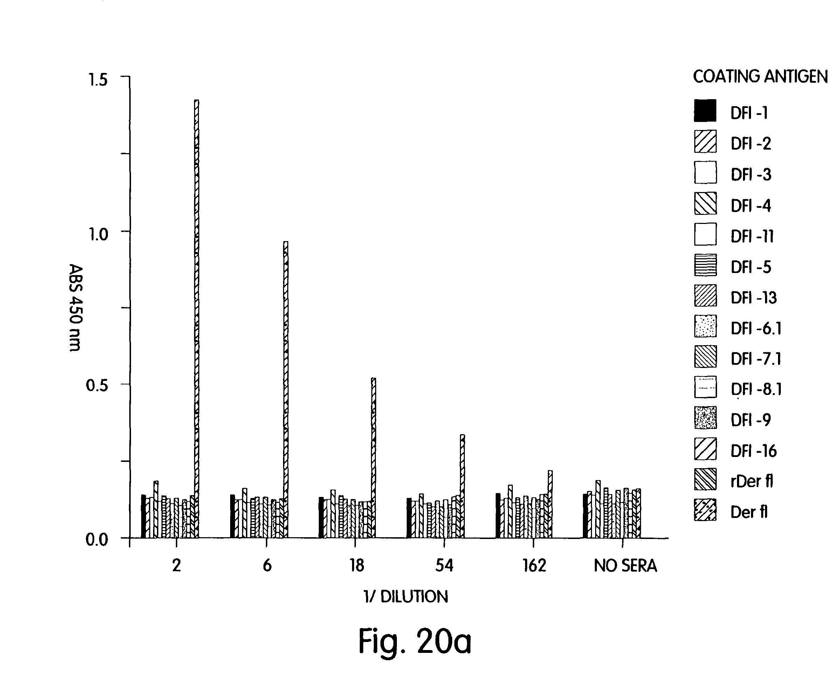

Fig. 20a-20b are graphic representations of the results of a direct binding assay of IgE to affinity purified and recombinant Der f I and Der f II proteins and certain Der f I and Der f II overlapping peptides.

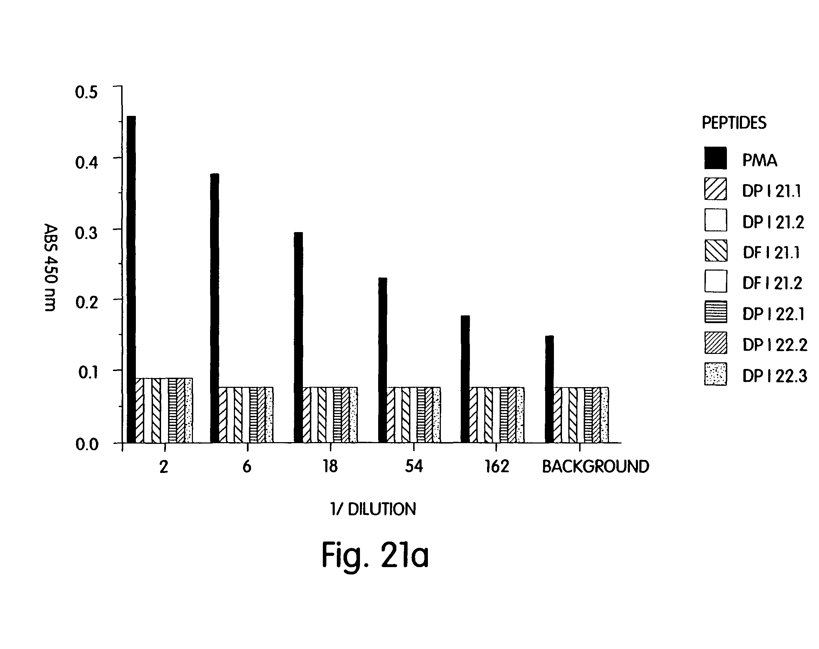

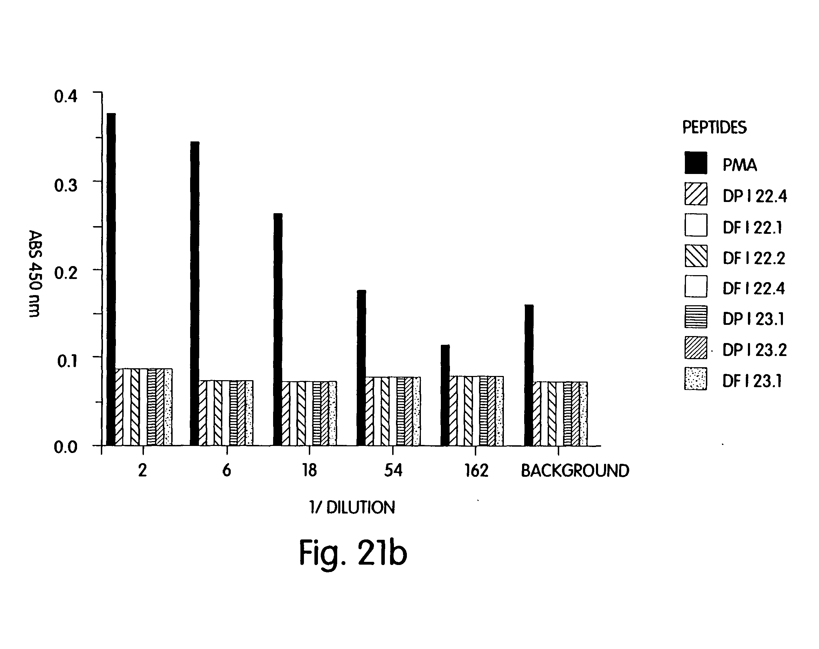

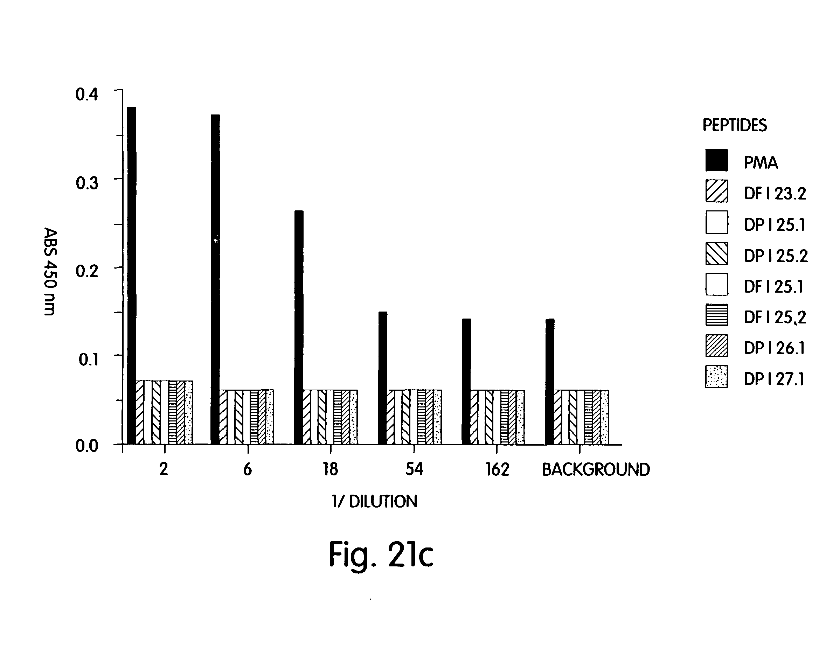

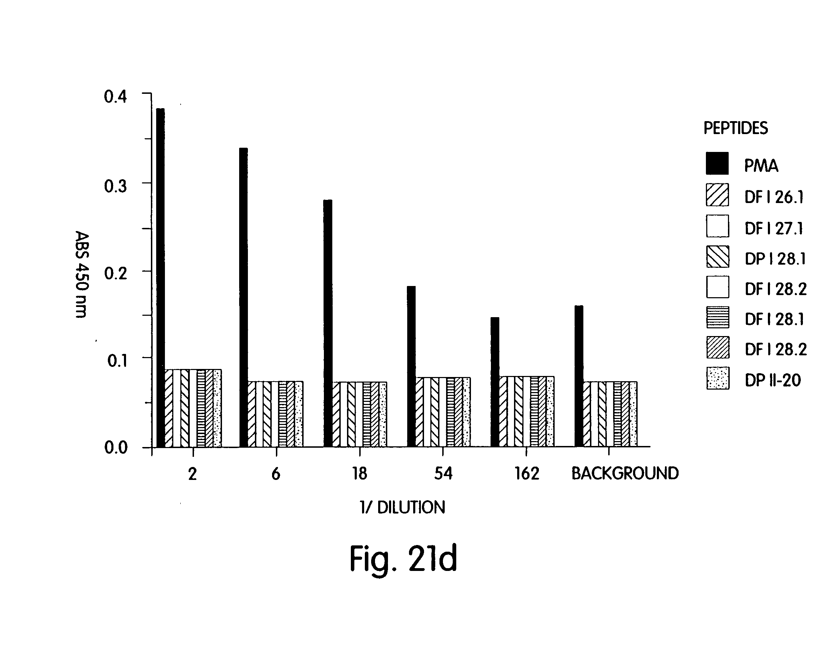

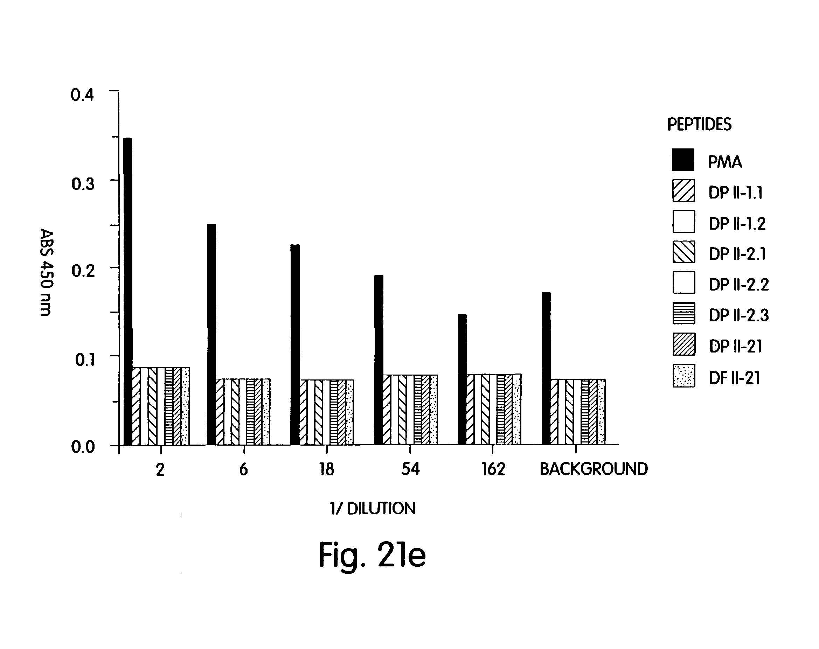

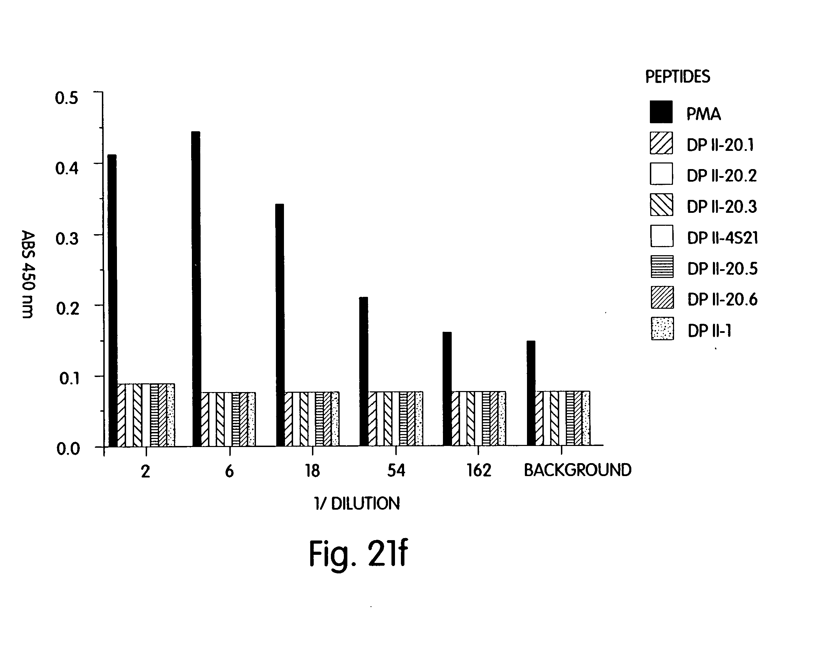

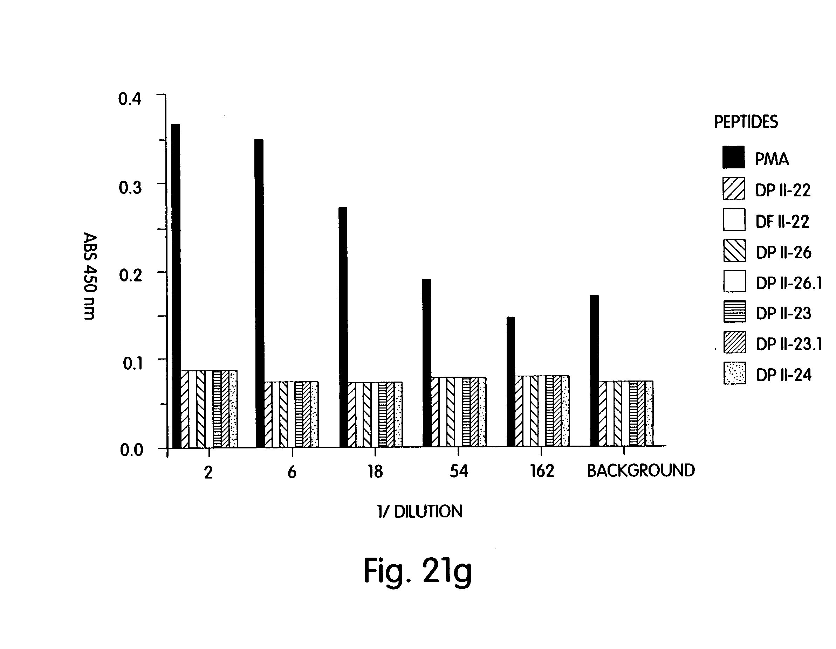

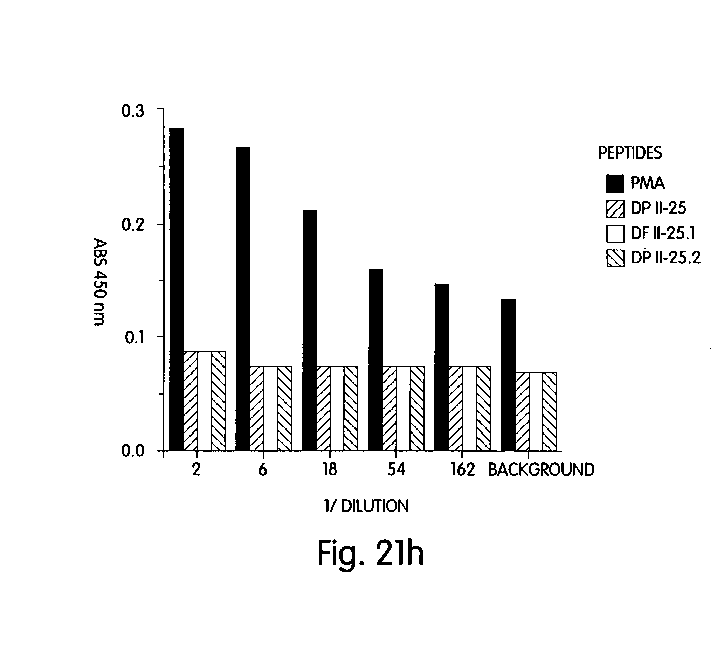

Fig. 21a-21h are graphic representations of the results of a direct binding assay of IgE to a mixture of biochemically purified mite allergens (PMA) and various peptides derived from Der p I, Der f I, Der p II and Der f II.

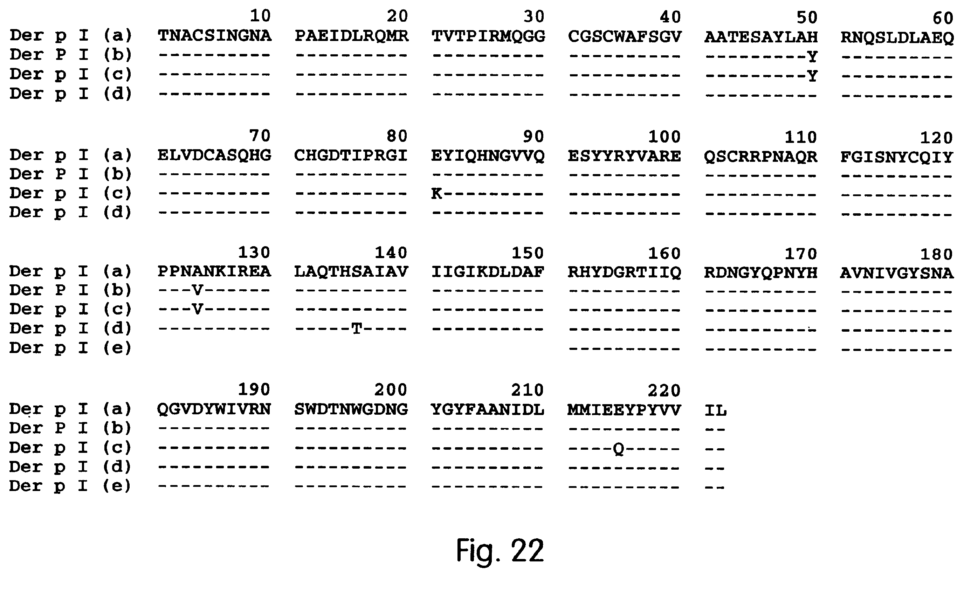

Fig. 22 is a composite alignment of the amino acid sequences of five Der p I clones (a)-(e) which illustrates polymorphism in the Der p I protein. The numbering refers to the sequence of the Der p I(a) clone. The symbol (-) is used to indicate that the amino acid residue of a Der p I clone is identical to the corresponding amino acid sequences of Der p I(a) at that position. The amino acid sequences of these clones indicate that there may be significant variation in Der p I. with five polymorphic amino acid residues found in the five sequences.

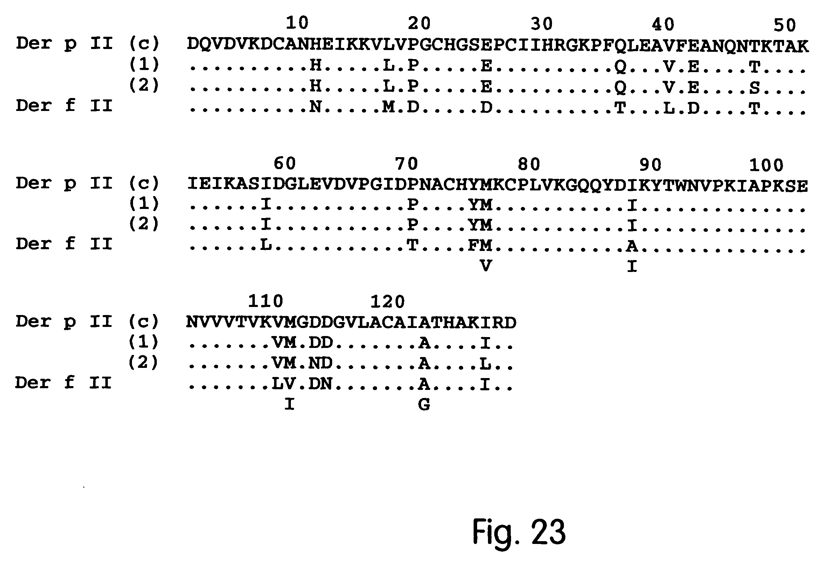

Fig. 23 is a composite alignment of the amino acid sequences of three Der p II clones (c), (1) and (2) which illustrates polymorphism in the Der p II protein. The numbering refers to the sequence of the Der p II(c) clone. The symbol (.) is used to indicate that the amino acid residue of a Der p II clone is identical to the corresponding amino acid residue of Der p II (c) at that position.

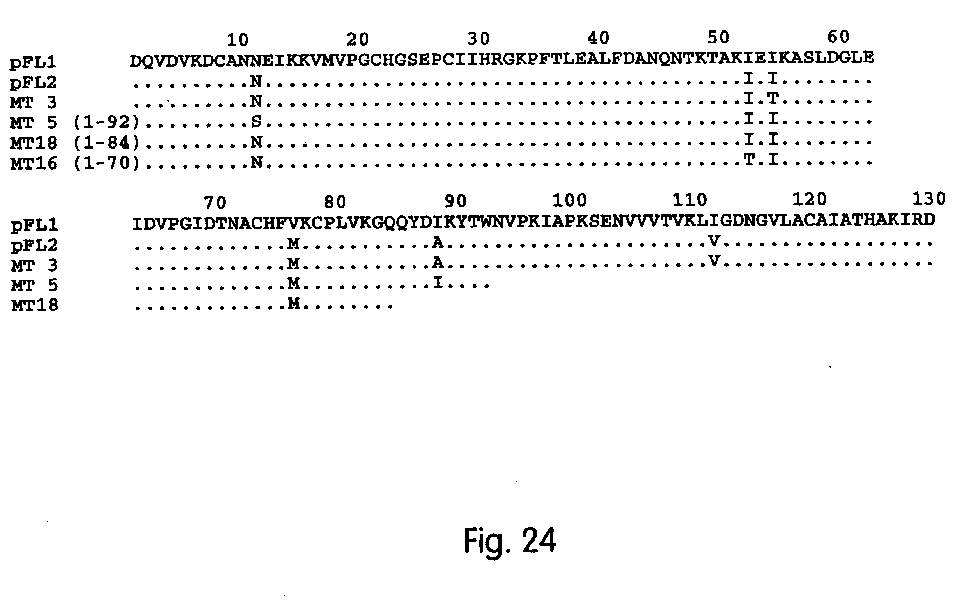

Fig. 24 is a composite alignment of the amino acid sequences of six Der f II clones (i.e., pFL1, pFL2, MT3, MT5, MT18 and MT16) which illustrates polymorphism is the Der f II protein. The numbering refers to the sequences of the Der f pLF1 clone. The symbol (.) is used to indicate that the amino acid residue of a Der f II clone is identical to the corresponding amino acid residue of Der f II pFL1 at that position.

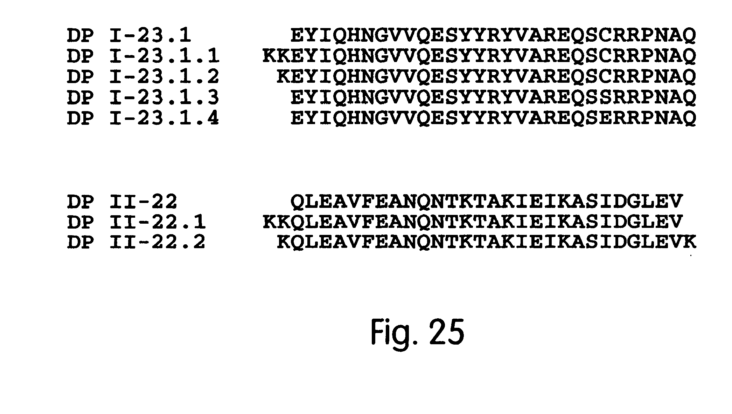

Fig. 25 shows the amino acid sequences of modified peptides.

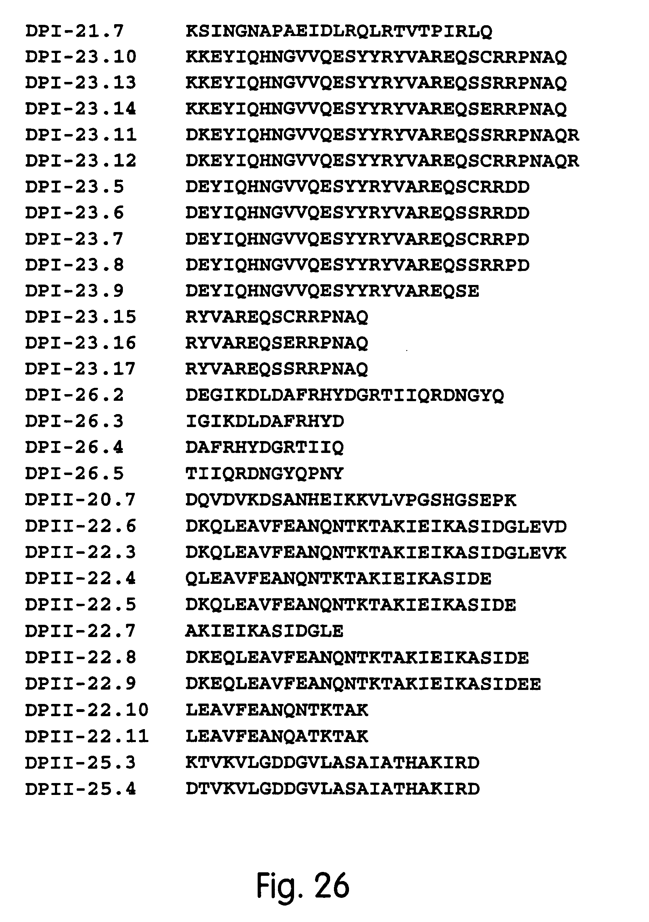

Fig. 26 shows the amino acid sequenced of modified peptides in accordance with the invention.

## Detailed Description of the Invention

The present invention provides isolated modified peptides derived from the major protein allergens of the genus Dermatophagoides as defined in the claims. As used herein, a peptide or fragment of a protein refers to an amino acid sequence having fewer amino acid residues than the entire amino acid sequence of the protein. As used herein, a modified peptide refers to a peptide deried from a protein allergen of the genus Dermatophagoides is which the amino acid sequence has been altered by amino acid substitution, deletion or addition.

Isolated peptides of the invention can be produced by recombinant DNA techniques in a host cell transformed with a nucleic acid having a sequence encoding such peptide. The isolated peptides of the invention can also be produced by chemical synthesis. In certain limited situations, isolated peptides can be produced by chemical cleavage of the protein allergen. When a peptide is produced by recombinant techniques, host cells transformed with a nucleic acid having a sequence encoding the peptide or the functional equivalent of the nucleic acid sequence are cultured in a medium suitable for the cells and peptides can be purified from cell culture medium, host cells, or both using techniques known in the art for purifying peptides and proteins including ion-exchange chromatography, gel filtration chromatography, ultrafiltration, electrophoresis or immunopurification with antibodies specific for the peptide, the protein allergen of the genus Dermatophagoides from which the peptide is derived, or a portion thereof. Isolated peptides of the invention are substantially free of cellular material or culture medium when produced by recombinant DNA techniques, or substantially free of chemical precursors or other chemicals when synthesized chemically.

The present invention provides expression vectors and host cells transformed to express the nucleic acid sequences of the invention. A nucleic acid sequence coding for a mite allergen, or at least one fragment thereof may be expressed in bacterial cells such as *E. coli,* insect cells (baculovirus), yeast, or mammalian cells such as Chinese hamster ovary cells (CHO). Suitable expression vectors, promoters, enhancers, and other expression control elements may be found in Sambrook et al. *Molecular Cloning: A Laboratory Manual,* second edition, Cold Spring Harbor Laboratory Press, Cold Spring Harbor, New York (1989). Other suitable expression vectors, promoters, enhancers, and other expression elements are known to those skilled in the art. Expression in mammalian, yeast or insect cells leads to partial or complete glycosylation of the recombinant material and formation of any inter- or intra-chain disulfide bonds. Suitable vectors for expression in yeast include YepSecl 1 (Baldari et al. (1987) *Embo J.* 6: 229-234); pMFa (Kurjan and Herskowitz (1982) *Cell* 30: 933-943); JRY88 (Schultz et al. (1987) *Gene* 54: 113-123) and pYES2 (Invitrogen Corporation, San Diego, CA). These vectors are freely available. Baculovirus and mammalian expression systems are also available. For example, a baculovirus system is commercially available (PharMingen, San Diego, CA) for expression in insect cells while the pMSG vector is commerically available (Pharmacia, Piscataway, NJ) for expression in mammalian cells.

For expression in *E. coli,* suitable expression vectors include, among others, pTRC (Amann et al. (1988) *Gene* 69: 301-315); pGEX (Amrad Corp., Melbourne, Australia); pMAL (N.E. Biolabs, Beverly, MA); pRIT5 (Pharmacia, Piscataway, NJ); pET-11d (Novagen, Madison, WI) Jameel et al., (1990) *J. Virol.* 64:3963-3966; and pSEM (Knapp et al. (1990) *BioTechniques* 8: 280-281). The use of pTRC, and pET-11d, for example, will lead to the expression of unfused protein. The use of pMAL, pRIT5 pSEM and pGEX will lead to the expression of allergen fused to maltose E binding protein (pMAL), protein A (pRIT5), truncated β-galactosidase (PSEM), or glutathione S-transferase (pGEX). When a mite protein allergen, fragment, or fragments thereof is expressed as a fusion protein, it is particularly advantageous to introduce an enzymatic cleavage site at the fusion junction between the carrier protein and a mite protein allergen or fragment thereof. A mite protein allergen or fragment thereof may then be recovered from the fusion protein through enzymatic cleavage at the enzymatic site and biochemical purification using conventional techniques for purification of proteins and peptides. Suitable enzymatic cleavage sites include those for blood clotting Factor Xa or thrombin for which the appropriate enzymes and protocols for cleavage are commercially available from, for example, Sigma Chemical Company, St. Louis, MO and N.E. Biolabs, Beverly, MA. The different vectors also have different promoter regions allowing constitutive or inducible expression with, for example, IPTG induction (PRTC, Amann et al., (1988) *supra*; pET-11d, Novagen. Madison. WI) or temperature induction (pRIT5, Pharmacia. Piscataway, NJ).

To obtain isolated peptides of the present invention, a mite allergen is divided into non-overlapping peptides of desired length or overlapping peptides of desired lengths as discussed in Example III which can be produced recombinantly, synthetically, or in certain limited situations, by chemical cleavage of the allergen. Peptides comprising at least one T cell epitope are capable of eliciting a T cell response, such as stimulation (i.e. proliferation or lymphokine secretion) and/or are capable of inducing T cell anergy (i.e., tolerization). To determine peptides comprising at least one T cell epitope, isolated peptides are tested by, for example, T cell biology techniques, to determine whether the peptides elicit a T cell response or induce T cell anergy. Those peptides found to elicit a T cell response or induce T cell anergy are defined as having T cell stimulating activity.

As discussed in the Examples, human T cell stimulating activity can be tested by culturing T cells obtained from an individual sensitive to a mite allergen, (i.e., an individual who has an IgE mediated immune response to a mite allergen) with a peptide derived from the allergen and determining whether proliferation of T cells occurs in response to the peptide as measured, e.g., by cellular uptake of tritiated thymidine. Stimulation indices for responses by T cells to peptides can be calculated as the maximum CPM in response to a peptide divided by the control CPM. A T cell stimulation index (S.I.) equal to or greater than two times the background level is considered "positive." Positive results are used to calculate the mean stimulation index for each peptide for the group of peptides tested. Preferred peptides of this invention comprise at least one T cell epitope and have a mean T cell stimulation index of greater than or equal to 2.0. A peptide having a T cell stimulation index of greater than or equal to 2.0 is considered useful as a therapeutic agent. Preferred peptides have a mean T cell stimulation index of at least 2.5, more preferably at least 3.5, even more preferably at least 4.0, and most preferably at least 5.0.

In addition, preferred peptides have a positivity index (P.I.) of at least about 100, more preferably at least 150, even more preferably at least about 200 and most preferably at least about 250. The positivity index for a peptide is determined by multiplying the mean T cell stimulation index by the percent of individuals, in a population of individuals sensitive to house dust mite (e.g., preferably at least 9 individuals, more preferably at least 16 individuals or more, more preferably at least 29 individuals or more, or even more preferably at least 30 individuals or more), who have T cells that respond to the peptide. Thus, the positivity index represents both the strength of a T cell response to a peptide (S.I.) and the frequency of a T cell response to a peptide in a population of individuals sensitive to house dust mite.

In order to determine precise T cell epitopes by, for example, fine mapping techniques, a peptide having T cell stimulating activity and thus comprising at least one T cell epitope as determined by T cell biology techniques is modified by addition or deletion of amino acid residues at either the amino or carboxy terminus of the peptide and tested to determine a change in T cell reactivity to the modified peptide. If two or more peptides which share an area of overlap in the native protein sequence are found to have human T cell stimulating activity, as determined by T cell biology techniques, additional peptides can be produced comprising all or a portion of such peptides and these additional peptides can be tested by a similar procedure. Following this technique, peptides are selected and produced recombinantly or synthetically. Peptides are selected based on various factors, including the strength of the T cell response to the peptide (e.g., stimulation index), the frequency of the T cell response to the peptide in a population of individuals sensitive to house dust mite, and the potential cross-reactivity of the peptide with other allergens of the genus Dermatophagoides. The physical and chemical properties of these selected peptides (e.g., solubility, stability) are examined to determine whether the peptides are suitable for use in therapeutic compositions or whether the peptides require modification as described herein. The ability of the selected peptides or selected modified peptides to stimulate human T cells (e.g., induce proliferation, lymphokine secretion) is determined.

Additionally, preferred peptides of the invention do not bind immunoglobulin E (IgE) or bind IgE to a substantially lesser extent than the protein allergen from which the peptide is derived binds IgE. The major complications of standard immunotherapy are IgE-mediated responses such as anaphylaxis. Immunoglobulin E is a mediator of anaphylactic reactions which result from the binding and cross-linking of antigen to IgE on mast cells or basophils and the release of mediators (e.g., histamine, serotonin, eosinophil chemotacic factors). Thus, anaphylaxis in a substantial percentage of a population of individuals sensitive to house dust mite could be avoided by the use in immunotherapy of a peptide or peptides which do not bind IgE in a substantial percentage (e.g., at least about 75%) of a population of individuals sensitive to a house dust mite allergen, or if the peptide binds IgE, such binding does not result in the release of mediators from mast cells or basophils. The risk of anaphylaxis could be reduced by the use in immunotherapy of a peptide or peptides which have reduced IgE binding. Moreover, peptides which have minimal IgE stimulating activity are desirable for therapeutic effectiveness. Minimal IgE stimulating activity refers to IgE production that is less than the amount of IgE production and/or IL-4 production stimulated by the native protein allergen (e.g., Der p I).

A peptide of the invention, when administered to a house-dust mite-sensitive individual, is capable of modifying the allergic response of the individual to the allergen. Particularly, peptides of the invention comprising at least one T cell epitope of a mite allergen or at least two regions derived from a mite allergen, each comprising at least one T cell epitope, when administered to an individual sensitive to house dust mite are capable of modifying T cell response of the individual to the allergen. As used herein, modification of the allergic response of an individual to a house dust mite allergen can be defined as non-responsiveness or diminution in symptoms upon exposure to a mite allergen, as determined by standard clinical procedures (see e.g., Varney et al., British Medical Journal 302: 265-269 (1990)) including diminution in mite protein allergen induced asthmatic symptoms. As referred to herein, a diminunition in symptoms includes any reduction in allergic response of an individual to the allergen after the individual has completed a treatment regimen with a peptide or protein of the invention. This diminution may be subjective (i.e. the patient feels more comfortable in the presence of the allergen). Diminution in symptoms can be determined clinically as well, using standard skin tests as is known in the art.

As a result of the work described herein, modified peptides derived from mite allergens comprising at least one T cell epitope have been produced. T cell epitopes are believed to be involved in initiation and perpetuation of the immune responses to mite allergen(s) which are responsible for the clinical symptoms of mite allergy. These T cell epitopes are thought to trigger early events at the level of the T helper cell by binding to an appropriate HLA molecule on the surface of an antigen presenting cell and stimulating the relevant T cell subpopulation. These events lead to T cell proliferation, lymphokine secretion, local inflammatory reactions, the recruitment of additional immune cells to the site, and activation of the B cell cascade leading to production of antibodies. One isotype of these antibodies, IgE, is fundamentally important in the development of allergic symptoms and its production is influenced early in the cascade of events at the level of the T helper cell, by the nature of the lymphokines secreted. A T cell epitope is the basic element or smallest unit of recognition by a T cell receptor where the epitope comprises amino acid residues essential to receptor recognition which may be contiguous and/or non-contiguous in the amino acid sequence of the protein. Amino acid sequences which mimic those of T cell epitopes and which modify the allergic response to protein allergens of the genus Dermatophagoides are within the scope of this invention.

Exposure of mite allergic patients to peptides of the present invention may tolerize or anergize appropriate T cell subpopulations such that they become unresponsive to mite allergen(s) and do not participate in mounting an immune response upon such exposure. In addition, administration of a peptide of the present invention may modify the lymphokine secretion profile as compared with exposure to the naturally-occurring mite protein allergen or portion thereof (e.g., result in a decrease of IL-4 and/or an increase in IL-2). Furthermore, exposure to a peptide of the invention may influence T cell subpopulations which normally participate in the response to mite allergen(s) such that these T cells are drawn away from the site(s) of normal exposure to the allergen (e.g., nasal mucosa, skin, and lung) towards the site(s) of therapeutic administration of the peptide. This redistribution of T cell subpopulations may ameliorate or reduce the ability of an individual's immune system to mount an immune response at the site of normal exposure to the mite allergen(s), resulting in a diminution in allergic symptoms.

For purposes of therapeutic effectiveness, therapeutic compositions preferably comprise at least two T cell epitopes of a mite allergen. Accordingly, isolated peptides preferably comprise at least two T cell epitopes and accordingly, the peptide comprises at least approximately eight amino acid residues, and preferably fifteen amino acid residues. Additionally, therapeutic compositions preferably comprise a sufficient percentage of the T cell epitopes of the entire protein allergen such that a therapeutic regimen of administration of the composition to an individual sensitive to house dust mite, results in T cells of the individual being tolerized to the protein allergen. Synthetically produced peptides of the invention are particularly desirable as increases in length may result in difficulty in peptide synthesis as well as retention of an undesirable property (e.g., immunoglobulin binding or enzymatic activity) due to maintenance of conformational similarity between the peptide and the protein allergen from which it is derived.

The individual peptide regions can be produced and tested to determine which regions bind immunoglobulin E specific for a mite allergen and which of such regions would cause the release of mediators (e.g., histamine) from mast cells or basophils. Those peptide regions found to bind immunoglobulin E and cause the release of mediators from mast cells or basophils in greater than approximately 10-15% of the allergic sera tested are preferably not included in the peptide regions arranged to form peptides of the invention.

Preferred peptides comprise various combinations of two or more regions, each region comprising at least one T-cell epitope of a protein allergen of the genus <u>Dermatophagoides</u> the regions may be derived from the same or different protein allergens of the genus <u>Dermatophagoides,</u> wherein at least one region comprises an amino acid sequence selected from the following group: DP I-21.1 (SEQ ID NO: 27); DP I-21.2 (SEQ ID NO: 28); DP I-22.1 (SEQ ID NO:29); DP I-23.1 (SEQ ID NO: 33); DP I-25.2 (SEQ ID NO: 36); DP I-26.1 (SEQ ID NO: 37); DP-I-28.1 (SEQ ID NO: 39); DP I-1 (SEQ ID NO: 9); DF I-1 (SEQ ID NO:72); DF I-21.1 (SEQ ID NO:90); DF I-22.1 (SEQ ID NO: 92); DF I-23.1 (SEQ ID NO: 95); DF I-25.1 (SEQ ID NO: 97): DP II-1 (SEQ ID NO: 41); DP II-1.2 (SEQ ID NO: 58): DP II-2.0 (SEQ ID NO: 56); DP II-20.3 (SEQ ID NO: 53); DP II-21 (SEQ ID NO: 62); DP II-22 (SEQ ID NO: 63); DP II-25 (SEQ ID NO: 69); DP II-25.2 (SEQ ID NO: 71); DF II-2 (SEQ ID NO: 104); DF II-4.5 (SEQ ID NO: 107); DF II-15 (SEQ ID NO: 109); DF II-17 (SEQ ID NO: 111); DF II-19.1 (SEQ ID NO: 114); DF I-22.2 (SEQ ID NO:93); and DP II-20.6 (SEQ ID NO:56), all as shown in Figs. 3-4 and wherein at least one region comprises an amino acid sequence selected from the group consisting of: DP I-21.7; DP I-23.10; DP I-23.11; DP I-23.12; DP I-23.5; DP I-23.6; DP I-23.7; DP I-23.8; DP I-23.9; DP I-26.2; DP II-20.7; DP II-22.6; DP II-22.3; DP II-22.4; DP II-22.5; DP II-25.3; DP II-25.4; DP I-23.13; DP I-23.14; DP I-23.15; DP I-23.16; DP I-23.17; DP I-26.3; DP I-26.4; DP I-26.5; DP II-22.7; DP II-22.8; DP II-22.9; DP II-22.10; and DP II-22.11 all as shown in Fig. 26, wherein at least one region comprises the amino acid sequence of DPI-21.7.

Preferred peptides comprise a combination of two or more regions (each region having an amino acid sequence as shown in Fig. 3, Fig. 4. and Fig. 25) including: DP I-21.7 and or DF I-22.2, DP I-23.13, DP I-26.1, DP II-20.6, DP II-22.3, and DP II-25.2; DP I-21.2, DF I-22.2, DP I-23.10, DP I-26.2, DP I-20.6, DP II-22.4, and DP II-25.3, DP I-21.7, DF I-22.2, DP I-23.11, DP I-26.3, DP II-20.7, DP . II-22.5, and DP II-25.4; DP I-21.7, DF I-22.2, DP I-23.12, DP I-26.4, DP II-20.6, DP II-22.6, and DP II-25.2; DP I-21.7, DF I-22.2, DP I-23.5, DP I-26.5, DP II-20.7, DP II-22.7, and DP II-25.3; DP I-21.7. DF I-22.2, DP I-23.6. DP I-26.2, DP II-20.6, DP II-22.8, and DP II-25.4; DP I-21.7, DF I-22.2, DP I-23.7, DP I-26.3. DP II-20.7, DP II-22.9, and DP II-25.3; DP I-21.7, DF I-22.2, DP I-23.8. DP I-26.4. DP II-20.6, DP II-22.3 and DP II-25.4; DP I-21.7, DF I-22.2, DP I-23.9, DP I-26.1, DP II-20.7, DP II-22.4, and DP II-25.3; DP I-21.7, DF I-22.2, DP I-23.13, DP I-26.2. DP II-20.6. DP II-22.5. and DP II-25.4; DP I-21.7 DFI-22.2, DP I 23.14, DP I-26.3, DP II-20.7. DP II-22.6. and DP II-25.3; DP I-21.7, DF I-22.2, DP I-23.15, DP I-26.4, DP II-20.6, DP II-22.6, and DP II-25.3; DP I-21.7, DF I-22.2, DP I-23.16, DP I-26.5, DP II-20.6, DP II-22.3, and DP II-25.4; DP I-21.7, DF I-22.2, DP I-23.17, DP I-26.2, DP II-20.6, DP II-22.4, and DP II-25.3; DP I-21.7, DF I-22.2, DP I-23.8, DP I-26.3, DP II-20.6, DP II-22.5, and DP II-25.4; DP I-21.7, DF I-22.2, DP I-23.9, DP I-26.4, DP II-20.6, DP II-22.6 and DP II-25.3; and DP I-21.7. DF I-22.2, DP I-23.5. DP I-26.1, DP II-20.6. DP II-22.7, and DP II-25.4.

Additional preferred peptides comprise at least two of the following combination of regions in any arrangement: X,Y,Z,A.B,C,D. wherein X is DP I-21.2 or DP <u>I</u>-21.7; Y is DF I-22.2; Z is DP I-23.1, DP I-23.10, DP I-23.11, DP I-23.12, DP I-23.13, DP I-23.14, DP I-23.15, DP I-23.16, DP I-23.17, DP I-23.5, DP I-23.6, DP I-23.7, DP I-23.8 or DP I-23.9.; A is DP I-26.1, DP I-26.2, DP I-26.3, DP I-26.4, or DP I-26.5; B is DP I-20.6 or DP II-20.7; C is DP II-22. DP II-22.6, DP II-22.7, DP II-22.8, DP II-22.9, DP II-22.10, DP II-22.11, DP II-22.3, DP II-22.4, or DP II-22.5; and D is DP II-25.2, DP II-25.3. or DP II-25.4, with the proviso that X, Y, Z, A, B, C, D is not the following combination of regions: DP I-21.2, DF I-22.2, DP I-23.1, DP I-26.1, DP II-20.6, DP II-22, and DP II-25.2, wherein at least one region comprises DPI-21.7.

Another preferred peptide comprises a specific sequential arrangement of amino acid sequences, said specific sequential arrangement having the formula:

ADYCZBX

wherein X is DP I-21.2 or DP I-21.7; Y is DF I-22.2; Z is DP I-23.1, DP I-23.10, DP I-23.11, DP I-23.12, DP I-23.13, DP I-23.14, DP I-23.15, DP I-23.16, DP I-23.17, DP I-23.5. DP I-23.6, DP I-23.7, DP I-23.8 or DP I-23.9.; A is DP I-26.1, DP I-26.2, DP I-26.3, DP I-26.4, or DP I-26.5; B is DP I-20.6 or DP II-20.7; C is DP II-22, DP II-22.6, DP II-22.7, DP II-22.8, DP II-22.9, DP II-22.10, DP II-22.11, DP II-22.3, DP II-22.4, or DP II-22.5; and D is DP II-25.2, DP II-25.3, or DP II-25.4, with the proviso that ADYCZBX is not DP I-26.1, DP II-25.2, DF I-22.2, DP II-22, DP I-23.1, DP II-20.6 and DP I-21.2 respectively, wherein at least one sequence is DPI -21.7.

Another preferred peptide comprises a specific sequencial arrangement of amino acid sequences, said specific sequential arrangement having the formula:

DYZCAXB

wherein X is DP I-21.2 or DP I-21.7: Y is DF I-22.2: Z is DP I-23.1. DP I-23.10, DP I-23.11, DP I-23.12, DP I-23.13. DP I-23.14. DP I-23.15. DP I-23.16, DP I-23.17, DP I-23.5, DP I-23.6. DP I-23.7, DP I-23.8 or DP I-23.9.; A is DP I-26.1, DP I-26.2, DP I-26.3, DP I-26.4, or DP I-26.5: B is DP I-20.6 or DP II-20.7; C is DP II-22, DP II-22.6, DP II-22.7, DP II-22.8. DP II-22.9, DP II-22.10. DP II-22.11, DP II-22.3, DP II-22.4, or DP II-22.5: and D is DP II-25.2, DP II-25.3, or DP II-25.4, with the proviso that DYZCAXB is not DP II-25.2, DF I-22.2, DP I-23.1, DP II-22, DP I-26.1, DP I-21.2 and DP II-20.6 respectively, wherein at least one sequence is DPI-21.7.

Yet another preferred peptide has a specific arrangement of amino acid sequences, said specific sequential arrangement having the formula:

DXZACBY

wherein X is DP I-21.2 or DP I-21.7; Y is DF I-22.2; Z is DP I-23.1, DP I-23.10, DP I-23.11, DP I-23.12, DP I-23.13, DP I-23.14, DP I-23.15, DP I-23.16, DP I-23.17, DP I-23.5, DP I-23.6, DP I-23.7, DP I-23.8 or DP I-23.9.; A is DP I-26.1, DP I-26.2, DP I-26.3, DP I-26.4, or DP I-26.5; B is DP I-20.6 or DP II-20.7; C is DP II-22, DP II-22.6, DP II-22.7, DP II-22.8, DP II-22.9, DP II-22.10. DP II-22.11, DP II-22.3, DP II-22.4, or DP II-22.5; and D is DP II-25.2, DP II-25.3, or DP II-25.4, with the proviso that DXZACBY is not DP II-25.2, DP I-21.2, DP I-23.1, DP I-26.1, DP II-22, DP II-20.6, and DF I-22.2 respectively, wherein at least one sequence is DPI-21.7.

Peptides of protein allergens of the genus Dermatophagoides within the scope of the invention can be used in methods of treating and preventing allergic reactions to mite allergens. Thus, one aspect of the present invention provides therapeutic compositions comprising a peptide or a modified peptide of the invention and a pharmaceutically acceptable carrier or diluent. In another aspect, the therapeutic composition comprises a pharmaceutically acceptable carrier or diluent and a peptide comprising at least two regions, each region comprising at least one T cell epitope of a mite allergen and derived from the same or from different mite protein allergens.

The composition comprises a sufficient percentage of the T cell epitopes of at least one protein allergen such that a therapeutic regimen of administration of the composition to an individual sensitive to a house dust mite allergen resulting in T cells of the individual being tolerized to the protein allergen. Preferably, the composition comprises a sufficient percentage of the T cell epitopes of one or more protein allergens such that at least about 40%, and more preferably at least about 60% of the T cell reactivity of each protein is included in the composition. Such compositions can be administered to an individual to treat or prevent sensitivity to house dust mite or to an allergen which is immunologically cross-reactive with house dust mite.

Administration of the therapeutic compositions of the present invention to desensitive an individual can be carried out using known techniques. For example, a peptide derived from a mite allergen comprising at least one T cell epitope can be administered in combination with an appropriate diluent, a carrier, and/or an adjuvant. To induce T cell anergy in an individual, the terapeutic composition is preferably administered in non-immunogenic form, e.g. it does not contain adjuvant. Pharmaceutically acceptable diluents include saline and aqueous buffer solutions. Pharmaceutically acceptable carriers include polyethylene glycol (Wie et al. International Archives of Allergy and Applied Immunology 64: 84-99 (1981)) and liposomes (Strejan et al., Journal of Neuroimmunology 7: 27 (1984)). Such compositions will generally be administered by injection, (subcutaneous, intravenous, etc.) oral administration, (e.g. as in the form of a capsule), inhalation, transdermal application or rectal administration. The therapeutic compositions of the invention are administered to individuals sensitive to house dust mite at dosages and for lengths of time effective to reduce sensitivity (i.e.. reduce the allergic response) of the individual to a house dust mite allergen. A therapeutically effective amount of one or more of the same or of different therapeutic compositions can be administered simultaneously or sequentially to an individual sensitive to house dust mite. Effective amounts of the therapeutic compositions will vary according to factors such as the degree of sensitivity of the individual to house dust mites, the age, sex, and weight of the individual, and the ability of the peptide to stimulate a T cell response in the individual.

A preferred therapeutic composition of the inventino comprises DPI-21.7 and at least one of the following modified peptides of the invention comprising at least one T cell epitope and a pharmaceutically acceptable carrier or diluent: DP I-23.10; DP I-23.11; DP I-23.12; DP I-23.5; DP I-23.6; DP I-23.7; DP I-23.8; DP I-23.9; DP I-26.2; DP II-20.7; DP II-22.6; DP II-22.3; DP II-22.4; DP II-22.5; DP II-25.3; DP II-25.4; DP I-23.13; DP I-23.14; DP I-23.15; DP I-23.16; DP I-23.17; DP I-26.3; DP I-26.4; DP I-26.5; DP II-22.7; DP II-22.8; DP II-22.9; DP II-22.10; and DP II-22.11 all as shown in Fig. 26.

In yet another aspect of the present invention, a composition is provided comprising at least two peptides (e.g., a physical mixture of at least two peptides), each comprising at least one T cell epitope of a protein allergen of the genus Dermatophagoides. The peptides or modified peptides are derived from the same or from different mite allergens. Such compositions can be administered in the form of a therapeutic composition with a pharmaceutically acceptable carrier of diluent. A therapeutically effective amount of one or more of such compositions can be administered simultaneously or sequentially to an individual sensitive to house dust mite.

Preferred therapeutic compositions comprising a pharmaceutically acceptable carrier or diluent and at least two peptides each comprising at least one T cell epitope, comprise at least one peptide selected from the group consisting of: DP I-1 (SEQ ID NO: 9); DP I-2 (SEQ ID NO: 10); DP I-3 (SEQ ID NO: 11); DP I-4 (SEQ ID NO: 12); DP I-11.1 (SEQ ID NO: 13); DP I-12.1 (SEQ ID NO: 14); DP I-5 (SEQ ID NO: 15); DP I-13 (SEQ ID NO: 17); DP I-14 (SEQ ID NO: 18); DP I-15 (SEQ ID NO: 19); DP I-6.1 (SEQ ID NO: 20); DP I-7.1 (SEQ ID NO: 21); DP I-8 (SEQ ID NO: 22); DP I-9 (SEQ ID NO: 23); DP I-16 (SEQ ID NO: 24); DP I-10 (SEQ ID NO: 25); DP I-17 (SEQ ID NO: 26); DP I-21.1 (SEQ ID NO: 27); DP I-21.2 (SEQ ID NO: 28); DP I-22.1 (SEQ ID NO: 29); DP I-22.2 (SEQ ID NO: 30); DP I-22.3 (SEQ ID NO: 31); DP I-22.4 (SEQ ID NO: 32); DP I-23.1 (SEQ ID NO: 33); DP I-23.2 (SEQ ID NO: 34); DP I-25.1 (SEQ ID NO: 35); DP I-25.2 (SEQ ID NO: 36); DP I-26.1 (SEQ ID NO: 37); DP I-27.1 (SEQ ID NO: 38); DP I-28.1 (SEQ ID NO: 39); DP I-28.2 (SEQ ID NO: 40), DF I-1 (SEQ ID NO: 72); DF I-2.1 (SEQ ID NO: 73); DF I-3 (SEQ ID NO: 74); DF I-4 (SEQ ID NO: 75); DF I-11 (SEQ ID NO: 76); DF I-12 (SEQ ID NO: 77); DF I-5 (SEQ ID NO: 78); DF I-13 (SEQ ID NO: 79); DF I-14 (SEQ ID NO: 80); DF I-15 (SEQ ID NO: 81); DF I-6 (SEQ ID NO: 82); DF I-7 (SEQ ID NO: 83); DF I-8.1 (SEQ ID NO: 84); DF I-8 (SEQ ID NO: 85); DF I-9 (SEQ ID NO: 86); DF I-16 (SEQ ID NO: 87): DF I-10 (SEQ ID NO: 88); DF I-17 (SEQ ID NO: 89): DF I-21.1 (SEQ ID NO: 90): DF I-21.2 (SEQ ID NO: 91): DF I-22.1 (SEQ ID NO: 92): DF I-22.2 (SEQ ID NO: 93), DF I-22.4 (SEQ ID NO: 94); DF I-23.1 (SEQ ID NO: 95): DF I-23.2 (SEQ ID NO: 96): DF I-25.1 (SEQ ID NO: 97): DF I-25.2 (SEQ ID NO: 98); DF I-26.1 (SEQ ID NO: 99): DF I-27.1 (SEQ ID NO: 100); DF I-28.1 (SEQ ID NO: 101); DF I-28.2 (SEQ ID NO: 102): DP II-20 (SEQ ID NO: 50): DP II-20.1 (SEQ ID NO: 51); DP II-20.2 (SEQ ID NO: 52); DP II-20.3 (SEQ ID NO: 53); DP II-20.4 (SEQ ID NO: 54); DP II-20.5 (SEQ ID NO: 55); DP II 20.6 (SEQ ID NO: 56): DP II-1 (SEQ ID NO: 41): DP II-2 (SEQ ID NO: 42); DP II-3.1 (SEQ ID NO: 43); DP II-4 (SEQ ID NO: 44); DP II-5 (SEQ ID NO: 45); DP II-6 (SEQ ID NO: 46); DP II-7 (SEQ ID NO: 47); DP II-8 (SEQ ID NO: 48); DP II-9 (SEQ ID NO: 49); DP II-1.1 (SEQ ID NO: 57); DP II-1.2 (SEQ ID NO: 58); DP II-2.1 (SEQ ID NO: 59): DP II-2.2 (SEQ ID NO: 60); DP II-2.3 (SEQ ID NO: 61): DP II-21 (SEQ ID NO: 62); DP II-22 (SEQ ID NO: 63); DP II-26 (SEQ ID NO: 64); DP II-26.1 (SEQ ID NO: 65); DP II-23 (SEQ ID NO: 66): DP II-23.1 (SEQ ID NO: 67); DP II-24 (SEQ ID NO: 68); DP II-25 (SEQ ID NO: 69); DP II-25.1 (SEQ ID NO: 70); DP II-25.2 (SEQ ID NO: 71); DF II-1 (SEQ ID NO: 103); DF II-2 (SEQ ID NO: 104); DF II-13.1 (SEQ ID NO: 105); DF II-3.1 (SEQ ID NO: 106); DF II-4.5 (SEQ ID NO: 107); DF II-4.3 (SEQ ID NO: 108); DF II-15 (SEQ ID NO: 109); DF II-16 (SEQ ID NO: 110); DF II-17 (SEQ ID NO: 111); DF II-18 (SEQ ID NO: 112); DF II-19 (SEQ ID NO: 113); DF II-19.1 (SEQ ID NO: 114); DF II-21 (SEQ ID NO: 115); and DF II-22 (SEQ ID NO: 116), DP I-23.1.1, DP I-23.1.2, DP I-23.1.3, DP I-23.1.4, DP II-22.1. DP II-22.2 (all as shown in Figs. 3; 4, and 25), and at least one region comprises a DPI-21.7 and a modified amino acid sequence selected from the following group: DP I-23.10; DP I-23.11; DP I-23.12; DP I-23.5; DP I-23.6; DP I-23.7: DP I-23.8; DP I-23.9; DP I-26.2; DP II-20.7; DP II-22.6; DP II-22.3; DP II-22.4; DP II-22.5; DP II-25.3; DP II-25.4; DP I-23.13; DP I-23.14; DP I-23.15; DP I-23.16; DP I-23.17; DPI-26.3; DP I-26.4; DP I-26.5; DP II-22.7; DP II-22.8; DP II-22.9; DP II-22.10; and DP II-22.11 all as shown in Fig. 26.

Even more preferred therapeutic compositions comprising a pharmaceutically acceptable carrier or diluent and at least two peptides each comprising at least one T cell epitope, comprise at least one peptide selected from the group consisting of: DP I-21.1 (SEQ ID NO: 27): DP I-21.2 (SEQ ID NO: 28): DP I-22.1 (SEQ ID NO: 29): DP:I-22.2 (SEQ ID NO: 30): DP I-22.3 (SEQ ID NO: 31); DP I-22..I (SEQ ID NO: 32): DP I-23.1 (SEQ ID NO: 33): DP I-23.2 (SEQ ID NO: 34); DP I-25.1 (SEQ ID NO: 35): DP I-25.2 (SEQ ID NO: 36); DP I-26.1 (SEQ ID NO: 37): DP I-27.1 (SEQ ID NO: 38): DP I-28.1 (SEQ ID NO: 39): DP I-28.2 (SEQ ID NO: 40); DP I-1 (SEQ ID NO: 9); DF I-1 (SEQ ID NO: 72); DF I-21.1 (SEQ ID NO: 90); DF I-21.2 (SEQ ID NO: 91); DF I-22.1 (SEQ ID NO: 92): DF I-22.2 (SEQ ID NO: 93); DF I-22.4 (SEQ ID NO: 94); DF I-23.1 (SEQ ID NO: 95); DF I-23.2 (SEQ ID NO: 96); DF I-25.1 (SEQ ID NO: 97): DF I-25.2 (SEQ ID NO: 98); DF I-26.1 (SEQ ID NO: 99); DF I-27.1 (SEQ ID NO: 100); DF I-28.1 (SEQ ID NO: 101); DF I-28.2 (SEQ ID NO: 102); DP II-20 (SEQ ID NO: 50); DP II-20.1 (SEQ ID NO: 51); DP II-20.2 (SEQ ID NO: 52); DP II-20.3 (SEQ ID NO: 53); DP II-20.4 (SEQ ID NO: 54); DP II-20.5 (SEQ ID NO: 55): DP II 20.6 (SEQ ID NO: 56): DP II-1 (SEQ ID NO: 41); DP II-1.1 (SEQ ID NO: 57); DP II-1.2 (SEQ ID NO: 58); DP II-2.1 (SEQ ID NO: 59); DP II-2.2 (SEQ ID NO: 60); DP II-2.3 (SEQ ID NO: 61): DP II-21 (SEQ ID NO: 62); DP II-22 (SEQ ID NO: 63); DP II-26 (SEQ ID NO: 64); DP II-26.1 (SEQ ID NO: 65); DP II-23 (SEQ ID NO: 66); DP II-23.1 (SEQ ID NO: 67); DP II-24 (SEQ ID NO: 68); DP II-25 (SEQ ID NO: 69); DP II-25.1 (SEQ ID NO: 70); DP II-25.2 (SEQ ID NO: 71); DF II-1 (SEQ ID NO: 103); DF II-2 (SEQ ID NO: 104); DF II-13.1 (SEQ ID NO: 105); DF II-3.1 (SEQ ID NO: 106): DF II-4.5 (SEQ ID NO: 107); DF II-4.3 (SEQ ID NO: 108); DF II-15 (SEQ ID NO: 109); DF II-16 (SEQ ID NO: 110); DF II-17 (SEQ ID NO: 111): DF II-18 (SEQ ID NO: 112); DF II-19 (SEQ ID NO: 113); DF II-19.1 (SEQ ID NO: 114); DF II-21 (SEQ ID NO: 115); and DF II-22 (SEQ ID NO: 116), and at least one modified peptide is selected from the group consisting of DP I-21.7; DP I-23.10; DP I-23.11; DP I-23.12; DP I-23.5; DP I-23.6: DP I-23.7; DP I-23.8; DP I-23.9; DP I-26.2; DP II-20.7; DP II-22.6; DP II-22.3; DP II-- 22.4; DP II-22.5: DP II-25.3; DP II-25.4 DP-I-23.13; DP I-23.14; DP I-23.15; DP I-23.16; DP I-23.17; DP I-26.3; DP I-26.4; DP I-26.5; DP II-22.7; DP II-22.8; DP II-22.9; DP II-22.10; and DP II-22.11 all as shown in Fig. 26. wherein said composition comprises a sufficient percentage of the T cell epitopes of at least one protein allergen such that upon administration of the composition to an individual sensitive to a house dust mite allergen, T cells of the individual are tolerized to said at least one protein allergen and wherein at least one modified peptide is DP I-21.7.

Other preferred compositions of the invention comprise the following combination of peptides and/or modified peptides and a pharmaceutically acceptable carrier or diluent: DP I-21.7. DF I-22.2, DP I-23.13. DP I-26.1. DP II-20.6. DP II-22.3, and DP II-25.2: DP I-21.2. DF I-22.2, DP I-23.10. DP I-26.2. DP I-20.6, DP II-22.4, and DP II-25.3, DP I-21.7, DF I-22.2, DP I-23.11, DP I-26.3, DP II-20.7, DP II-22.5, and DP II-25.4; DP I-21.7, DF I-22.2. DP I-23.12, DP I-26.4, DP II-20.6, DP II-22.6, and DP II-25.2; DP I-21.7, DF I-22.2. DP I-23.5. DP I-26.5, DP II-20.7, DP II-22.7, and DP II-25.3; DP I-21.7, DF I-22.2, DP I-23.6, DP I-26.2, DP II-20.6. DP II-22.8, and DP II-25.4; DP I-21.7, DF I-22.2. DP I-23.7, DP I-26.3, DP II-20.7, DP II-22.9, and DP II-25.3; DP I-21.7. DF I-22.2. DP I-23.8. DP I-26.4, DP II-20.6. DP II-22.3 and DP II-25.4: DP I-21.7, DF I-22.2, DP I-23.9, DP I-26.1, DP II-20.7, DP II-22.4, and DP II-25.3; DP I-21.7. DF I-22.2. DP I-23.13, DP I-26.2, DP II-20.6, DP II-22.5, and DP II-25.4; DP I-21.7, DF I-22.2, DP I-23.14, DP I-26.3, DP II-20.7, DP II-22.6, and DP II-25.3; DP I-21.7, DF I-22.2, DP I-23.15, DP I-26.4, DP II-20.6, DP II-22.6, and DP II-25.3; DP I-21.7, DF I-22.2, DP I-23.16, DP I-26.5, DP II-20.6, DP IL-22.3, and DP II-25.4; DP I-21.7, DF I-22.2, DP I-23.17, DP I-26.2, DP II-20.6, DP II-22.4, and DP II-25.3; DP I-21.7, DF I-22.2, DP I-23.8, DP I-26.3, DP II-20.6, DP II-22.5, and DP II-25.4; DP I-21.7, DF I-22.2, DP I-23.9, DP I-26.4, DP II-20.6, DP II-22.6 and DP II-25.3; and DP I-21.7, DF I-22.2, DP I-23.5, DP I-26.1, DP II-20.6, DP II-22.7, and DP II-25.4.

In yet another aspect of the invention a composition is provided comprising a pharmaceutically acceptable carrier or diluent and at least two, preferably at least four, more preferably at least six, even more preferably at least seven of the following combination of peptides: X, Y, Z, A, B, C, D, wherein X is DP I-21.2 or DP I-21.7; Y is DF I-22.2; Z is DP I-23.1, DP I-23.10, DP I-23.11, DP I-23.12, DP I-23.13, DP I-23.14, DP I-23.15, DP I-23.16, DP I-23.17, DP I-23.5, DP I-23.6, DP I-23.7, DP I-23.8 or DP I-23.9.; A is DP I-26.1, DP I-26.2, DP I-26.3, DP I-26.4, or DP I-26.5; B is DP I-20.6 or DP I-20.7; C is DP II-22, DP II-22.6, DP II-22.7, DP II-22.8, DP II-22.9, DP II-22.10, DP II-22.11, DP II-22.3, DP II-22.4, or DP II-22.5: and D is DP II-25.2, DP II-25.3, or DP II-25.4, with the provisos that: (a) X, Y, Z, A, B, C. D is not the following combination of peptides: DP I-21,2, DF I-22.2, DP I-23.1. DP I-26.1, DP II-20.6, DP II-22, and DP II-25.2; and (b) the composition comprises DP I-21.7.

The present invention also provides methods of detecting sensitivity in individuals to house dust mite allergens comprising combining a blood sample obtained from the individual with a peptide of the present invention, under conditions appropriate for binding of blood components with the peptide and determining the extent to which such binding occurs. The extent to which binding occurs is determined by assessing T cell function, T cell proliferation or a combination thereof.

It is also possible to modify or further modify the structure of a peptide of the invention for such purposes as increasing solubility, enhancing therapeutic or preventive efficacy, or stability (e.g., shelf life ex vivo, and resistance to proteolytic degradation in vivo). A modified peptide can be produced in which the amino acid sequence has been altered, such as by amino acid substitution, deletion, or addition, to modify immunogenicity and/or reduce allergenicity, or to which a component has been added for the same purpose.

For example, a peptide can be modified so that it maintains the ability to induce T cell anergy and bind MHC proteins without the ability to induce a strong proliferative response or possibly, any proliferative response when administered in immunogenic form. In this instance, critical binding residues for the T cell receptor can be determined using known techniques (e.g.. substitution of each residue and determination of the presence or absence of T cell reactivity). Those residues shown to be essential to interact with the T cell receptor can be modified by replacing the essential amino acid with another, preferably similar amino acid residue (a conservative substitution) whose presence is shown to enhance, diminish but not eliminate, or not effect T cell reactivity. In addition, those amino acid residues which are not essential for T cell receptor interaction can be modified by being replaced by another amino acid whose incorporation may enhance, diminish or not effect T cell reactivity but does not eliminate binding to relevant MHC.

Additionally, peptides of the invention can be modified by replacing an amino acid shown to be essential to interact with the MHC protein complex with another, preferably similar amino acid residue (conservative substitution) whose presence is shown to enhance, diminish but not eliminate or not effect T cell activity. In addition, amino acid residues which are not essential for interaction with the MHC protein complex but which still bind the MHC protein complex can be modified by being replaced by another amino acid whose incorporation may enhance, not effect, or diminish but not eliminate T cell reactivity. Preferred amino acid substitutions for non-essential amino acids include, but are not limited to substitutions with alanine, glutamic acid, or a methyl amino acid.

Another example of a modification of peptides is substitution of cysteine residues preferably with serine, threonine, leucine or glutamic acid to minimize dimerization via disulfide linkages. For example, the stability of a peptide of Der p II or Der f II which includes the first ten amino acid residues of either allergen can be enhanced by replacing the cysteine located at the 8th amino acid residue, preferably with alanine, or glutamic acid, or alternatively with serine or threonine.

In order to enhance stability and/or reactivity, peptides can also be modified to incorporate one or more polymorphisms in the amino acid sequence of a protein allergen resulting from natural allelic variation.. Additionally. D-amino acids, non-natural amino acids or non-amino acid analogues can be substituted or added to produce a modified peptide within the scope of this invention. Furthermore, peptides of the present invention can be modified using the polyethylene glycol (PEG) method of A. Sehon and co-workers (Wie et al. supra) to produce a peptide conjugated with PEG. In addition, PEG can be added during chemical synthesis of a peptide of the invention. Modifications of peptides or portions thereof can also include reduction/alkylation (Tarr in: Methods of Protein Microcharacterization, J.E. Silver ed. Humana Press, Clifton, NJ, pp 155-194 (1986)): acylation (Tarr, supra); esterification (Tarr, supra); chemical coupling to an appropriate carrier (Mishell and Shiigi, eds, Selected Methods in Cellular Immunology, WH Freeman, San Francisco, CA (1980); U.S. Patent 4.939,239); or mild formalin treatment (Marsh International Archives of Allergy and Applied Immunology 41: 199-215 (1971)).

Peptides within an allergen group (e.g., Der p I or Der p II) can be modified to enhance T cell reactivity. Given the cross-reactivity within the Group I and Group II allergens, a peptide of one group allergen which may be less reactive than a peptide of another group allergen corresponding in amino acid position can have one or more amino acids substituted with one or more amino acids from the corresponding peptide.. Additionally, peptides can be modified to incorporate a polymorphism in the amino acid sequence of a protein allergen resulting from natural allelic variation. Modification of peptides to include one or more of these polymorphisms may result in enhanced stability and/or reactivity.

To facilitate purification and potentially increase solubility of peptides of the invention, it is possible to add reporter group(s) to the peptide backbone. For example, poly-histidine can be added to a peptide to purify the peptide on immobilized metal ion affinity chromatography (Hochuli, E. et al., Bio/Technology, 6:1321-1325 (1988)). In addition, specific endoprotease cleavage sites can be introduced, if desired, between a reporter group and amino acid sequences of a peptide to facilitate isolation of peptides free of irrelevant sequences. In order to successfully desensitize an individual to a protein antigen, it may be necessary to increase the solubility of a peptide by adding functional groups to the peptide or by not including hydrophobic T cell epitopes or regions containing hydrophobic epitopes in the peptides.

To potentially aid proper antigen processing of T cell epitopes within a peptide, canonical protease sensitive sites can be recombinantly or synthetically engineered between regions, each comprising at least one T cell epitope. For example, charged amino acid pairs, such as KK or RR, can be introduced between regions within a peptide during recombinant construction of the peptide. The resulting peptide can be rendered sensitive to cathepsin and/or other trypsin-like enzymes cleavage to generate portions of the peptide containing one or more T cell epitopes. In addition, such charged amino acid residues can result in an increase in solubility of a peptide.

Site-directed mutagenesis of DNA encoding a peptide of the invention can be used to modify the structure of the peptide. Such methods may involve PCR (Ho et al., Gene, 77:51-59 (1989)) or total synthesis of mutated genes (Hostomsky, Z., et al., Biochem. Biophys. Res. Comm., 161: 1056-1063 (1989)). To enhance bacterial expression, the aforementioned methods can be used in conjunction with other procedures to change the eucaryotic codons in DNA constructs encoding peptides of the invention to ones preferentially used in E. coli.

Specific examples of peptides modified in accordance with one or more of the modification procedures discussed above include but are not limited to modifications to peptide DPI-23.1 as shown in Fig. 25 and to peptide DPII-22 as shown in Fig. 25. More specifically, modifications to peptides 23.1 include the addition of charged amino acids (DP I-23.7) charged amino acid pairs (DP I-23.5) to increase solubility and replacement of a cysteine residue with serine (DP I-23.6) or glutamic acid to increase solubility. Changes to peptide DP II-22 include the addition of charged amino acid pairs (22.6 and 22.3) to increase solubility as shown in Fig. 25.

The present invention also provides nucleic acids having sequences encoding peptides of the invention. Nucleic acid sequences used in any embodiment of this invention can be cDNA as described herein, or alternatively, can be any oligodeoxynucleotide sequence having all or a portion of a sequence represented herein, or their functional equivalents. Such oligodeoxynucleotide sequences can be produced chemically or mechanically, using known techniques.

This invention is further illustrated by the following non-limiting examples.

### Example I Native Mite Allergen Purification

What follows is a description of the work done to purify the group I and group II allergens of the house dust mites Dermatophagoides pteronyssinus and Dermatophagoides farinae in their native form as primary antigens for human T-cell epitope mapping.

All four protein allergens, Der f I, Der p I, Der f II, and Der p II, were immunoaffinity purified from spent mite culture media obtained from either Commonwealth Sera Laboratories Melbourne, Australia or Dr. Larry G. Arlian at Wright State University Dayton, Ohio. A 10% (wt./vol.) aqueous extract of dessicated spent mite culture media was prepared in PBS (Phosphate Buffered Saline) with stirring overnight at 4°C. Insoluble material was removed by centrifugation at 10,000 X g for 1 hour at 4°C. The supernatant was then filtered on a vacuum manifold through Whatman #1 paper, and re-centrifuged at 15,000 X g for 1 hour at 4°C. A final filtration was carried out through a 0.45 m cellulose acetate filter.

Monoclonal antibodies 4C1 and 6D6 (University of Virginia, NC) were used for immunoaffinity purification of group I or group II mite allergens respectively (Chapman et al., J Allergy Clin. Immunol. 80: 1479-1484 (1987); Heymann et al., J. Allergy Clin. Immunol. 83: 1055-1067 (1989)). The 4C1 monoclonal antibody reacts with an epitope shared by both the Der f I and Der p I proteins; similarly, the 6D6 monoclonal antibody reacts with both the Der f II and Der p II proteins.

For each monoclonal antibody, ascites fluid was cut in 50% ammonium sulfate and the antibodies coupled to CNBr-activated Sepharose 4B (Pharmacia) in 100 mM NaHCO$_3$. 500 mM NaCl, pH 8.3, overnight at 4°C.

Monoclonal antibody columns were equilibrated in PBS and the filtered extracts loaded at 15 ml per hour. The column was then washed in 20 volumes of PBS, after which a more stringent wash of 20 column volumes was carried out with PBS supplemented with 500 mM NaCl. Proteins were eluted in 500 mM NaCl, 100 mM glycine pH 11.0, and fractions evaluated for protein content by spectrophotometric absorbance at 280 nm. Peak fractions were pooled and dialyzed extensively against PBS, concentrated with a negative pressure dialysis device (obtained from Amicon, Beverly MA) and were used in T-cell epitope mapping studies of the mite group I and group II allergens. Recovered proteins were obtained at purities ranging from 80% (group II) to 90% (group I).

Reverse Phase HPLC chromatography was applied to further purify the immunoaffinity purified group II mite protein allergen according to the conditions in Heymann et al., J. Allergy Clin. Immunol. 83: 1055-1067 (1989). Briefly, immunoaffinity purified protein was applied to a 5 μm 300 Å C-8 column (Applied Biosystems Inc.) in 0.1% vol./vol. TFA/H$_2$O. The flow rate for the column was 1 ml/min. with a gradient of 0-60% acetonitrile/0.1% TFA over 60 minutes. Group II proteins eluted around 45% acetonitrile/0.1% TFA. Fractions were analyzed for purity by SDS-polyacrylamide gel electrophoresis followed by densitometric scanning. Those fractions with group II protein of purity greater than 90% were subsequently utilized for human T-cell mapping of the allergen.

### Example II Recombinant Mite Allergen Expression

What follows is a description of the work done to produce the group I and group II allergens of the house dust mites Dermatophagoides pteronyssinus and Dermatophagoides farinae as recombinant proteins in E. coli.

All four protein allergens, Der f I, Der p I, Der f II, and Der p II, were fused at their mature amino termini with the leader sequence MGHHHHHHEF (where amino acids EF are encoded by the EcoR I restriction site GAATTC). Since the H$_6$ stretch of amino acids coordinates Ni$^{++}$ ions on NTA agarose columns (Diagen GmbH), this activity was exploited in the purification of the recombinant proteins (Hochuli et al., Biotechnology 86: 1321-1325 (1988)). Ultimately, all four allergens were subcloned into the expression vector pET 11d (Studier et al., Methods In Enzymology 185: 60-88 (1990)) under the transcriptional control of the phage T7 gn 10 promoter.

The cDNAs encoding the group I allergens from D. pteronyssinus and D. farinae were obtained from Dr. Wayne Thomas in plasmid form as subclones from λgt11 (Chua et al., J. Exp. Med. 167: 175-182 (1988); Dilworth et al., Clin. Exp. Allergy 21: 25-32 (1991)). The Der p I cDNA had been subcloned from an M 13 RF plasmid as an EcoRI fragment into pUC 18 by Dr. Roland Buelow at ImmuLogic (Palo Alto), while the Der f I cDNA was manipulated directly from the M13mp19 RF plasmid Df I(1).

Initially the cDNAs were subcloned into the expression vector pTrc99A His$_6$ Insert RRRS which is a modified version of pTrc99A (Amann et al., Gene 69: 301-315 (1988)). The original vector was modified by the addition of a synthetic adaptor (consisting of two complimentary oligonucleotides) encoding the leader sequence MGHHHHHHEF between its NcoI (MG) and EcoRI (EF) sites at the 5' end of the polylinker, and an RRS (Retro Regulatory Sequence) into its Hind III site at the polylinker's 3' end. The leader sequence was used as a purification aid as described above, while the RRS was added to enhance recombinant message stability and thereby increase recombinant protein yield (Skoglund et al., Gene 88: 1-5 (1990)). Lastly, an insert, in this case an Amb a I. 1 cDNA encoding the major allergen of the short ragweed (Rafnar et al., J. Biol. Chem. 226: 1229-1236 (1991)) was subcloned in frame with the H$_6$ leader as an EcoRI to PstI fragment.

To express the mite group I allergens, the Amb a I.1 cDNA was excised by EcoRI/Hind III digestion and replaced by adaptors with φEcoRI/Hind III overhangs (composed of a pair of complimentary oligonucleotides). These adaptors (Fig. 2a) encoded the first 5 amino acids, up to the Pst I site, of the mature Der p I NH$_2$ terminus, and the first 10 amino acids, up to the HpaI site, of the mature Der f I NH$_2$ terminus. Upon ligation of the EcoRI site of the vector and the φ EcoRI site in the adaptors, the EcoRI site in the vector was destroyed, leaving the EcoRI-site encoded internal to the adaptor as the sole EcoRI site in the intermediate constructs pTrc99A His$_6$ 5' pI RRS and pTrc99A His$_6$ 5' fI RRS. The Der p I cDNA was inserted as a PstI/EcoRI fragment into PstI/EcoRI digested pTrc99A His$_6$ 5' pI RRS, while the Der fI cDNA was inserted as a HpaI/EcoRI fragment into HpaI/EcoRI digested pTrc99A His$_6$ 5' fI RRS. The sequence of the 5' end of each construct, pTrc99A His$_6$ 5' pI RRS and pTrc99A His$_6$ fI RRS, was verified by dideoxy chain termination DNA sequencing using a Sequenase™ II kit (U.S. Biochemicals). Both the H$_6$ pI and H$_6$ fI coding cassettes were excised by NcoI/NheI digestion (an NheI site existed at the 3' end of the RRS adaptor) and inserted into NcoI/NheI digested pET11d. These two constructs, pET11d His$_6$ pI RRS and pET11d His$_6$ fI RRS, were transformed into competent BL21 [DE3] bacteria for expression of the recombinant proteins. BL21 [DE3] contains a recombinant phage λ lysogen, DE 3, with a phage T7 RNA polymerase gene under the transcriptional control of the lac UV5 promoter. T7 RNA polymerase gene expression is induced by the addition of IPTG (Isopropyl-B-D-thiogalactopyrano- side), which in turn leads to high level expression of the recombinant gene subcloned 3' of the pET vector's T7 gn 10 promoter.

The cDNAs encoding the group II allergens from D. pteronyssinus and D. farinae were also obtained from Dr.

Wayne Thomas in plasmid form as subclones from λgt11 (Chua et al., Int Arch. Appl. Immun. 91: 118-123 (1990); Trudinger et al., Clin. Exp. Allergy 21: 33-37 (1991)). The original Der p II cDNA was subcloned from an M13RF plasmid into the pCA and pGEX vectors by Dr. Roland Buelow at ImmuLogic (Palo Alto). Dr. Thomas' group had supplied the Der f II cDNA as a subclone in pGEX. Both pGEX plasmids harboring group II cDNAs were used as templates for PCR amplification with the same 5' sense/3' antisense primer pair (Fig. 2b). The primers were designed to fuse an EcoRI site (GAATTC encoding the amino acids EF) in frame with the $NH_2$ terminus of the mature group II proteins and place a PstI site 3' of the group II coding region. An MJ Research Thermal Controller with a program of 30 X (94°C 1 min./55°1 min. 30 sec./72°C 2 min.) was used in conjunction with reagents from a Perkin Elmer - Cetus Gene Amp kit for PCR amplification. The PCR products were EcoRI/PstI digested and subcloned into EcoRI/PstI digested M13mp19RF. DNA sequence analysis was performed to verify the sequence of the PCR products. Correct M13RF were EcoRI/PstI digested, their group II cDNA inserts isolated, and subcloned into EcoRI/PstI digested pTrc99A $His_6$ Amb a I.1 RRS (which served to exchange the group II cDNAs with the ragweed cDNA (Fig. 1)).

The Der f II cDNA possessed a sequence polymorphism at position 54 (i.e., threonine residue in place of isoleucine). To alter this polymorphism, site directed mutagenesis of the $T_{54}$ residue in the Der f II cDNA was performed using a Muta-Gene kit from Bio-Rad Laboratories, based on the method of Kunkel, Proc. Natl. Acad. Sci. USA 82: 488-492 (1985). The original M13mp19RF with the $T_{54}$ Der f II cDNA was transformed into CJ236 bacteria, which tolerate the incorporation of uracil in place of thymidine during DNA replication, and single stranded phage DNA prepared as template. A mutagenic 17 base pair primer (Fig. 2b) was annealed to the phage template DNA. T4 DNA polymerase was used to copy the template DNA primed by the mutagenic oligonucleotide, and T4 DNA ligase served to seal gaps in the DNA strand. The reaction was transformed into MV1190 bacteria, which are wild type for the editing of uracil residues from DNA and therefore selectively replicate the mutagenized (non-uracil containing) strand, and single stranded phage DNA prepared from recombinant (white) plaques. Several recombinants were subjected to DNA sequence analysis and Der f II cDNAs with the corrected $I_{54}$ sequence subcloned as EcoRI/PstI fragments into EcoRI/PstI digested pTrc99A $His_6$ Amb a I.1 RRS.

Since one previous work had shown that the pET11d vector was, in most cases, capable of expressing recombinant proteins at higher levels than the pTrc99A vector, the two mite group II cDNAs were subcloned as $H_6$ fusion proteins into T7 vector. pTrc ppA $His_6$ f II RRS and pTrc99A $His_6$ p II RRS were Nco I/NheI digested, the cDNA inserts isolated, and subcloned into NcoI/NheI digested pET11d $His_6$ pI (Fig. 1). Recombinant plasmids were transformed into BL 21 [DE3] bacteria for expression.

BL21 DE3 host bacteria harboring the pET11d mite allergen expression constructs were freshly streaked onto a BHI agar plate (3.7% wt./vol. Difco Brain Heart Infusion; 1.5% wt./vol. Difco agar) supplemented with 200 μg/ml ampicillin and incubated overnight at 37°C. A single colony was innoculated into a 2 ml of 200 μg/ml ampicillin/BH1 media (3.7% wt./vol. Difco Brain Heart Infusion) and shaken at 300 rpm at 37°C until turbid but not saturated. The 2 ml culture was then added to 100 ml of 200 μg/ml ampicillin/BH1 media, shaken at 300 rpm at 37°C until turbid but not saturated. at which point the culture was divided into 18 X 500 ml (9 litres) of 200 μg/ml ampicillin/BH1 media and shaken at 300 rpm at 37°C. When the $OD_{595}$ of the culture reached 1.0, expression of the recombinant molecules was induced by the addition of IPTG to 400 μM, and allowed to continue for two hours.

Bacteria were harvested by centrifugation at 10,000 X g for 15 minutes, and resuspended in $1/20^{th}$ volume of lysis buffer (0.2 mg/ml lysozyme, 100 mM $NaPO_4$ pH 8.0, 50 mM NaCl), incubated on ice for 30 minutes, and frozen at -70°C. The frozen bacteria were fractured by a quick thaw at 37°C and then sonicated 5 times for 20 seconds at 30 second intervals. The sonicated samples were centrifuged at 15.000 X g to separate soluble and particulate bacterial proteins. The soluble proteins were poured off, and the pelleted protein resuspended in 6 M guanidine HCl, 100 mM 2-mercaptoethanol, 100 mM $NaPO_4$, 10 mM Tris pH 8.0. This suspension was subjected to centrifugation at 15,000 X g, and the supernatant removed, adjusted to pH 8.0 with 10 N NaOH, and applied to an NTA agarose column that had been equilibrated in 6 M guanidine HCl, 100 mM $NaPO_4$. 10 mM Tris pH 8.0. The column was washed in 6 M guanidine HCl, 100 mM $NaPO_4$, 10 mM Tris pH 8.0 until the $OD_{280}$ of the effluent reached background. The column buffer was then switched to 8 M urea, 100 mM $NaPO_4$, 10 mM Tris pH 8.0. After equilibration, a more stringent wash was performed in 8 M urea, 100 mM NaOAc, 10 mM Tris pH 6.3 until the $OD_{280}$ of the effluent reached background. Recombinant mite protein (as an $H_6$ fusion) was then eluted in 8 M urea, 100 mM NaOAc, 10 mM Tris pH 4.5 and collected in aliquots whose $OD_{280}$ profile was monitored. The protein peak was dialyzed 3 times into 500 volumes of PBS for human T-cell analysis. Yield ranged from 10 to 70 mg of recombinant protein per liter with purity (as determined by densitometric scanning) ranging from 80 (Der f II) to 95%.

To further purify the recombinant Der f II protein allergen, Reverse Phase HPLC chromatography was applied. Approximately 100 mg of $His_6$-Der f II protein was reduced in 20 mM DTT at 36°C for 30 minutes and then applied to a Pharmacia PRO RPC HR 10/10 column in 0.1% vol./vol. $TFA/H_2O$. The flow rate for the column was 1.5 ml/min. with a gradient of 0-70% acetonitrile in 0.1 % TFA over 40 minutes, followed by a gradient of 70-100% acetonitrile in 0.1% TFA. $His_6$-Der f II protein eluted between 54-96% acetonitrile. Fractions detected at 214 nm and 280 nm were analyzed for purity by SDS-polyacrylamide gel electrophoresis/densitometric scanning. Those fractions with $His_6$-Der f II protein of purity greater than 95% were subsequently utilized for human T-cell mapping of the allergen. Yield from the preparative Reverse Phase HPLC was approximately 69%.

Determination of Nucleotide Sequence Polymorphisms in the Der p I. Der p II and Der f II Allergens

It was expected that there were sequence polymorphisms in the nucleic acid sequence coding for Der I. Der p II. Der f I and Der f II, due to natural allelic variation among individual mites. Several nucleotide and resulting amino acid sequence polymorphisms have been discovered during the sequencing of different Der p I, Der p II and Der f II clones. The amino acid sequence polymorphisms are shown in Figs. 22, 23 and 24.

## Example III Synthesis of Overlapping Peptides

Der p I, Der f I, Der p II, and Der f II overlapping peptides as shown in Figs. 3 and 4 were synthesized using standard Fmoc/tBoc synthetic chemistry and purified by dialysis or Reverse Phase HPLC. The amino acid residues of the synthesized peptides are in brackets by the peptide name and the amino acid sequence (in single letter code) is next to the peptide name. The peptide names are consistent throughout the Figures. The Der p I, Der f I, Der p II and Der f II proteins were divided into overlapping peptides in such a way that the overlapping peptides of Der p I and Der f I as well as for Der p II and Der f II have corresponding amino acid residue numbers, e.g. DP I-1 and DF I-1 both contain amino acid residues 1-20 of the Der p I and Der f I allergens, respectively. This correspondence in amino acid position between the Der p and Der f peptides was done purposefully in order to best test for cross-reactivity of Der p and Der f T cell epitopes and, thus, determine peptides which, upon administration to an individual sensitive to dust mite, would treat sensitivity to both Der p and Der f allergens. In the design of the overlapping peptides the relationship of the Group I and Group II allergens at the level of T cell cross-reactivity was considered. In addition, the function of the Group I allergens as serine proteases was considered and the amino acid sequences of other known serine proteases, e.g., papain, actinidin, were examined to identify similar conserved and variable regions within the Group I allergens. It was expected that conserved regions within the Group I allergens would contain "shared" T cell epitopes.

## Example IV Mite Allergic Patient Primary T Cell Responses to Der p I and Der p II Proteins and Peptides

Peripheral blood mononuclear cells (PBMC) were purified by Ficoll-Hypaque centrifugation of a peripheral blood specimen from mite-allergic patient R.B. and were assayed for proliferation in response to various antigens, i.e., affinity-purified Der p I, affinity purified Der p II. various Der p I and Der p II peptides. For assay, 5 X 10^4 PBMC were cultured in triplicate microwells for 7 days at 37°C in the presence of various concentrations of antigen in 200 μl RPMI-1640 containing 5% human AB serum. Each well then received 1 μCi tritiated thymidine for 16 hours. The counts incorporated were collected onto glass fiber filters and processed for liquid scintillation counting. Table I shows the results of this assay. The CPM +/- standard deviation are shown. The stimulation index of each response (S.I.) is the ratio of the $^3$H-thymidine CPM incorporated by the cells in response to antigen divided by the $^3$H-thymidine CPM incorporated by cells in medium only. The results indicate that this patient responds with an S.I. of at least 2.0 to peptides DP I-1, DP I-3, DP I-8, DP I-10, DP I-5.2, DP II-4 and DP II-9. Thus, these peptides contain Der p I or Der p II T cell epitopes recognized by T cells from this particular allergic patient.

TABLE I

| Antigen | Concentration μg/ml | CPM + S.D. | S.I. |
|---|---|---|---|
| Medium | -- | 1071±30 | -- |
| Der p I | 10 | 920±15 | 0.9 |
| | 30 | 2122±93 | 2.0 |
| | 100 | 1492±13 | 1.4 |
| DP I-1 | 10 | 1099±48 | 1.0 |
| | 30 | 3527+73 | 3.3 |
| | 100 | 2746±81 | 2.6 |
| DP I-2 | 10 | 1395±47 | 1.3 |
| | 30 | 1283±34 | 1.5 |
| | 100 | 1486±38 | 1.4 |
| DP I-3 | 10 | 2608±52 | 2.4 |
| | 30 | 1561±13 | 1.5 |
| | 100 | 5252±67 | 4.9 |

Table continued

| Antigen | Concentration μg/ml | CPM + S.D. | S.I. |
|---|---|---|---|
| DP I-8 | 10 | 1439±32 | 1.3 |
| | 30 | 1045±32 | 1.0 |
| | 100 | 2272±40 | 2.1 |
| DP I-10 | 10 | 1936±50 | 1.8 |
| | 30 | 3042±89 | 2.8 |
| | 100 | 2644±63 | 2.5 |
| DP I-5.2 | 10 | 1374±20 | 1.3 |
| | 30 | 2241±87 | 2.1 |
| | 100 | 3132±111 | 2.9 |
| Der p II | 10 | 1113+35 | 1.0 |
| | 30 | 2104±43 | 2.0 |
| | 100 | 1057±27 | 1.0 |
| DP II-4 | 10 | 2126±25 | 2.0 |
| | 30 | 1979±94 | 1.8 |
| | 100 | 2314±116 | 2.2 |
| DP II-9 | 10 | 3970±87 | 3.7 |
| | 30 | 4464±86 | 4.2 |
| | 100 | 2237±53 | 2.1 |

## Example V T Cell Epitope Studies with Der p I

Peripheral blood mononuclear cells (PBMC) were purified by Ficoll-Hypaque centrifugation of 60 ml of heparinized peripheral blood from house dust mite-allergic individuals who exhibited clinical symptoms of mite allergy and who were skin test positive for house dust mite.

$10^7$ PBMC from individual 543 were cultured in 10 ml RPMI-1640 containing 5% pooled human AB serum and supplemented with glutamine, penicillin, streptomycin and HEPES buffer in the presence of 20 μg/ml purified native Der p I/ml at 37°C for 7 days. Viable cells were then purified by Ficoll-Hypaque centrifugation and cultured for 2 additional weeks in RPMI-1640/5% AB serum containing 5 units recombinant human IL-2/ml and 5 units recombinant human IL-4/ml. The resting T cells were then tested in a secondary proliferation assay to assess T cell responses to various house dust mite proteins and peptides. For assay, 2 X $10^4$ resting T cells were cultured in 200 μl of RPMI-1640/5% AB serum for 3 days at 37°C in the presence of 2 X $10^4$ autologous Epstein-Barr virus transformed B cells (20,000 Rads) as antigen presenting cells with various concentrations of purified native Der p I or synthetic Der p I peptides. Each well then received 1 μCi tritiated thymidine for 16 hours. The counts incorporated were collected onto glass fiber filters and processed for liquid scintillation counting. Medium alone, acting as negative control, contained no allergen or peptide. The results of this experiment indicate that this particular patient responds with an S.I. of at least 2.0 to several peptides derived from the Der p I protein, including DP I-1, DP I-2, DP I-4. DP I-11, DP I-5, DP I-13, DP I-15, DP I-6.1, DP I-8, DP I-9, DP I-16, DP I-10 and DP I-17 (data not shown).

The above procedure was followed with a number of other house dust mite allergic individuals except a) in individual cases, the length of time of cultivation with IL-2 and IL-4 varied; b) in individual cases, the T cells were primed with either purified native (N) or recombinant (R) Der p I protein at either 20 μg/ml or 10 μg/ml; and c) in individual cases, x-irradiated (3500 Rads) autologous PBMC were used as antigen presenting cells in the secondary proliferation assay. In addition, three peptides (DP I-11 (SEQ ID NO: 117), DP I-12 (SEQ ID NO: 118), and DP II-3 (SEQ ID NO: 119)) were found to contain a low number of conservative changes from the native sequence in their amino acid sequence. Three additional peptides (DP I-11.1 (SEQ ID NO: 13), DP I-12.1 (SEQ ID NO: 14) and DP II-3.1 (SEQ ID NO: 43) were synthesized with no changes from the native sequence. Some T cell analysis was done with the original peptides (i.e., DP I-11, DP I-12 and DP II-3). Following T cell analysis conducted with the additional peptides (i.e., DP I-11.1, DP I-12.1 and DP II-3.1) no significant difference in mean S.I. or percentage of positive responses between the original peptides and the additional peptides was detected. Thus, the data from both groups of peptides was pooled.

Individual results were considered positive and used if the individual responded to the Der p I protein and at least one peptide derived from Der p I at an S.I. of 2.0 or greater. A summary of the results of 33 positive experiments is shown in Fig. 5. The resting T cell lines primed with recombinant or native Der p I-stimulated PBMC were tested for reactivity to synthetic Der p I peptides. The maximum response for each peptide in a titration of the antigen is expressed as the T cell stimulation index (S.I.). The S.I. is the counts-per-minute (CPM) incorporated by cells in response to the peptide divided by the CPM incorporated by cells in medium only. An S.I. value greater than the background level indicates that the peptide contains a T cell epitope. However, only individual S.I. values greater than or equal to 2.0 (a response two-fold or greater over background), referred to herein as "positive" results, were used in calculating mean T cell stimulation indices for each peptide for the patient or group of patients tested. In parentheses above each bar on the histogram are the mean T cell stimulation indices calculated after discarding the highest and lowest positive responses for each peptide to minimize the effect of extreme outliers. The T cell stimulation index is calculated by:

$$\frac{(\text{CPM of T cell}+\text{APC}+\text{Antigen})}{\text{CPM of T cell }+\text{APC}+\text{Control}}$$

The bar represents the cumulative rank of the peptide response in the group of patients. To determine the cumulative rank, the 5 peptides with the highest S.I. in each individual were determined and assigned a numerical rank in descending order, with 5 representing the strongest response. The ranks for each peptide were then summed in the group of patients to determine the cumulative rank for the peptide. Above each bar is the percent of positive responses with an S.I. of at least 2 to the peptide in the group of patients tested. Given the percent positive and the mean T cell stimulation index, the positivity index (P.I.) for each peptide can be calculated. The P.I. for each individual is determined by multiplying the mean S.I. by the percent of individuals, in a population of individuals sensitive to house dust mite (e.g. preferably at least 15 individuals, more preferably at least 30 individuals or more) who responded with an S.I. of at least 2.0 to that peptide (e.g., for DP I-1 in Fig. 5, the P.I. would be about 343 (73% x 4.7). The P.I. therefore represents both the strength of a T cell response to a peptide (S.I.) and the frequency of a T cell response to a peptide in a population of individuals sensitive to house dust mite. Fig. 5 demonstrates that peptides DP I-1, DP I-2, DP I-3, DP I-4, DP I-5, DP I-6.1, DP I-7.1, DP I-8. DP I-9, DP I-16, and DP I-10 contain significant regions of T cell reactivity in this panel of patients.

### Example VI T Cell Epitope Studies with Der f I

Experiments similar to those of Example V were performed to determine the T cell-reactive areas of the Der f I protein. For example, PBMC from house dust mite-allergic patient 783 were isolated as described in Example V and were stimulated in vitro with recombinant purified Der f I at 20 μg/ml. The results of the proliferation assay with Der fI peptides using x-irradiated (3500 Rads) autologous PBMC as antigen presenting cells indicate that T cells from this patient respond to the peptides DF I-8.1, DF I-9. DF I-6, DF I-10, DF I-2.1, DF I-3, DF I-11, DF I-5, DF I-1, and DF I-17 (data not shown).

The above procedure was followed in a number of patients except in individual cases, T cell lines were primed with affinity purified Der f I at 20μg/ml or at 10 μg/ml and x-irradiated (25,000 Rads) autologous Epstein-Barr virus transformed B cells were used as antigen presenting cells. A summary of the results of 16 positive experiments is shown in Fig. 6. The data was analyzed as described in Example V. The data indicate that significant areas of T cell reactivity in the Der f I protein are found in the peptides DF I-1, DF I-2, DF I-3, DF I-4, DF I-11, DF I-5, DF I-6, DF I-7, DF I-14, DF I-15> DF I-8.1 and DF I-9.

### Example VII A Study Indicating the Cross-reactivity of Der p I and Der f I T Cell Epitopes

The Der p I and Der f I proteins are very homologous (81 % identity). Thus, experiments, similar to those of Example V, were carried out to determine the T cell responses of Der-p I primed T cell lines when challenged with various Der p I peptides and substantially matching D f II peptides. T cell lines were primed in vitro as described in Example V and tested for response to a set of substantially matching Der p I and Der f I peptides (e.g., DP I-1 (amino acid residues 1-20 of Der p I) or DF I-1 (amino acid residues 1-20 of Der f (I). The data was analyzed as described in Example V except the highest and lowest S.I. values of the positive responses to each peptide were not omitted from the mean S.I. calculations. A summary of a number of such experiments is shown in Fig. 7. The results of 14 positive experiments indicate that Der p I-primed T cells respond to various Der f I peptides indicating cross-reactivity within the Group I allergens. Der p I primed T cells respond significantly to peptides DF I-1, DF I-2, DF I-3, DF I-4, DF I-11, DF I-12, DF I-15, DF I-8, DF I-9, DF I-15 and DF I-6. In some patients, the Der f I peptide was a more potent stimulator of Der p I-primed T cells than the corresponding Der p I peptide. Fig. 8 shows the results of inverse experiments in which T cells from a number of patients were primed in vitro to the Der f I protein and analyzed for response to various Der p- I peptides and a set of substantially matching Der f I peptides. The results of 8 positive experiments indicate that Der f I primed T cells respond significantly to peptides DP I-1, DP I-3, DP I-4, DP I-11/11.1, DP I-14, DP I-5, DP I-15, and DP I-8.

**Example VIII T Cell Epitope Studies with Der p II.**

Experiments similar to those of Example V were performed to determine the T cell-reactive areas of the Der p II protein. For example, PBMC from house dust mite-allergic patient 348 were isolated as described in Example V and were stimulated in vitro with 20 μg/ml purified native Der p II. The results of a proliferation assay using x-irradiated (25,000 Rads) Epstein-Barr virus transformed autologous B cells as antigen-presenting cells with various Der p II peptides demonstrate that this particular patient responds well to peptides DP II-1. DP II-7, DP II-8, DP II-2, and DP II-9 (data not shown).

The above procedure was followed with a number of patients except in individual cases, T cell lines were primed with recombinant Der p II at 20 μg/ml or at 3 μg/ml and x-irradiated (3500 Rads) autologous PBMC were used as antigen-presenting cells. A summary of the results of 26 positive experiments is shown in Fig. 9. The data was analyzed as described in Example V, except the ranked sum of peptide responses was analyzed assigning a value of 3, 2 or 1 to the three highest S. I. responses. Areas of significant T cell reactivity within the Der p II protein for this panel of patients are found in peptides DP II-1, DP II-2, DP II-3, DP II-4, DP II-7, DP II-8 and DP II-9.

**Example IX T Cell Epitope Studies with Der f II**

Experiments similar to those of Example V were performed to determine the T cell-reactive areas of the Der f II protein. For example, PBMC from house dust mite-allergic patient 384 were stimulated in vitro with purified recombinant Der f II and the T cell line was then challenged in the presence of x-irradiated (25,000 Rads) autologous Epstein-Barr virus transformed B cells as antigen presenting cells with various overlapping Der f II peptides. The results of this proliferation assay indicate that T cells from this particular patient respond well to peptides DF II-1, DF II-2, DF II-3.1, DF II-4.5, DF II-15, DF II-16, and DF II-19.1 (data not shown).

The above procedure was followed with 10 patients, except in individual cases, T cell lines were primed by stimulating the patient PBMC with 20 μg/ml or 3 μg/ml purified native Der f II, and were assayed in the presence of x-irradiated (3500 Rads) autologous PBMC as antigen-presenting cells. A summary of the results of 10 positive experiments is shown in Fig. 10. The data was analyzed as detailed in Example IX, except the highest and lowest S.I. values of the positive responses to each peptide were not omitted from the calculations. The data indicate that significant areas of T cell reactivity within the Der f II protein are found in peptides DF II-1, DF II-2, DF II-13.1, DF II-4.5, DF II-15, DF II-17, and DF II-19.1.

**Example X A Study Indicating the Cross-Reactivity of Der p II and Der f II T Cell Epitopes**

A study similar to that described in Example VII was carried out to determine the T cell cross-reactivity of the Der p II and Der f II proteins. T cells primed with the Der f II protein were challenged with various Der f II peptides and a set of substantially matching Der p II peptides. A summary of the results of 10 positive experiments is shown in Fig. 11. The results indicate that Der f II primed T cells respond significantly to peptides DP II-1, DP II-3/3.1, DP II-4, and DP II-7. Fig. 12 shows the results of inverse experiments in which T cells from a number of patients were primed in vitro to the Der p II protein and analyzed for response to various Der f II peptides. The results of 26 positive experiments indicate that Der p II primed T cells respond significantly to peptides DF II-1, DF II-4.5, DF II-15, DF II-17, DF II-18 and DF II-19.1.

**Example XI Synthesis of Dominant Peptides**

Based on the analyses described in Examples V-X, major areas of T cell reactivity within Der p I, Der p II, Der f I and Der f II were identified. In each study, all of the patients tested responded to the protein allergen (e.g., Der p I) and at least one peptide derived from a major area of T cell reactivity within the protein. Seven regions (Region 1, Region 2, Region 3, Region 4, Region 5, Region 6a and Region 6b) of major T cell reactivity were identified in the Der p I and Der f I proteins. These regions are defined as follows: Region 1, amino acid residues 1-28 of the Der p I and Der f I proteins; Region 2, amino acid residues 36-68 of the Der p I and Der f I proteins; Region 3, amino acid residues 74-109 of the Der p I and Der f I proteins; Region 4, amino acid residues 118-139 of the Der p I and Der f I proteins; Region 5, amino acid residues 141-166 of the Der p I and Der f I proteins; and Region 6a, amino acid residues 161-185 of the Der p I and Der f I proteins and Region 6b, amino acid residues 173-201 of the Der p I and Der f I proteins.

Similarly, four regions of major T cell reactivity (Region 7, Region 8, Region 9, and Region 10) were identified in the Der p II and Der f II proteins. These regions are defined as follows: Region 7, amino acid residues 1-26 of the Der p II and Der f II proteins; Region 8, amino acid residues 33-67 of the Der p II and Der f II proteins; Region 9, amino acid residues 79-104 of the Der p II and Der f II proteins: and Region 10. amino acid residues 107-129 of the Der p II and Der f II proteins. Based in part on the T cell reactivity described in Examples V-X, peptides derived from Der p 1. Der f I, Der p II, and Der f II were selected and modified by addition or deletion of amino acid residues at either the 5' or 3' end of the peptide. In designing these selected peptides, various factors were considered, including the ranked sum of the overlapping peptides, the percentage of responses with an S.I. of at least 2.0 to the peptides, the potential cross-reactivity of the peptides, the difficulty of manufacture of the peptides, etc. T cell studies similar to those described in Examples V-X were performed using these selected peptides to more precisely define the major areas of T cell reactivity within Regions 1-6a and 6b of the Der p I and Der f I protein and Regions 7-10 of the Der p II and Der f II protein.

The results of T cell studies using selected peptides derived from the Der p I, the Der f I, the Der p II and the Der f II proteins are shown in Figs. 13-18a-d. The procedure described in Example V was followed with T cell lines from a number of patients primed in vitro to the Der p I protein then analyzed for response to selected peptides derived from the Der p I sequence. The results of 33 positive experiments shown in Fig. 13 indicate that the Der p I primed T cells respond significantly to peptides found in peptides DP I-21.1, DP I-21.2, DP I-22.2, DP I-25.2, DP I-22.1, DP I-23.1, DP I-23.2, DP I-26.1, and DP I-28.1.

Similarly, the procedure described in Example VI was followed with T cell lines from 9 patients primed in vitro to the Der f I protein then analyzed for response to selected peptides derived from the protein. The data was analyzed as described in Example VI. The results of the 9 patients shown in Fig. 14 demonstrate T cell reactivity to selected peptides from Der f I.

In another experiment, the T cell lines from a number of patients were primed in vitro to the Der p I protein and analyzed for response to selected peptides derived from the Der p I protein and a set of substantially matching peptides derived from the Der f I protein. The data from 30 positive experiments was analyzed as described in Example V. As shown in Fig. 15a, the Der p I primed T cells respond significantly to peptides DF I-21.1, DF I-21.2, DF I-23.1, DF I-22.2, DF I-22.3. DF I-22.4, DF I-23.2, DF I-25.1, DF I-26.1 and DF I-27.1. Fig. 15b is a subset of the data shown in Fig 15a and shows the response of Der p I primed T cells to peptides analyzed by ranked sum. Fig. 15b shows that DPI-23.1 has the highest ranked sum of this group of peptides in this study. Fig. 16a shows the results of the inverse experiment in which T cells from 9 patients were primed in vitro to the Der f I protein and challenged with selected Der f I peptides and a set of substantially matching Der p I peptides. The results indicate that Der f I primed T cells from 9 patients respond to selected peptides from Der p I. Fig. 16a shows that DF I-22.1 and DF I-25.1 have the highest stimulation indexes of this group of peptides. Fig. 16b is a subset of the same data as shown in Fig. 16a and shows the response of preferred Der f I and Der p I peptides is shown. Fig 16b shows that DF1-22.2 has the highest stimulation index of this group of preferred peptides in this experiment.

Another experiment following the procedure described in Example VIII analyzed the response of 29 patients primed in vitro to the Der p II protein and challenged with selected peptides derived from the Der p II sequence. Fig. 17a shows that Der p II primed T cells from one patient respond to selected peptides from Der p II. Fig. 17b shows the results from an experiment similar to that shown in Fig. 17a with a set of 30 patients and with the high and low omitted from the mean. Fig. 17b shows that DPII-20.6 has the highest ranked sum of this group of preferred peptides in this study. Fig. 18a shows the inverse experiment in which T cells from 1 patient were primed in vitro to the Der f II protein and challenged with selected peptides derived from the Der p II sequence. Fig. 18a shows that Der f II primed T cells from 10 patients respond to selected peptides from Der p II. As shown in Fig. 18a DP II-25 has the highest stimulation index. Fig. 18b is a subset of the same data shown in Fig. 18a and shows the response of native Der f II primed T cell lines to preferred Der p II peptides analyzed by ranked sum. As is shown in Fig. 18b, DPII-25.2 has the highest ranked sum of the preferred peptides in this study.

### Example XII Study Indicating Cross-reactivity of Selected Group I and Group II Epitopes

A study similar to that described in Example VII was carried out with T cells from 4 matched patients primed in vitro with Group I proteins from Der f and Der p then analyzed for response to selected preferred peptides from Der p I and Der f I. The results in Fig. 18c demonstrate that T cell reactivity to a number of the selected preferred Der p I peptides was essentially equivalent for their Der f I counterpart and vice-versa.

Fig. 18d shows the results of a similar study with T cells from 6 matched patients primed in vitro with Group II proteins from Der p and Der f, then analyzed for response to selected preferred Der p II and Der f II peptides. Similar to the results in Fig. 18c, T cell reactivity to a number of the selected preferred peptides of Der p II are essentially equivalent to their Der f II counterparts and vice-versa.

### Example XIII Direct Binding, Assay of IgE to Mite Allergen Proteins and Peptides

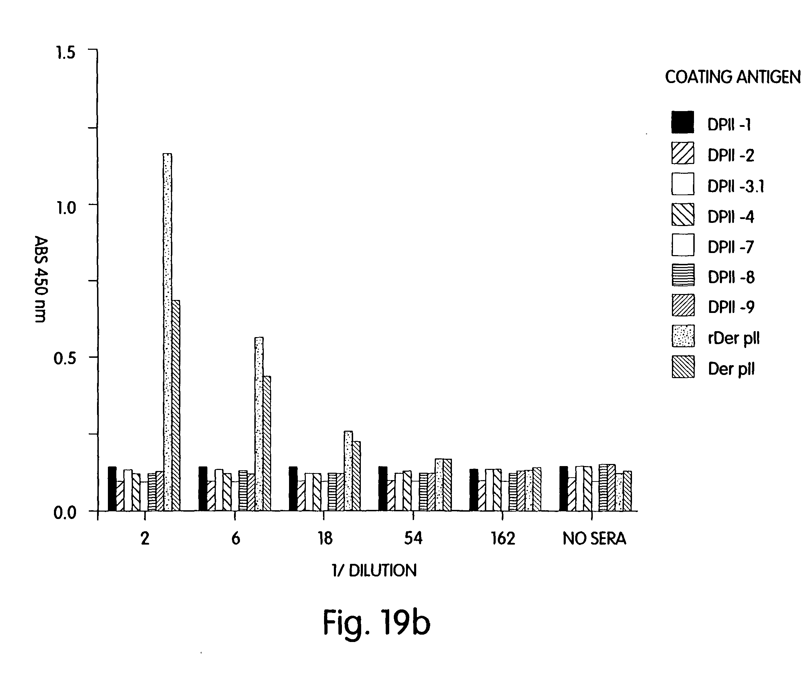

Coming assay plates (#25882-96) were coated with 5 μg/ml of each coating antigen listed in Figs. 19, 20 and 21 at 50 μl/100ml/well and incubated overnight at 4°C. The coating antigens were removed and the wells are blocked with 0.5% gelatin in PBS, 300 μl/well for 2 hours at room temperature. Pooled human plasma (a mix of plasma samples from 20 patients that were skin test positive for commerical mite extract) was serially diluted with PBS-Tween 20 (PBS with 0.05% nonionic detergent Tween-20 Sigma, St. Louis, MO) and 100 μl/well a\was added and incubated overnight at 4°C (plasma dilutions were tested in duplicate). The second antibody (biotinylated goat anti-Human IgE, 1:1000, Kirkegaard & Perry Laboratories Inc, Gaithersburg, MD), was added at 100 μl/well for one hour at room temperature. This solution was removed and streptavidin-HRPO, 1:10000, (Southern Biotechnology Associates, Inc., Birmingham, AL) was then added at 100 μl/well for one hour at room temperature (all wells are washed three times with PBS-Tween between each incubation step). TMB Membrane Peroxidase Substrate system (Kirkegaard & Perry Laboratories) was freshly miced, and added at 100ml/well. The color was allowed to develop for 2-5 minutes. The reaction was stopped by the addition of 100ml/well of 1M phosphoric acid. Plates were read on a Microplate IL310 Autoreader (Biotech Instruments, Winooski, VT) with a 450 nm filter. The absorbance levels of duplicate wells were averaged. The graphed results (log of the dilution versus absorbance) of the ELISA assays are shown in Figs. 19a-b, 20a-b and 21a-h. The order of coating antigens listed vertically in these figure legends corresponds in order from left to right to the coating antigens listed for each histogram.

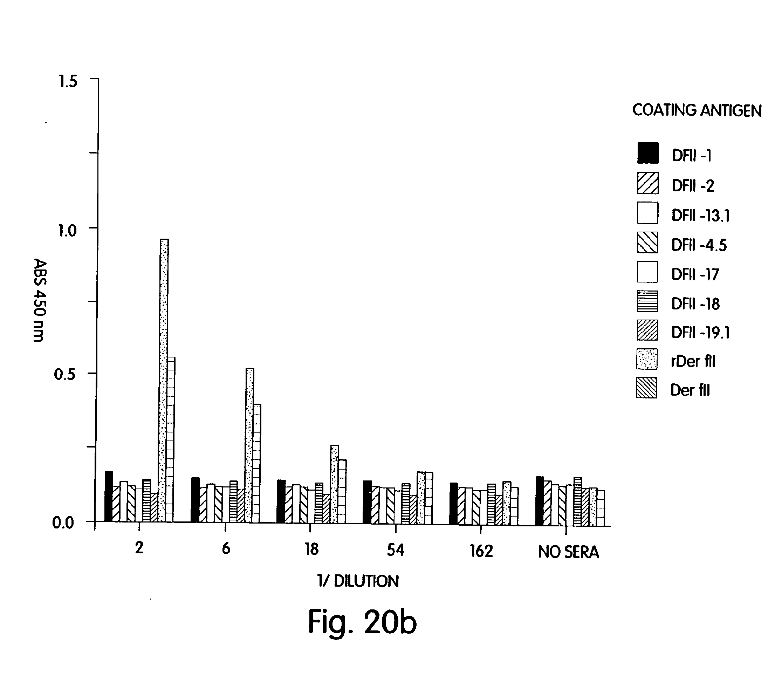

The results of the ELISA assay shown in Fig. 19b demonstrate good binding of both biochemically purified Der p II and recombinant Der p II (rDer p II) with human IgE and no detectable binding to the Der p II peptides. The IgE binding to the Der p set of peptides and proteins (Figs. 20a and 20b) shows the same pattern of reactivity as the Der f set. That is, no detectable binding to Der f I or Der f II peptides or recombinant Der f I with binding to only biochemically purified Der f I and recombinant and biochemically purified Der f II. In both cases there appears to be better binding to recombinant Der p II or Der f II than to the biochemically purified forms. All the conclusions derived from the above ELISA assay data were corraborated by another assay method, dot blots on nitrocellulose paper, using the same set of antibody and antigen reagents.

The antigen preparation that was used as a positive control was a mixture of the four major biochemically purified mite allergens (term PMA for Purified Mite Allergen); Der f I, Der f II, Der p I and Der p II. The stock was generated at a concentration of 100 μgs of each protein per millimeter or 400 μgs total protein/ml. This preparation was used on each coated ELISA plate. The results from these ELISA assays are shown in Fig. 21a-h. There is clear binding to either the purified or recombinant protein or the PMA antigen preparation on each plate indicating good IgE reactivity. However, the PMA antigen preparation, does exhibit a high degree of non-specific reactivity shown in the background dilution where no first antibody solution was added. This non-specific reactivity occurs between the PMA antigen and the biotinyulated second antibody and does not compromise the finding of specific IgE reactivity to the antigen. Using a quantitative value of two-fold over background at the highest plasma concentration as a positive reading, there is no detectable IgE reactivity to any one of the 56 peptides screened by this assay method.

**Claims**

1. An isolated modified peptide of a protein allergen of the genus *Dermatophagoides* comprising at least one T cell epitope of said protein allergen, said peptide being DP I-21.7 having the sequence:

K S I N G N A P A E I D L R Q L R T V T P I R L Q.

2. An isolated nucleic acid having a sequence encoding a peptide of claim 1.

3. An isolated modified peptide of any one of the preceding claims which has been further modified by the addition of charged amino acids or charged amino acid pairs to said peptide to increase solubility.

4. An isolated peptide comprising at least two regions, each region comprising at least one T cell epitope of a protein allergen of the genus *Dermatophagoides,* said regions derived from the same or different protein allergens of the genus *Dermatophagoides,* wherein at least one region comprises an amino acid sequence selected from:

> a) DPI-21.1 (SEQ ID NO:27):
> b) DPI-21.2 (SEQ ID NO:28):
> c) DPI-22.1 (SEQ ID NO:29):
> d) DPI-23.1 (SEQ ID NO:33):
> e) DPI-25.2 (SEQ ID NO:36):
> f) DPI-26.1 (SEQ ID NO:37):
> g) DPI-28.1 (SEQ ID NO:39):
> h) DPI-1 (SEQ ID NO:9):
> i) DFI-1 (SEQ ID NO:72):
> j) DFI-21.1 (SEQ ID NO:90):
> k) DFI-22.1 (SEQ ID NO:92):
> 1) DFI-23.1 (SEQ ID NO:95):
> m) DFI-25.1 (SEQ ID NO:97):
> n) DPII-1 ((SEQ ID NO:41):
> o) DPII-1.2 (SEQ ID NO:58):
> p) DPII-2.0 (SEQ ID NO:56):
> q) DPII-20.3 (SEQ ID NO:53):
> r) DPII-21 (SEQ ID NO:62):
> s) DPII-22 (SEQ ID NO:63):
> t) DPII-25 (SEQ ID NO:69):
> u) DPII-25.2 (SEQ ID NO:71):
> v) DFII-2 (SEQ ID NO: 104):
> w) DFII-4.5 (SEQ ID NO:107):
> x) DFII-15 (SEQ ID NO:109):
> y) DFII-17 (SEQ ID NO:111):
> z) DFII-19.1 (SEQ ID NO:114):
> aa) DFI-22.2 (SEQ ID NO:93): and
> bb) DPII-20.6 (SEQ ID NO:56)

and wherein at least one region comprises the amino acid sequence DPI-21.7 having the sequence:

K S I N G N A P A E I D L R Q L R T V T P I R L Q.

5. An isolated peptide as claimed in claim 4, wherein said peptide comprises the amino acid sequence DP I-21.7 together with a combination of regions selected from:

> a) DF I-22.2, DP I-23.13, DP I-26.1, DP II-20.6, DP II-22.3 and DP II-25.2;
> b) DFI-22.2, DP I-23.11, DP I-26.3, DP II-20.7, DP II-22.5 and DP II-25.4;
> c) DF I-22.2, DP I-23.12, DP I-26.4, DP II-20.6, DP II-22.6 and DP II-25.2;
> d) DF I-22.2, DP I-23.5, DP I-26.5, DP II-20.7, DP II-22.7 and DP II-25.3;
> e) DF I-22.2, DP I-23.6, DP I-26.2, DP II-20.6, DP II-22.8 and DP II-25.4;
> f) DP I-22.2, DP I-23.7, DP I-26.3, DP II-20.7, DP II-22.9 and DP II-25.2;
> g) DF I-22.2, DP I-23.8, DP I-26.4, DP II-20.6, DP II-22.3 and DP II-25.4;
> h) DF I-22.2, DP I-23.9; DP I-26.1, DP II-20.7; DP II-22.4 and DP II-25.3;
> i) DF I-22.2, DP I-23.13, DP I-26.2, DP II-20.6, DP II-22.5 and DP II-25.4;
> j) DF I-22.2, DP I-23.14, DP I-26.3, DP II-20.7, DP II-22.6 and DP II-25.3;
> k) DF I-22.2, DP I-23.15, DP I26.4, DP II-20.6, DP II-22.6 and DP II-25.3;

l) DF I-22.2, DP I-23.16, DP I-26.5, DP II-20.6, DP II-22.3 and DP II-25.2;
m) DF I-22.2, DP I-23.17, DP I-26.2, DP II-20.6, DP II-22.4 and DP II-25.3;
n) DF I-22.2, DP I-23.8, DP I-26.3, DP II-20.6, DP II-22.5 and DP II-25.4;
o) DF I-22.2, DP I-23.9, DP I-26.4, DP II-20.6, DP II-22.6 and DP II-25.3; or
p) DF I-22.2, DP I-23.13, DP I-26.5, DP II-20.6, DP II-22.7 and DP II-25.4.

6. An isolated peptide of claim 4, wherein said peptide comprises any arrangement of the following combination of regions: X, Y, Z, A, B, C, D;
wherein:

X is DP I-21.7;
Y is DF I-22.2;
Z is DP I-23.1, DP I-23.10, DP I-23.11, DP I-23.12, DP I-23.13, DP I-23.14, DP I-23.15, DP I-23.16, DP I-23.17, DP I-23.5, DP I-23.6, DP I-23.7, DP I-23.8 or DP I-23.9;
A is DP I-26.1, DP I-26.2, DP I-26.3, DP I-26.4 or DP I-26.5;
B is DP I-20.6 or DP II-20.7;
C is DP II-22, DP II-22.6, DP II-22.7, DP II-22.8, DP II-22.9, DP II-22.10, DP II-22.11, DP II-22.3, DP II-22.4 or DP II-22.5; and
D is DP II-25.2, DP II-25.3 or DP II-25.4.

7. An isolated peptide of claim 4, wherein said peptide has a specific sequential arrangement of amino acid sequences said arrangements having the formula:

a) ADYCZBX;
b) DYZCAXB; or
c) DXZACBY;

wherein X, Y, Z, A, B, C and D are as defined in claim 6.

8. An isolated nucleic acid of claim 2 having a sequence encoding all or an epitope-containing portion of a peptide of any one of claims 4 to 7.

9. An isolated peptide of any (appropriate) preceding claim produced in a host cell transformed with the nucleic acid of claim 2 or claim 8.

10. An isolated peptide of a protein allergen of the genus *Dermatophagoides,* said peptide or portion thereof comprising at least one T cell epitope of said protein allergen, said peptide having the formula $X_n$-Y-$Z_m$;
wherein Y is the amino acid sequence DPI-21.7 as defined in claim 1;
$X_n$ are amino acid residues contiguous to the amino terminus of Y in the amino acid sequence of said protein allergen; and
$Z_m$ are amino acid residues contiguous to the carboxy terminus of Y in said protein allergen;
m and n are each 0-30.

11. A therapeutic composition comprising at least one isolated peptide of any one of claims 1, 3 to 7, 9 or 10 and a pharmaceutically acceptable carrier or diluent.

12. A therapeutic composition comprising a pharmaceutically acceptable carrier or diluent and at least two peptides, said peptides each comprising at least one T cell epitope of a protein allergen of the genus *Dermatophagoides*, wherein at least one peptide is selected from:

a) DPI-21.1 (SEQ ID NO:27):
b) DPI-21.2 (SEQ ID NO:28):
c) DPI-22.1 (SEQ ID NO:29):
d) DPI-22.2 (SEQ ID NO:30):
e) DPI-22.3 (SEQ ID NO:31):
f) DPI-22.4 (SEQ ID NO:32):
g) DPI-23.1 (SEQ ID NO:33):
h) DP I-23.2 (SEQ ID NO: 34);

i) DP I-25.1 (SEQ ID NO: 35);

j) DP I-25.2 (SEQ ID NO: 36);

k) DP I-26.1 (SEQ ID NO: 37);

l) DP I-27.1 (SEQ ID NO: 38);

m) DP I-28.1 (SEQ ID NO: 39);

n) DP I-28.2 (SEQ ID NO: 40);

o) DP I-1 (SEQ ID NO: 9);

p) DF I-1 (SEQ ID NO: 72);

q) DF I-21.1 (SEQ ID NO: 90);

r) DF I-21.2 (SEQ ID NO: 91);

s) DF I-22.1 (SEQ ID NO: 92);

t) DF I-22.2 (SEQ ID NO: 93);

u) DF I-22.4 (SEQ ID NO: 94);

v) . DF I-23.1 (SEQ ID NO: 95);

w) DF I-23.2 (SEQ ID NO: 96);

x) DF I-25.1 (SEQ ID NO: 97);

y) DF I-25.2 (SEQ ID NO: 98);

z) DF I-26.1 (SEQ ID NO: 99);

aa) DF I-27.1 (SEQ ID NO: 100);

bb) DF I-28.1 (SEQ ID NO: 101);

cc) DF I-28.2 (SEQ ID NO: 102);

dd) DP II-20 (SEQ ID NO: 50);

ee) DP II-20.1 (SEQ ID NO: 51);

ff) DP II-20.2 (SEQ ID NO: 52);

gg) DP II-20.3 (SEQ ID NO: 53);

hh) DP II-20.4 (SEQ ID NO: 54);

ii) DP II-20.5 (SEQ ID NO: 55);

jj) DP II-20.6 (SEQ ID NO: 56);

kk) DP II-1 (SEQ ID NO: 41);

ll) DP II-1.1 (SEQ ID NO: 57);

mm) DP II-1.2 (SEQ ID NO: 58);

nn) DP II-2.1 (SEQ ID NO:59);

oo) DP II=2.2 (SEQ ID NO: 60);

pp) DP II-2.3 (SEQ ID NO: 61);

qq) DP II-21 (SEQ ID NO: 62);

rr) DP II-22 (SEQ ID NO: 63);

ss) DP II-26 (SEQ ID NO: 64);

tt) DP II-26.1 (SEQ ID NO: 65);

uu) DP II-23 (SEQ ID NO: 66);

vv) DP II-23.1 (SEQ ID NO: 67);

ww) DP II-24 (SEQ ID NO: 68);

xx) DP II-25 (SEQ ID NO: 69);

yy) DP II-25.1 (SEQ ID NO: 70);

zz) DP II-25.2 (SEQ ID NO: 71);

aaa) DF II-1 (SEQ ID NO: 103);

bbb) DF II-2 (SEQ ID NO: 104);

ccc) DF II-13.1 (SEQ ID NO: 105);

ddd) DF II-3.1 (SEQ ID NO: 106);

eee) DF II-4.5 (SEQ ID NO: 107);

fff) DF II-4.3 (SEQ ID NO: 108);

ggg) DF II-15 (SEQ ID NO: 109);

hhh) DF II-16 (SEQ ID NO: 110);

iii) DF II-17 (SEQ ID NO: 111);

jjj) DF II-18 (SEQ ID NO: 112);

kkk) DF II-19 (SEQ ID NO: 113);

lll) DF II-19.1 (SEQ ID NO: 114);

mmm) DF II-21 (SEQ ID NO: 115); and

nnn) DF II-22 (SEQ ID NO: 116);

and wherein at least one peptide is DPI-21.7 having the sequence:

K S I N G N A P A E I D L R Q L R T V T P I R L Q;

wherein said composition comprises a sufficient percentage of T cell epitopes of at least one protein allergen such that upon administration of the composition to an individual sensitive to a house dust mite allergen, T cells of the individual are tolerized to said at least one protein allergen.

13. A composition of claim 12 comprising the amino acid sequence DP I-21.7 as defined above together with a combination of peptides selected from:

    a) DF I-22.2, DP I-23.13, DP I-26.1, DP II-20.6, DP II-22.3 and DP II-25.2;
    b) DF I-22.2, DP I-23.11, DP I-26.3, DP II-20.7, DP II-22.5 and DP II-25.4;
    c) DF I-22.2, DP I-23.12, DP I-26.4, DP II-20.6, DP II-22.6 and DP II-25.2;
    d) DF I-22.2, DP I-23.5, DP I-26.5, DP II-20.7, DP II-22.7 and DP II-25.3;
    e) DF I-22.2, DP I-23.6, DP I-26.2, DP II-20.6, DP II-22.8 and DP II-25.4;
    f) DP I-22.2, DP I-23.7, DP I-26.3, DP II-20.7, DP II-22.9 and DP II-25.2;
    g) DF I-22.2, DP I-23.8, DP I-26.4, DP II-20.6, DP II-22.3 and DP II-25.4;
    h) DF I-22.2, DP I-23.9; DP I-26.1, DP II-20.7; DP II-22.4 and DP II-25.3;
    i) DF I-22.2, DP I-23.13, DP I-26.2, DP II-20.6, DP II-22.5 and DP II-25.4;
    j) DF I-22.2, DP I-23.14, DP I-26.3, DP II-20.7, DP II-22.6 and DP II-25.3;
    k) DF I-22.2, DP I-23.15, DP I26.4, DP II-20.6, DP II-22.6 and DP II-25.3;
    l) DF I-22.2, DP I-23.16, DP I-26.5, DP II-20.6, DP II-22.3 and DP II-25.2;
    m) DF I-22.2, DP I-23.17, DP I-26.2, DP II-20.6, DP II-22.4 and DP II-25.3;
    n) DF I-22.2, DP I-23.8, DP I-26.3, DP II-20.6, DP II-22.5 and DP II-25.4;
    o) DF I-22.2, DP I-23.9, DP I-26.4, DP II-20.6, DP II-22.6 and DP II-25.3; and
    p) DF I-22.2, DP I-23.13, DP I-26.5, DP II-20.6, DP II-22.7 and DP II-25.4.

14. A composition of claim 12 comprising at least two of the peptides X, Y, Z, A, B, C and D as defined in claim 6.

15. The use of a peptide as defined in any one of claims 1, 3 to 7, 9 or 10 in the manufacture of a medicament for treating sensitivity to a house dust mite in an individual; optionally said medicament providing for simultaneous, or sequential administration of a therapeutically effective amount of at least two different compositions of any one of claims 11 to 14.

16. An in vitro method of detecting sensitivity to house dust mite in an individual, comprising combining a blood sample from the individual with at least one peptide of any one of claims 1, 3 to 7, 9 or 10 under conditions appropriate for binding of blood components with the peptide, and determining the sensitivity in the individual to house dust mite; optionally wherein the extent to which binding occurs is determined by assessing T cell function, T cell proliferation or a combination thereof.

17. A peptide of any one of claims 1, 3 to 7, 9 or 10 for use in medicine.


**Revendications**

1. Peptide modifié isolé d'un allergène protéique du genre *Dermatophagoides* comprenant au moins un déterminant antigénique lymphocyte T dudit allergène protéique, ledit peptide étant le peptide DP I-21.7 possédant la séquence :

K S I N G N A P A E I D L R Q L R T V T P I R L Q.

2. Acide nucléique isolé possédant une séquence encodant un peptide selon la revendication 1.

3. Peptide modifié isolé selon l'une quelconque des revendications précédentes qui a été soumis à une modification ultérieure par l'addition audit peptide d'acides aminés chargés ou de paires d'acides aminés chargés afin d'augmenter la solubilité.

**4.** Peptide isolé comprenant au moins deux régions, chaque région comprenant au moins un déterminant antigénique lymphocyte T d'un allergène protéique du genre *Dermatophagoides,* lesdites régions dérivant des mêmes allergènes protéiques ou d'allergène protéiques différents du genre *Dermatophagoides,* dans lequel au moins une région comprend une séquence d'acides aminés choisie parmi le groupe comprenant :

a) DPI-21.1 (SEQ ID NO:27):
b) DPI-21.2 (SEQ ID NO:28):
c) DPI-22.1 (SEQ ID NO:29):
d) DPI-23.1 (SEQ ID NO:33):
e) DPI-25.2 (SEQ ID NO:36):
f) DPI-26.1 (SEQ ID NO:37):
g) DPI-28.1 (SEQ ID NO:39):
h) DPI-1 (SEQ ID NO:9):
i) DFI-1 (SEQ ID NO:72):
j) DFI-21.1 (SEQ ID NO:90):
k) DFI-22.1 (SEQ ID NO:92):
l) DFI-23.1 (SEQ ID NO:95):
m) DFI-25.1 (SEQ ID NO:97):
n) DPII-1 ((SEQ ID NO:41):
o) DPII-1.2 (SEQ ID NO:58):
p) DPII-2.0 (SEQ ID NO:56):
q) DPII-20.3 (SEQ ID NO:53):
r) DPII-21 (SEQ ID NO:62):
s) DPII-22 (SEQ ID NO:63):
t) DPII-25 (SEQ ID NO:69):
u) DPII-25.2 (SEQ ID NO:71):
v) DFII-2 (SEQ ID NO:104):
w) DFII-4.5 (SEQ ID NO:107):
x) DFII-15 (SEQ ID NO: 109):
y) DFII-17 (SEQ ID NO:111):
z) DFII-19. (SEQ ID NO: 114):
aa) DFI-22.2 (SEQ ID NO:93) : et
bb) DPII-20.6 (SEQ ID NO:56)

et dans lequel au moins une région comprend la séquence d'acides aminés DP I-21.7 possédant la séquence :

## K S I N G N A P A E I D L R Q L R T V T P I R L Q.

**5.** Peptide isolé selon la revendication 4, dans lequel ledit peptide comprend la séquence d'acides aminés DP I-21.7 de manière conjointe avec une combinaison de régions choisies parmi le groupe comprenant :

a) DF I-22.2, DP I-23.13, DP I-26.1, DP II-20.6, DP II-22.3 et DP II-25.2 ;
b) DF I-22.2, DP I-23.11, DP I-26.3, DP II-20.7, DP II-22.5 et DP II-25.4 ;
c) DF I-22.2, DP I-23.12, DP I-26.4, DP II-20.6, DP II-22.6 et DP II-25.2 ;
d) DF I-22.2, DP I-23.5, DP I-26.5, DP II-20.7, DP II-22.7 et DP II-25.3 ;
e) DF I-22.2, DP I-23.6, DP I-26.2, DP II-20.6, DP II-22.8 et DP II-25.4 ;
f) DF I-22.2, DP I-23.7, DP I-26.3, DP II-20.7, DP II-22.9 et DP II-25.2 ;
g) DF I-22.2, DP I-23.8, DP I-26.4, DP II-20.6, DP II-22.3 et DP II-25.4 ;
h) DF I-22.2, DP I-23.9, DP I-26.1, DP II-20.7, DP II-22.4 et DP II-25.3 ;
i) DF I-22.2, DP I-23.13, DP I-26.2, DP II-20.6, DP II-22.5 et DP II-25.4
j) DF I-22.2, DP I-23.14, DP I-26.3, DP II-20.7, DP II-22.6 et DP II-25.3 ;
k) DF I-22.2, DP I-23.15, DP I-26.4, DP II-20.6, DP II-22.6 et DP II-25.3 ;

l) DF I-22.2, DP I-23.16, DP I-26.5, DP II-20.6, DP II-22.3 et DP II-25.2;
m) DF I-22.2, DP I-23.17, DP I-26.2, DP II-20.6, DP II-22.4 et DP II-25.3 ;
n) DF I-22.2, DP I-23.8, DP I-26.3, DP II-20.6, DP II-22.5 et DP II-25.4 ;
o) DF I-22.2, DP I-23.9, DP I-26.4, DP II-20.6, DP II-22.6 et DP II-25.3 ; ou
p) DF I-22.2, DP I-23.13, DP I-26.5, DP II-20.6, DP II-22.7 et DP II-25.4.

**6.** Peptide isolé selon la revendication 4, dans lequel ledit peptide comprend un quelconque arrangement de la combinaison suivante des régions : X, Y, Z, A, B, C, D, dans lequel :

X représente DP I-21.7 ;
Y représente DP I-22.2 ;
Z représente DP I-23.1, DP I-23.10, DP I-23.11, DP I-23.12, DP I-23.13, DP I-23.14, DP I-23.15, DP I-23.16, DP I-23.17, DP I-23.5, DP I-23.6, DP I-23.7, DP I-23.8 ou DP I-23.9 ;
A représente DP I-26.1, DP I-26.2, DP I-26.3, DP I-26.9 ou DP I-26.5 ;
B représente DP I-20.6 ou DP II-20.7 ;
C représente DP II-22, DP II-22.6, DP II-22.7, DP II-22.8, DP II-22.9, DP II-22.10, DP II-22.11, DP II-22.3, DP II-22.4 ou DP II-22.5 ; et
D représente DP II-25.2, DP II-25.3 ou DP II-25.4.

**7.** Peptide isolé selon la revendication 4, dans lequel ledit peptide possède un arrangement séquentiel spécifique de séquences d'acides aminés, lesdits arrangements répondant à la formule :

a) ADYCZBX;
b) DYZCAXB; ou
c) DXZACBY;

où X, Y, Z, A, B, C et D sont tels que définis à la revendication 6.

**8.** Acide nucléique isolé selon la revendication 2, possédant une séquence encodant la totalité du peptide selon l'une quelconque des revendications 4 à 7 ou une portion de ce dernier contenant un déterminant antigénique.

**9.** Peptide isolé selon l'une quelconque des revendications précédentes (appropriée), produit dans une cellule hôte transformée avec l'acide nucléique selon la revendication 2 ou selon la revendication 8.

**10.** Peptide isolé d'un allergène protéique du genre *Dermatophagoides,* ledit peptide ou une portion de ce dernier comprenant au moins un déterminant antigénique lymphocyte T dudit allergène protéique, ledit peptide répondant à la formule $X_n$-Y-$Z_m$ :

dans laquelle Y représente la séquence d'acides aminés DP I-21.7 telle que définie à la revendication 1 ;
$X_n$ représente des résidus d'acides aminés contigus à la terminaison amino de Y dans la séquence d'acides aminés dudit allergène protéique ; et
$Z_m$ représente des résidus d'acides aminés contigus à la terminaison carboxyle de Y dans ledit allergène protéique ;
m et n représentent respectivement une valeur de 0 à 30.

**11.** Composition thérapeutique comprenant au moins un peptide isolé selon l'une quelconque des revendications 1, 3 à 7, 9 ou 10, et un support ou un diluant pharmaceutiquement acceptable.

**12.** Composition thérapeutique comprenant un support ou un diluant pharmaceutiquement acceptable et au moins deux peptides, lesdits peptides comprenant chacun au moins un déterminant antigénique lymphocyte T d'un allergène protéique du genre *Dermatophagoides,* dans laquelle au moins un peptide est choisi parmi le groupe comprenant :

a) DPI-21.1 (SEQ ID NO:27):
b) DPI-21.2 (SEQ ID NO:28):
c) DPI-22.1 (SEQ ID NO-29):
d) DPI-22.2 (SEQ ID NO:30):
e) DPI-22.3 (SEQ ID-NO:31):
f) DPI-22.4 (SEQ ID NO:32):

g) DPI-23.1 (SEQ ID NO:33):
h) DP I-23.2 (SEQ ID NO: 34);
i) DP I-25.1 (SEQ ID NO: 35);
j) DP I-25.2 (SEQ ID NO: 36);
k) DP I-26.1 (SEQ ID NO: 37);
l) DP I-27.1 (SEQ ID NO: 38);
m) DP I-28.1 (SEQ ID NO: 39);
n) DP I-28.2 (SEQ ID NO: 40);
o) DP I-1 (SEQ ID NO: 9);
p) DF I-1 (SEQ ID NO: 72);
q) DF I-21.1 (SEQ ID NO: 90);
r) DF I-21.2 (SEQ ID NO: 91);
s) DF I-22.1 (SEQ ID NO: 92);
t) DF I-22.2 (SEQ ID NO: 93);
u) DF I-22.4 (SEQ ID NO: 94);
v) DF I-23.1(SEQ ID NO: 95);
w) DF I-23.2 (SEQ ID NO: 96);
x) DF I-25.1 (SEQ ID NO: 97);
y) DF I-25.2 (SEQ ID NO: 98);
z) DF I-26.1 (SEQ ID NO: 99);
aa) DF I-27.1 (SEQ ID NO: 100);
bb) DF I-28.1 (SEQ ID NO: 101);
cc) DF I-28.2 (SEQ ID NO: 102);
dd) DP II-20 (SEQ ID NO: 50);
ee) DP II-20.1 (SEQ ID NO:51);
ff) DP II-20.2 (SEQ ID NO:52);
gg) DP II-20.3 (SEQ ID NO: 53);
hh) DP II-20.4 (SEQ ID NO:54);
ii) DP II-20.5 (SEQ ID NO:55);
jj) DP II-20.6 (SEQ ID NO: 56);
kk) DP II-1 (SEQ ID NO: 41);
ll) DP II-1.1 (SEQ ID NO: 57);
mm) DP II-1.2 (SEQ ID NO: 58);
nn) DP II-2.1 (SEQ ID NO:59);
oo) DP II-2.2 (SEQ ID NO: 60);
pp) DP II-2.3 (SEQ ID NO: 61);
qq) DP II-21 (SEQ ID NO: 62);
rr) DP II-22 (SEQ ID NO: 63);
ss) DP II-26 (SEQ ID NO: 64);
tt) DP II-26.1 (SEQ ID NO: 65);
uu) DP II-23 (SEQ ID NO: 66);
vv) DP II-23.1 (SEQ ID NO: 67);
ww) DP II-24 (SEQ ID NO: 68);
xx) DP II-25 (SEQ ID NO:69);
yy) DP II-25.1 (SEQ ID NO:70);
zz) DP II-25.2 (SEQ ID NO: 71);
aaa) DF II-1 (SEQ ID NO:103);
bbb) DF II-2 (SEQ ID NO:104);
ccc) DF II-13.1 (SEQ ID NO:105);
ddd) DF II-3.1 (SEQ ID NO: 106);
eee) DF II-4.5 (SEQ ID NO:107);
fff) DF II-4.3 (SEQ ID NO:108);
ggg) DF II-15 (SEQ ID NO: 109);
hhh) DF II-16 (SEQ ID NO:110);
iii) DF II-17 (SEQ ID NO:111);
jjj) DF II-18 (SEQ ID NO:112);
kkk) DF II-19 (SEQ ID NO: 113);
lll) DF II-19.1 (SEQ ID NO:114);

mmm) DF II-21 (SEQ ID NO:115); et
nnn) DF II-22 (SEQ ID NO:116);

et dans laquelle au moins un peptide est un peptide DP I-21.7 possédant la séquence :

KSINGNAPAEIDLRQLRTVTPIRLQ;

dans laquelle ladite composition comprend un pourcentage suffisant de déterminant antigénique lymphocyte T d'au moins un allergène protéique de telle sorte que lors de l'administration de la composition à un individu sensible à un allergène d'acarien dermatophagoïde, les lymphocytes T de l'individu sont rendus tolérants audit au moins un allergène protéique.

13. Composition selon la revendication 12, comprenant la séquence d'acides aminés DP I21-7 telle que définie ci-dessus, de manière conjointe avec une combinaison de peptides choisis parmi le groupe comprenant :

a) DF I-22.2, DP I-23.13, DP I-26.1, DP II-20.6, DP II-22.3 et DP II-25.2 ;
b) DF I-22.2, DP I-23.11, DP I-26.3, DP II-20.7, DP II-22.5 et DP II-25.4 ;
c) DF I-22.2, DP I-23.12, DP I-26.4, DP II-20.6, DP II-22.6 et DP II-25.2 ;
d) DF I-22.2, DP I-23.5, DP I-26.5, DP II-20.7, DP II-22.7 et DP II-25.3 ;
e) DF I-22.2, DP I-23.6, DP I-26.2, DP II-20.6, DP II-22.8 et DP II-25.4 ;
f) DF I-22.2, DP I-23.7, DP I-26.3, DP II-20.7, DP II-22.9 et DP II-25.2 ;
g) DF I-22.2, DP I-23.8, DP I-26.4, DP II-20.6, DP II-22.3 et DP II-25.4 ;
h) DF I-22.2, DP I-23.9, DP I-26.1, DP II-20.7, DP II-22.4 et DP II-25.3 ;
i) DF I-22.2, DP I-23.13, DP I-26.2, DP II-20.6, DP II-22.5 et DP II-25.4 ;
j) DF I-22.2, DP I-23.14, DP I-26.3, DP II-20.7, DP II-22.6 et DP II-25.3 ;
k) DF I-22.2, DP I-23.15, DP I-26.4, DP II-20.6, DP II-22.6 et DP II-25.3 ;
l) DF I-22.2, DP I-23.16, DP I-26.5, DP II-20.6, DP II-22.3 et DP II-25.2 ;
m) DF I-22.2, DP I-23.17, DP I-26.2, DP II-20.6, DP II-22.4 et DP II-25.3 ;
n) DF I-22.2, DP I-23.8, DP I-26.3, DP II-20.6, DP II-22.5 et DP II-25.4 ;
o) DF I-22.2, DP I-23.9, DP I-26.4, DP II-20.6, DP II-22.6 et DP II-25.3 ; ou
p) DF I-22.2, DP I-23.13, DP I-26.5, DP II-20.6, DP II-22.7 et DP II-25.4.

14. Composition selon la revendication 12, comprenant au moins deux des peptides X, Y, Z, A, B, C et D tels que définis à la revendication 6.

15. Utilisation d'un peptide tel que défini dans l'une quelconque des revendications 1, 3 à 7, 9 ou 10 dans la préparation d'un médicament pour traiter une sensibilité à un acarien dermatophagoïde chez un individu ; le cas échéant, ledit médicament procurant une administration simultanée ou séquentielle d'une quantité thérapeutiquement efficace d'au moins deux compositions différentes selon l'une quelconque des revendications 11 à 14.

16. Procédé in vitro de détection d'une sensibilité aux acariens dermatophagoïdes chez un individu, comprenant le fait de combiner un échantillon de sang prélevé de l'individu avec au moins un peptide selon l'une quelconque des revendications 1, 3 à 7, 9 ou 10 dans des conditions appropriées pour lier des composants du sang au peptide, et le fait de déterminer la sensibilité de l'individu aux acariens dermatophagoïdes ; le cas échéant, dans lequel l'étendue selon laquelle une telle liaison apparaît est déterminée en évaluant la fonction des lymphocytes T, la prolifération des lymphocytes T ou une combinaison des deux.

17. Peptide selon l'une quelconque des revendications 1, 3 à 7, 9 ou 10 à utiliser en médecine.

**Patentansprüche**

1. Isoliertes modifiziertes Peptid eines Protein-Allergens der Art *Dermatophagoides,* das zumindest ein T-Zell-Epitop des Protein-Allergens umfasst, wobei das Peptid DP I-21.7 mit der folgenden Sequenz ist:

## K S I N G N A P A E I D L R Q L R T V T P I R L Q.

**2.** Isolierte Nukleinsäure mit einer Sequenz, die ein Peptid nach Anspruch 1 kodiert.

**3.** Isoliertes modifiziertes Peptid nach einem der vorhergehenden Ansprüche, das weiter durch Zusatz von geladenen Aminosäuren oder geladenen Aminosäurepaaren zu dem Peptid modifiziert wurde, um die Löslichkeit zu erhöhen.

**4.** Isoliertes Peptid, das zumindest zwei Regionen umfasst, wobei jede Region zumindest ein T-Zell-Epitop eines Protein-Allergens der Art *Dermatophagoides* umfasst, wobei die Regionen von dem selben oder unterschiedlichen Protein-Allergenen der Art *Dermatophagoides* abgeleitet sind, wobei zumindest eine Region eine Aminosäuresequenz umfasst, die aus folgendem ausgewählt ist:

a) DP I-21.1 (SEQ ID NO: 27);
b) DP I-21.2 (SEQ ID NO: 28);
c) DP I-22.1 (SEQ ID NO: 29);
d) DP I-23.1 (SEQ ID NO: 33);
e) DP I-25.2 (SEQ ID NO: 36);
f) DP I-26.1 (SEQ ID NO: 37);
g) DP I-28.1 (SEQ ID NO: 39);
h) DP I-1 (SEQ ID NO: 9);
i) DF I-1 (SEQ ID NO: 72);
j) DF I-21.1 (SEQ ID NO: 90);
k) DF I-22.1 (SEQ ID NO: 92);
l) DF I-23.1 (SEQ ID NO: 95);
m) DF I-25.1 (SEQ ID NO: 97);
n) DP II-1 (SEQ ID NO: 41);
o) DP I1-1.2 (SEQ ID NO: 58);
p) DP II-2.0 (SEQ ID NO: 56);
q) DP II-20.3 (SEQ ID NO: 53);
r) DP II-21 (SEQ ID NO: 62);
s) DP II-22 (SEQ ID NO: 63);
t) DP II-25 (SEQ ID NO: 69);
u) DP II-25.2 (SEQ ID NO: 71);
v) DF II-2 (SEQ ID NO: 104):
w) DF II-4.5 (SEQ ID NO: 107);
x) DF II-15 (SEQ ID NO: 109);
y) DF II-17 (SEQ ID NO: 111);
z) DF II-19.1 (SEQ ID NO: 114)
aa) DF I-22.2 (SEQ ID NO:93); und
bb) DP II-20.6 (SEQ ID NO:56);

und wobei zumindest eine Region die Aminosäuresequenz DP I-21.7 mit der folgenden Sequenz umfasst:

## K S I N G N A P A E I D L R Q L R T V T P I R L Q.

**5.** Isoliertes Peptid nach Anspruch 4, wobei das Peptid die Aminosäuresequenz DP I-21.7 zusammen mit einer Kombination von Regionen umfasst, die aus folgendem ausgewählt sind:

a) DF I-22.2, DP I-23.13, DP I-26.1, DP II-20.6, DP II-22.3, und DP II-25.2;
b) DF I-22.2, DP I-23.11, DP I-26.3, DP II-20.7, DP II-22.5, und DP II-25.4;
c) DF I-22.2, DP I-23.12, DP I-26.4, DP II-20.6, DP II-22.6, und DP II-25.2;
d) DF I-22.2, DP I-23.5, DP I-26.5. DP II-20.7, DP II-22.7, und DP II-25.3;
e) DF I-22.2, DP I-23.6, DP I-26.2, DP II-20.6, DP II-22.8, und DP II-25.4;
f) DF I-22.2, DP I-23.7, DP I-26.3, DP II-20.7, DP II-22.9, und DP II-25.2;

g) DF I-22.2, DP I-23.8, DP I-26.4, DP II-20.6, DP II-22.3 und DP II-25.4;

h) DF I-22.2, DP I-23.9, DP I-26.1, DP II-20.7, DP II-22.4, und DP II-25.3;

i) DF I-22.2, DP I-23.13, DP I-26.2, DP II-20.6, DP II-22.5, und DP II-25.4;

j) DF I-22.2, DP I-23.14, DP I-26.3, DP II-20.7, DP II-22.6, und DP II-25.3;

k) DF I-22.2, DP I-23.15, DP I-26.4, DP II-20.6, DP II-22.6, und DP II-25.3;

l) DF I-22.2, DP I-23.16, DP I-26.5, DP II-20.6, DP II-22.3, und DP II-25.2;

m) DF I-22.2, DP I-23.17, DP I-26.2, DP II-20.6, DP II-22.4, und DP II-25.3; ;

n) DF I-22.2, DP I-23.8, DP I-26.3, DP II-20.6, DP II-22.5, und DP II-25.4;

o) DF I-22.2, DP I-23.9, DP I-26.4, DP II-20.6, DP II-22.6 und DP II-25.3; oder

p) DF I-22.2, DP I-23.13, DP I-26.5, DP II-20.6, DP II-22.7 und DP II-25.4.

6. Isoliertes Peptid nach Anspruch 4, wobei das Peptid irgendeine Anordnung der folgenden Kombination von Regionen umfasst: X, Y, Z, A, B, C, D;

wobei

X DP I-21.7 ist;

Y DF I-22.2 ist;

Z DP I-23.1, DP I-23.10, DP I-23.11, DP I-23.12, DP I-23.13, DP I-23.14, DP I-23.15, DP I-23.16, DP I-23.17, DP I-23.5, DP I-23.6, DP I-23.7, DP I-23.8 oder DP I-23.9 ist;

A DP I-26.1, DP I-26.2, DP I-26.3, DP I-26.4, oder DP I-26.5; B DP I-20.6 oder DP II-20.7 ist;

C DP II-22, DP II-22.6, DP II-22.7, DP II-22.8, DP II-22.9, DP II-22.10, DP II-22.11, DP II-22.3, DP II-22.4, oder DP II-22.5 ist; und

D DP II-25.2, DP II-25.3, oder DP II-25.4 ist.

7. Isoliertes Peptid nach Anspruch 4, wobei das Peptid eine spezifische sequentielle Anordnung von Aminosäuresequenzen aufweist, wobei die Anordnungen die folgende Formel aufweisen:

a) ADYCZBX;

b) DYZCAXB; oder

c) DXZACBY;

wobei X, Y, Z, A, B, C und D wie in Anspruch 6 definiert sind.

8. Isolierte Nukleinsäure nach Anspruch 2 mit einer Sequenz, die den gesamten oder einen Epitop-enthaltenden Anteil eines Peptides nach einem der Ansprüche 4 bis 7 kodiert.

9. Isoliertes Peptid nach irgendeinem (geeigneten) vorhergehenden Anspruch, erzeugt in einer Wirtszelle, die mit der Nukleinsäure nach Anspruch 2 oder Anspruch 8 transformiert wurde.

10. Isoliertes Peptid eines Protein-Allergens der Art *Dermatophagoides,* wobei das Peptid oder der Teil hiervon zumindest ein T-Zell-Epitop des Protein-Allergens umfasst, wobei das Peptid die Formel $X_n$-Y-$Z_m$ umfasst;

wobei Y die Aminosäuresequenz DP I-21.7 wie in Anspruch 1 definiert aufweist;

$X_n$ Aminosäurereste sind, die dem Amino-Terminus von Y in der Aminosäuresequenz des Protein-Allergens benachbart sind; und

$Z_m$ Aminosäurereste sind, die dem Carboxy-Terminus von Y in dem Protein-Allergen benachbart sind;

m und n jeweils 0-30 sind.

11. Therapeutische Zusammensetzung, die zumindest ein isoliertes Peptid nach einem der Ansprüche 1, 3-7, 9 oder 10 und einen pharmazeutisch verträglichen Träger oder ein Verdünnungsmittel umfasst.

12. Therapeutische Zusammensetzung, die einen pharmazeutisch verträglichen Träger oder Verdünnungsmittel und zumindest zwei Peptide umfasst, wobei die Peptide jeweils zumindest ein T-Zell-Epitop eines Protein-Allergens der Art *Dermatophagoides* umfassen, wobei zumindest ein Peptid aus folgendem ausgewählt ist:

a) DP I-21.1 (SEQ ID NO: 27);

b) DP I-21.2 (SEQ ID NO: 28);

c) DP I-22.1 (SEQ ID NO: 29);

d) DP I-22.2 (SEQ ID NO: 30):

e) DP I-22.3 (SEQ ID NO: 31);

f) DP I-22.4 (SEQ ID NO: 32);
g) DP I-23.1 (SEQ ID NO: 33):
h) DP I-23.2 (SEQ ID NO: 34);
i) DP I-25.1 (SEQ ID NO: 35);
j) DP I-25.2 (SEQ ID NO: 36);
k) DP I-26.1 (SEQ ID NO: 37);
l) DP I-27.1 (SEQ ID NO: 38);
m) DP I-28.1 (SEQ ID NO: 39);
n) DP I-28.2 (SEQ ID NO: 40);
o) DP I-1 (SEQ ID NO: 9);
p) DF I-1 (SEQ ID NO: 72);
q) DF I-21.1 (SEQ ID NO: 90);
r) DF I-21.2 (SEQ ID NO: 91);
s) DF I-22.1 (SEQ ID NO: 92);
t) DF I-22.2 (SEQ ID NO: 93);
u) DF I-22.4 (SEQ ID NO: 94);
v) DF I-23.1 (SEQ ID NO: 95);
w) DF I-23.2 (SEQ ID NO: 96);
x) DF I-25.1 (SEQ ID NO: 97);
y) DF I-25.2 (SEQ ID NO: 98);
z) DF I-26.1 (SEQ ID NO: 99);
aa) DF I-27.1 (SEQ ID NO: 100);
bb) DF I-28.1 (SEQ ID NO: 101);
cc) DF I-28.2 (SEQ ID NO: 102);
dd) DP II-20 (SEQ ID NO: 50);
ee) DP II-20.1 (SEQ ID NO: 51);
ff) DP II-20.2 (SEQ ID NO: 52);
gg) DP II-20.3 (SEQ ID NO: 53);
hh) DP II-20.4 (SEQ ID NO: 54);
ii) DP II-20.5 (SEQ ID NO: 55);
jj) DP II 20.6 (SEQ ID NO: 56);
kk) DP II-1 (SEQ ID NO: 41);
ll) DP II-1.1 (SEQ ID NO: 57);
mm) DP II-1.2 (SEQ ID NO: 58);
nn) DP II-2.1 (SEQ ID NO: 59);
oo) DP II-2.2 (SEQ ID NO: 60);
pp) DP II-2.3 (SEQ ID NO: 61);
qq) DP II-21 (SEQ ID NO: 62);
rr) DP II-22 (SEQ ID NO: 63);
ss) DP II-26 (SEQ ID NO: 64);
tt) DP II-26.1 (SEQ ID NO: 65);
uu) DP II-23 (SEQ ID NO: 66);
vv) DP II-23.1 (SEQ ID NO: 67);
ww) DP II-24 (SEQ ID NO: 68);
xx) DP II-25 (SEQ ID NO: 69);
yy) DP II-25.1 (SEQ ID NO: 70);
zz) DP II-25.2 (SEQ ID NO: 71);
aaa) DF II-1 (SEQ ID NO: 103)
bbb) DF II-2 (SEQ ID NO: 104);
ccc) DF II-13.1 (SEQ ID NO: 105);
ddd) DF II-3.1 (SEQ ID NO: 106);
eee) DF II-4.5 (SEQ ID NO: 107);
fff) DF II-4.3 (SEQ ID NO: 108);
ggg) DF II-15 (SEQ ID NO: 109);
hhh) DF II-16 (SEQ ID NO:110) ;
iii) DF II-17 (SEQ ID NO:111) ;
jjj) DF II-18 (SEQ ID NO:112) ;
kkk) DF II-19 (SEQ ID NO:113) ;

lll) DF II-19.1 (SEQ ID NO:114);
mmm) DF II-21 (SEQ ID NO: 115); und
nnn) DF II-22 (SEQ ID NO: 116);

und wobei zumindest ein Peptid DP I-21.7 mit der folgenden Sequenz ist:

$$K \ S \ I \ N \ G \ N \ A \ P \ A \ E \ I \ D \ L \ R \ Q \ L \ R \ T \ V \ T \ P \ I \ R \ L \ Q;$$

wobei die Zusammensetzung einen ausreichenden Prozentsatz an T-Zell-Epitopen zumindest eines Protein-Allergens derart umfasst, dass nach Verabreichung der Zusammensetzung an ein Individuum, das gegenüber einem Hausstaubmilbenallergen empfindlich ist, T-Zellen des Individuums eine Toleranz gegenüber dem zumindest einen Protein-Allergen entwickeln.

13. Zusammensetzung nach Anspruch 12, die die Aminosäuresequenz DP I-21.7 wie oben definiert zusammen mit einer Kombination von Peptiden umfasst, die aus folgendem ausgewählt sind:

a) DF I-22.2, DP I-23.13, DP I-26.1, DP II-20.6, DP II-22.3, und DP II-25.2;
b) DF I-22.2, DP I-23.11, DP I-26.3, DP II-20.7, DP II-22.5, und DP II-25.4;
c) DF I-22.2, DP I-23.12, DP I-26.4, DP II-20.6,DP II-22.6, und DP II-25.2;
d) DF I-22.2, DP I-23.5, DP I-26.5. DP II-20.7, DP II-22.7, und DP II-25.3;
e) DF I-22.2, DP I-23.6, DP I-26.2, DP II-20.6, DP II-22.8, und DP II-25.4;
f) DF I-22.2, DP I-23.7, DP I-26.3, DP II-20.7, DP II-22.9, und DP II-25.2;
g) DF I-22.2, DP I-23.8, DP I-26.4, DP II-20.6, DP II-22.3 und DP II-25.4;
h) DF I-22.2, DP I-23.9, DP I-26.1, DP II-20.7, DP II-22.4, und DP II-25.3;
i) DF I-22.2, DP I-23.13, DP I-26.2, DP II-20.6, DP II-22.5, und DP II-25.4;
j) DF I-22.2, DP I-23.14, DP I-26.3, DP II-20.7, DP II-22.6, und DP II-25.3;
k) DF I-22.2, DP I-23.15, DP I-26.4, DP II-20.6, DP II-22.6, und DP II-25.3;
l) DF I-22.2, DP I-23.16, DP I-26.5, DP II-20.6, DP II-22.3, und DP II-25.2;
m) DF I-22.2, DP I-23.17, DP I-26.2, DP II-20.6, DP II-22.4, und DP II-25.3; ;
n) DF I-22.2, DP I-23.8, DP I-26.3, DP II-20.6, DP II-22.5, und DP II-25.4;
o) DF I-22.2, DP I-23.9, DP I-26.4, DP II-20.6, DP II-22.6 und DP II-25.3; und
p) DF -22.2, DP I-23.13, DP I-26.5, DP II-20.6, DP II-22.7 und DP II-25.4.

14. Zusammensetzung nach Anspruch 12, die zumindest zwei der Peptide X, Y, Z, A, B, C und D wie in Anspruch 6 definiert umfasst.

15. Verwendung eines Peptids wie in einem der Ansprüche 1, 3-7, 9 oder 10 definiert in der Herstellung eines Medikaments zur Behandlung einer Empfindlichkeit gegenüber einer Hausstaubmilbe in einem Individuum; wobei das Medikament wahlweise eine simultane oder aufeinanderfolgende Abreichung einer therapeutisch wirksamen Menge von zumindest zwei unterschiedlichen Zusammensetzungen nach einem der Ansprüche 11-14 bereitstellt.

16. In vitro Verfahren zum Nachweis einer Empfindlichkeit gegenüber einer Hausstaubmilbe in einem Individuum, das das Kombinieren einer Blutprobe aus dem Individuum mit zumindest einem Peptid nach einem der Ansprüche 1, 3-7, 9 oder 10 unter Bedingungen umfasst, die zur Bindung von Blutbestandteilen mit dem Peptid geeignet sind und die Bestimmung der Empfindlichkeit in dem Individuum gegenüber einer Hausstaubmilbe umfasst; wobei wahlweise der Umfang, in dem eine Bindung eintritt, durch Bestimmung der T-Zellfunktion, T-Zellproliferation oder einer Kombination hiervon bestimmt wird.

17. Peptid nach einem der Ansprüche 1,3-7, 9 oder 10 zur Verwendung in der Medizin.

Fig. 1

Der fI adaptor

```
                     T   S   A   C   R   I   N   S   V   N
       5' -AATTTACCTCTGCTTGCCGTATCAACTCTGTTAACGCGGAATTCA-3'Hind III
φEcoR  I 3'-ATGGAGACGAACGGCATAGTTGAGACAATTGCGCCTTAAGTTCGA-5'
                                              Hpa I      EcoR I
```

Der pI adaptor

```
                     T   N   A   C   S
       5' -AATTTACCAACGCCTGCAGGCGAAGAATTCA-3'Hind III
φEcoR  I 3'-ATGGTTGCGGACGTCCGCTTCTTAAGTTCGA-5'
                         Pst I        EcoR I
```

## Fig. 2a

5' Der fII/pII primer
5' -GGGAATTCGATCAAGTCGATGTCAAA-3'


3' fII/pII primer
5' -GGCTGCAGTTATATTTAATCGCGGATTTT-3'


Der fII mutagenesis primer
5' -AATTGAAATCAAAGCCA-3'

## Fig. 2b

EP 0 694 067 B1

## Peptide Name

| | |
|---|---|
| DPI-1(1-20) | TNACSING*NAPAEIDLRQMR |
| DPI-2(13-39) | EIDLRQMRTVTPIRMQGGCGSCWAFSG |
| DPI-3(21-49) | TVTPIRMQGGCGSCWAFSGVAATESAYLA |
| DPI-4(40-60) | VAATESAYLAHRNQSLDLAEQ |
| DPI-11.1(50-71) | HRNQSLDLAEQELVDCASQHGC |
| DPI-12.1(61-81) | ELVDCASQHGCHGDTIPRGIE |
| DPI-5(73-100) | GDTIPRGIEYIQHNGVVQESYYRYVARE |
| DPI-5.1(81-100) | EYIQHNGVVQESYYRYVARE |
| DPI-13(85-109) | HNGVVQESYYRYVAREQSCRRPNAQ |
| DPI-14(101-119) | QSCRRPNAQRFGISNYCQI |
| DPI-15(110-131) | RFGISNYCQIYPPNANKIREAL |
| DPI-6.1(120-143) | YPPNANKIREALAQTHSAIAVIIG |
| DPI-7.1(132-157) | AQTHSAIAVIIGIKDLDAFRHYDGRT |
| DPI-8(144-169) | IKDLDAFRHYDGRTIIQRDNGYQPNY |
| DPI-9(158-180) | IIQRDNGYQPNYHAVNIVGYSNA |
| DPI-16(170-191) | HAVNIVGYSNAQGVDYWIVRNS |
| DPI-10(181-204) | QGVDYWIVRNSWDTNWGDNGYGYF |
| DPI-17(197-222) | GDNGYGYFAANIDLMMIEEYPYVVIL |
| DPI-21.1(1-28) | TNACSING*NAPAEIDLRQMRTVTPIRMQ |
| DPI-21.2(5-28) | SING*NAPAEIDLRQMRTVTPIRMQ |
| DPI-22.1(36-64) | AFSGVAATESAYLAHRNQSLDLAEQELVD |
| DPI-22.2(40-64) | VAATESAYLAHRNQSLDLAEQELVD |
| DPI-22.3(36-60) | AFSGVAATESAYLAHRNQSLDLAEQ |
| DPI-22.4(40-68) | VAATESAYLAHRNQSLDLAEQELVDCASQ |
| DPI-23.1(81-109) | EYIQHNGVVQESYYRYVAREQSCRRPNAQ |
| DPI-23.2(74-102) | DTIPRGIEYIQHNGVVQESYYRYVAREQS |
| DPI-25.1(118-146) | QIYPPNANKIREALAQTHSAIAVIIGIKD |

## Fig. 3

EP 0 694 067 B1

```
DPI-25.2(118-139)     QIYPPNANKIREALAQTHSAIA
DPI-26.1(141-166)     IIGIKDLDAFRHYDGRTIIQRDNGYQ
DPI-27.1(161-185)     RDNGYQPNYHAVNIVGYSNAQGVDY
DPI-28.1(173-201)     NIVGYSNAQGVDYWIVRNSWDTNWGDNGY
DPI-28.2(173-195)     NIVGYSNAQGVDYWIVRNSWDTN
DPII-1(1-20)          DQVDVKDCANHEIKKVLVPG
DPII-2(11-35)         HEIKKVLVPGCHGSEPCIIHRGKPF
DPII-3.1(22-50)       HGSEPCIIHRGKPFQLEAVFEANQNTKTA
DPII-4(36-60)         QLEAVFEANQNTKTAKIEIKASIDG
DPII-5(51-77)         KIEIKASIDGLEVDVPGIDPNACHYMK
DPII-6(61-86)         LEVDVPGIDPNACHYMKCPLVKGQQY
DPII-7(78-104)        CPLVKGQQYDIKYTWNVPKIAPKSENV
DPII-8(87-112)        DIKYTWNVPKIAPKSENVVVTVKVMG
DPII-9(105-129)       VVTVKVMGDDGVLACAIATHAKIRD
DPII-20(1-26)         DQVDVKDCANHEIKKVLVPGCHGSEP
DPII-20.1(1-26)E8     DQVDVKDEANHEIKKVLVPGCHGSEP
DPII-20.2(1-26)S8     DQVDVKDSANHEIKKVLVPGCHGSEP
DPII-20.3(1-26)E21    DQVDVKDCANHEIKKVLVPGEHGSEP
DPII-20.4(1-26)S21    DQVDVKDCANHEIKKVLVPGSHGSEP
DPII-20.5(1-26)E8E21  DQVDVKDEANHEIKKVLVPGEHGSEP
DPII-20.6(1-26)S8S21  DQVDVKDSANHEIKKVLVPGSHGSEP
```

Fig. 3 cont.

EP 0 694 067 B1

```
DPII-1.1(1-20)E8              DQVDVKDEANHEIKKVLVPG
DPII-1.2(1-20)S8              DQVDVKDSANHEIKKVLVPG
DPII-2.1(11-26)              HEIKKVLVPGCHGSEP
DPII-2.2(11-26)E21           HEIKKVLVPGEHGSEP
DPII-2.3(11-26)S21           HEIKKVLVPGSHGSEP
DPII-21(33-60)              KPFQLEAVFEANQNTKTAKIEIKASIDG
DPII-22(36-63)              QLEAVFEANQNTKTAKIEIKASIDGLEV
DPII-26(41-67)              FEANQNTKTAKIEIKASIDGLEVDVPG
DPII-26.1(45-67)            QNTKTAKIEIKASIDGLEVDVPG
DPII-23(79-104)             PLVKGQQYDIKYTWNVPKIAPKSENV
DPII-23.1(79-104)Y92        PLVKGQQYDIKYTYNVPKIAPKSENV
DPII-24(100-112)            KSENVVVTVKVMG
DPII-25(107-129)            TVKVMGDDGVLACAIATHAKIRD
DPII-25.1(107-129)E119      TVKVMGDDGVLAEAIATHAKIRD
DPII-25.2(107-129)L111S119  TVKVLGDDGVLASAIATHAKIRD
```

Fig . 3 cont.

## Peptide Name

| | |
|---|---|
| DFI-1(1-20) | TSACRINSVNVPSELDLRSLR |
| DFI-2.1(13-39) | ELDLRSLRTVTPIRMQGGCGSCWAFSG |
| DFI-3(21-49) | TVTPIRMQGGCGSCWAFSGVAATESAYLA |
| DFI-4(40-60) | VAATESAYLAYRNTSLDLSEQ |
| DFI-11(50-71) | YRNTSLDLSEQELVDCASQHGC |
| DFI-12(61-81) | ELVDCASQHGCHGDTIPRGIE |
| DFI-5(73-100) | GDTIPRGIEYIQQNGVVEERSYPYVARE |
| DFI-13(85-109) | QNGVVEERSYPYVAREQRCRRPNSQ |
| DFI-14(101-119) | QRCRRPNSQHYGISNYCQI |
| DFI-15(110-131) | HYGISNYCQIYPPDVKQIREAL |
| DFI-6(120-143) | YPPDVKQIREALTQTHTAIAVIIG |
| DFI-7(132-157) | TQTHTAIAVIIGIKDLRAFRHYDGRT |
| DFI-8.1(144-169) | IKDLRAFQHYDGRTIIQHDNGYQPNY |
| DFI-8(154-168) | DGRTIIQHDNGYQPN |
| DFI-9(158-180) | IIQHDNGYQPNYHAVNIVGYGST |
| DFI-16(170-191) | HAVNIVGYGSTQGDDYWIVRNS |
| DFI-10(181-204) | QGDDYWIVRNSWDTTWGDSGYGYF |
| DFI-17(197-222) | GDSGYGYFQAGNNLMMIEQYPYVVIM |
| DFI-21.1(1-28) | TSACRINSVNVPSELDLRSLRTVTPIRMQ |
| DFI-21.2(5-28) | RINSVNVPSELDLRSLRTVTPIRMQ |
| DFI-22.1(36-64) | AFSGVAATESAYLAYRNTSLDLSEQELVD |
| DFI-22.2(40-64) | VAATESAYLAYRNTSLDLSEQELVD |
| DFI-22.4(40-68) | VAATESAYLAYRNTSLDLSEQELVDCASQ |
| DFI-23.1(81-109) | EYIQQNGVVEERSYPYVAREQRCRRPNSQ |
| DFI-23.2(74-102) | DTIPRGIEYIQQNGVVEERSYPYVAREQR |
| DFI-25.1(118-146) | QIYPPDVKQIREALTQTHTAIAVIIGIKD |
| DFI-25.2(118-139) | QIYPPDVKQIREALTQTHTAIA |
| DFI-26.1(141-166) | IIGIKDLRAFQHYDGRTIIQHDNGYQ |

## Fig. 4

EP 0 694 067 B1

```
DFI-27.1(161-185)       HDNGYQPNYHAVNIVGYGSTQGDDY
DFI-28.1(173-201)       NIVGYGSTQGDDYWIVRNSWDTTWGDSGY
DFI-28.2(173-195)       NIVGYGSTQGDDYWIVRNSWDTT
DFII-1(1-20)            DQVDVKDCANNEIKKVMVDG
DFII-2(11-35)           NEIKKVMVDGCHGSDPCIIHRGKPF
DFII-13.1(22-50)        HGSDPCIIHRGKPFTLEALFDANQNTKTA
DFII-3.1(30-48)         HRGKPFTLEALFDANQNTK
DFII-4.5(36-60)         TLEALFDANQNTKTAKIEIKASLDG
DFII-4.3(45-70)         QNTKTAKIEIKASLDGLEIDVPGIDT
DFII-15(51-77)          KIEIKASLDGLEIDVPGIDTNACHFMK
DFII-16(61-86)          LEIDVPGIDTNACHFMKCPLVKGQQY
DFII-17(78-104)         CPLVKGQQYDAKYTWNVPKIAPKSENV
DFII-18(87-112)         DAKYTWNVPKIAPKSENVVVTVKLVG
DFII-19(95-129)         PKIAPKSENVVVTVKLVGDNGVLACAIATHAKIRD
DFII-19.1(105-129)      VVTVKLVGDNGVLACAIATHAKIRD
DFII-21(33-60)          KPFTLEALFDANQNTKTAKIEIKASLDG
DFII-22(36-63)          TLEALFDANQNTKTAKIEIKASLDGLEI
```

# Fig. 4 cont.

EP 0 694 067 B1

Fig. 5

Fig. 6

Fig. 7

Fig. 8

DER f I PEPTIDES

DER p I PEPTIDES

38% (2.8) DPI-17
63% (3.2) DFI-17
38% (2.8) DPI-10
38% (3.9) DFI-10
25% (4.2) DPI-16
75% (3.1) DFI-16
50% (2.4) DPI-9
75% (6.5) DFI-9
88% (3.4) DPI-8
63% (3.9) DFI-8.1
0% (0.0) DFI-7.1
50% (5.2) DPI-7
50% (3.0) DFI-6.1
38% (2.9) DPI-6
50% (15.2) DFI-15
50% (7.7) DPI-15
50% (3.9) DPI-14
38% (4.1) DFI-14
25% (2.9) DPI-13
63% (3.1) DFI-13
75% (2.9) DPI-5
50% (64.0) DFI-5
13% (3.7) DPI-12/12.1
25% (4.1) DFI-12
75% (9.1) DPI-11/11.1
63% (3.5) DFI-11
75% (3.6) DPI-4
50% (5.1) DFI-4
50% (4.0) DPI-3
50% (7.8) DFI-3
13% (3.0) DPI-2
50% (2.7) DFI-2
75% (3.8) DPI-1
75% (3.5) DFI-1

25    20    15    10    5    0

RANKED SUM OF 5 HIGHEST PEPTIDE RESPONSES

91

Fig. 9

Fig. 10

Fig. 11

Fig. 12

Fig. 13

Fig. 14

RANKED SUM OF 5 HIGHEST PEPTIDE RESPONSES

DER p I PEPTIDES

DER f I PEPTIDES

STIMULATION INDEX

| | |
|---|---|
| 23 % | DFI-28.2 |
| 17% | DPI-28.2 |
| 10% | DFI-28.1 |
| 30% | DPI-28.1 |
| 17% | DFI-27.1 |
| 13% | DPI-27.1 |
| 27% | DFI-26.1 |
| 30% | DPI-26.1 |
| 10% | DFI-25.2 |
| 37% | DPI-25.2 |
| 10% | DFI-25.1 |
| 20% | DPI-25.1 |
| 43% | DFI-23.2 |
| 33% | DPI-23.2 |
| 23% | DFI-23.1 |
| 60% | DPI-23.1 |
| 17% | DFI-22.4 |
| 33% | DPI-22.4 |
| 47% | DFI-22.3 |
| 47% | DPI-22.3 |
| 47% | DFI-22.2 |
| 47% | DPI-22.2 |
| 17% | DFI-22.1 |
| 43% | DPI-22.1 |
| 47% | DFI-21.2 |
| 53% | DPI-21.2 |
| 43% | DFI-21.1 |
| 63% | DPI-21.1 |

Fig. 15a

Fig. 15b

Fig. 16a

Fig. 16b

Fig. 17a

| | |
|---|---|
| 53% | DPII-25.2 |
| 20% | DPII-25.1 |
| 43% | DPII-25 |
| 30% | DPII-24 |
| 10% | DPII-23.1 |
| 27% | DPII-23 |
| 33% | DPII-26.1 |
| 17% | DPII-26 |
| 43% | DPII-22 |
| 47% | DPII-21 |
| 40% | DPII-2.3 |
| 47% | DPII-2.2 |
| 10% | DPII-2.1 |
| 47% | DPII-1.2 |
| 10% | DPII-1.1 |
| 47% | DPII-1 |
| 50% | DPII-20.6 |
| 13% | DPII-20.5 |
| 63% | DPII-20.4 |
| 50% | DPII-20.3 |
| 7% | DPII-20.2 |
| 50% | DPII-20.1 |
| 50% | DPII-20 |

STIMULATION INDEX

Fig. 17b

Fig. 18a

STIMULATION INDEX

Fig. 18b

Fig. 18c

Fig. 18d

Fig. 19a

EP 0 694 067 B1

Fig. 19b

Fig. 20a

EP 0 694 067 B1

Fig. 20b

PEPTIDES

■ PMA
▨ DP I 21.1
☐ DP I 21.2
▨ DF I 21.1
☐ DF I 21.2
▦ DP I 22.1
▨ DP I 22.2
░ DP I 22.3

ABS 450 nm

0.5
0.4
0.3
0.2
0.1
0.0

2   6   18   54   162   BACKGROUND

1/ DILUTION

Fig. 21a

Fig. 21b

Fig. 21c

Fig. 21d

Fig. 21e

EP 0 694 067 B1

Fig. 21f

EP 0 694 067 B1

Fig. 21g

Fig. 21h

EP 0 694 067 B1

```
                    10          20          30          40          50          60
Der p I (a)   TNACSINGNA  PAEIDLRQMR  TVTPIRMQGG  CGSCWAFSGV  AATESAYLAH  RNQSLDLAEQ
Der P I (b)   ----------  ----------  ----------  ----------  ---------Y  ----------
Der p I (c)   ----------  ----------  ----------  ----------  ---------Y  ----------
Der p I (d)   ----------  ----------  ----------  ----------  ----------  ----------

                    70          80          90         100         110         120
Der p I (a)   ELVDCASQHG  CHGDTIPRGI  EYIQHNGVVQ  ESYYRYVARE  QSCRRPNAQR  FGISNYCQIY
Der P I (b)   ----------  ----------  ----------  ----------  ----------  ----------
Der p I (c)   ----------  ----------  K---------  ----------  ----------  ----------
Der p I (d)   ----------  ----------  ----------  ----------  ----------  ----------

                   130         140         150         160         170         180
Der p I (a)   PPNANKIREA  LAQTHSAIAV  IIGIKDLDAF  RHYDGRTIIQ  RDNGYQPNYH  AVNIVGYSNA
Der P I (b)   ---V------  ----------  ----------  ----------  ----------  ----------
Der p I (c)   ---V------  ----------  ----------  ----------  ----------  ----------
Der p I (d)   ----------  -----T----  ----------  ----------  ----------  ----------
Der p I (e)                                      ----------  ----------  ----------

                   190         200         210         220
Der p I (a)   QGVDYWIVRN  SWDTNWGDNG  YGYFAANIDL  MMIEEYPYVV  IL
Der P I (b)   ----------  ----------  ----------  ----------  --
Der p I (c)   ----------  ----------  ----------  ----Q-----  --
Der p I (d)   ----------  ----------  ----------  ----------  --
Der p I (e)   ----------  ----------  ----------  ----------  --
```

<div align="center">

Fig. 22

</div>

EP 0 694 067 B1

```
                           10         20         30         40         50
Der p II (c)   DQVDVKDCANHEIKKVLVPGCHGSEPCIIHRGKPFQLEAVFEANQNTKTAK
         (1)   ...........H.....L.P.....E..........Q...V.E....T....
         (2)   ...........H.....L.P.....E..........Q...V.E....S....
Der f II       ...........N.....M.D.....D..........T...L.D....T....


                           60         70         80         90        100
Der p II (c)   IEIKASIDGLEVDVPGIDPNACHYMKCPLVKGQQYDIKYTWNVPKIAPKSE
         (1)   ......I............P....YM............I.............
         (2)   ......I............P....YM............I.............
Der f II       ......L............T....FM............A.............
                                        V            I


                          110        120
Der p II (c)   NVVVTVKVMGDDGVLACAIATHAKIRD
         (1)   .......VM.DD.......A....I..
         (2)   .......VM.ND.......A....L..
Der f II       .......LV.DN.......A....I..
                       I         G
```

<div align="center">

Fig. 23

</div>

EP 0 694 067 B1

EP 0 694 067 B1

```
              10         20         30         40         50         60
pFL1        DQVDVKDCANNEIKKVMVPGCHGSEPCIIHRGKPFTLEALFDANQNTKTAKIEIKASLDGLE
pFL2        ..........N.........................................I.I........
MT  3       ...·..····N.........................................I.T........
MT  5 (1-92)........S...........................................I.I........
MT18 (1-84).........N...........................................I.I........
MT16 (1-70).........N...........................................T.I........


              70         80         90        100        110        120        130
pFL1        IDVPGIDTNACHFVKCPLVKGQQYDIKYTWNVPKIAPKSENVVVTVKLIGDNGVLACAIATHAKIRD
pFL2        ..............M..........A............................V...............
MT  3       ..............M..........A............................V...............
MT  5       ..............M..........I....
MT18        ..............M........··
```

## Fig. 24

```
DP I-23.1        EYIQHNGVVQESYYRYVAREQSCRRPNAQ
DP I-23.1.1    KKEYIQHNGVVQESYYRYVAREQSCRRPNAQ
DP I-23.1.2     KEYIQHNGVVQESYYRYVAREQSCRRPNAQ
DP I-23.1.3      EYIQHNGVVQESYYRYVAREQSSRRPNAQ
DP I-23.1.4      EYIQHNGVVQESYYRYVAREQSERRPNAQ


DP II-22        QLEAVFEANQNTKTAKIEIKASIDGLEV
DP II-22.1    KKQLEAVFEANQNTKTAKIEIKASIDGLEV
DP II-22.2     KQLEAVFEANQNTKTAKIEIKASIDGLEVK
```

# Fig. 25

EP 0 694 067 B1

```
DPI-21.7      KSINGNAPAEIDLRQLRTVTPIRLQ
DPI-23.10     KKEYIQHNGVVQESYYRYVAREQSCRRPNAQ
DPI-23.13     KKEYIQHNGVVQESYYRYVAREQSSRRPNAQ
DPI-23.14     KKEYIQHNGVVQESYYRYVAREQSERRPNAQ
DPI-23.11     DKEYIQHNGVVQESYYRYVAREQSSRRPNAQR
DPI-23.12     DKEYIQHNGVVQESYYRYVAREQSCRRPNAQR
DPI-23.5      DEYIQHNGVVQESYYRYVAREQSCRRDD
DPI-23.6      DEYIQHNGVVQESYYRYVAREQSSRRDD
DPI-23.7      DEYIQHNGVVQESYYRYVAREQSCRRPD
DPI-23.8      DEYIQHNGVVQESYYRYVAREQSSRRPD
DPI-23.9      DEYIQHNGVVQESYYRYVAREQSE
DPI-23.15     RYVAREQSCRRPNAQ
DPI-23.16     RYVAREQSERRPNAQ
DPI-23.17     RYVAREQSSRRPNAQ
DPI-26.2      DEGIKDLDAFRHYDGRTIIQRDNGYQ
DPI-26.3      IGIKDLDAFRHYD
DPI-26.4      DAFRHYDGRTIIQ
DPI-26.5      TIIQRDNGYQPNY
DPII-20.7     DQVDVKDSANHEIKKVLVPGSHGSEPK
DPII-22.6     DKQLEAVFEANQNTKTAKIEIKASIDGLEVD
DPII-22.3     DKQLEAVFEANQNTKTAKIEIKASIDGLEVK
DPII-22.4     QLEAVFEANQNTKTAKIEIKASIDE
DPII-22.5     DKQLEAVFEANQNTKTAKIEIKASIDE
DPII-22.7     AKIEIKASIDGLE
DPII-22.8     DKEQLEAVFEANQNTKTAKIEIKASIDE
DPII-22.9     DKEQLEAVFEANQNTKTAKIEIKASIDEE
DPII-22.10    LEAVFEANQNTKTAK
DPII-22.11    LEAVFEANQATKTAK
DPII-25.3     KTVKVLGDDGVLASAIATHAKIRD
DPII-25.4     DTVKVLGDDGVLASAIATHAKIRD
```

# Fig. 26